# EUROPÄISCHE PATENTANMELDUNG

(11) **EP 1 430 903 A1**
(43) Veröffentlichungstag der Anmeldung: **23.06.2004**
(21) Anmeldenummer: 04090076.3
(22) Anmeldetag: 11.10.2001
(51) Int. Cl.: A61K 38/58, A61K 31/00, A61K 38/06, A61K 38/07, A61K 38/05, A61K 38/04, A61K 31/365, A61K 31/704, A61K 31/4015, A61K 31/407, A61K 31/415, A61K 31/336, A61K 31/5375, A61K 31/47, A61K 31/166, A61P 31/12, A61P 31/20, A61P 31/18, A61P 31/14, A61P 25/28, A61P 43/00, A61P 35/00, A61P 1/16, A61K 48/00

(54) **Verwendung von Proteasome Hemmern zur Behandlung von Virus-Infektionen**

(30) Priorität: 12.10.2000 DE 10051716; 03.10.2001 DE 10149398
(62) Teilanmeldung aus: 01986607.8
(71) Anmelder: VIROMICS GMBH, 07743 Jena (DE)
(72) Erfinder: Schubert, Ulrich, Prof.Dr., 07743 Jena/Thüringen (DE); Will, Hans, Prof.Dr., 22549 Hamburg (DE); Tessmer, Uwe, 20253 Hamburg (DE); Sirma, Hüseyin, Dr, 69168 Wiesloch (DE); Prassalow, Alexij, Dr., 10117 Berlin (DE); Schubert, Evelyn, Dr., 07743 Jena/Thüringen (DE); Hohenberg, Heinz, Dr., 22767 Hamburg (DE); Welker, Reinhold, Dr., 42113 Wuppertal (DE)
(74) Vertreter: Wehlan, Helmut, Dr.

(57) **Zusammenfassung**

Die Erfindung betrifft die Verwendung von Proteasom-Inhibitoren als Mittel zur Behandlung von Virus-Infektionen, insbesondere von Infektionen mit Retroviren. Gegenstand der Erfindung sind Mittel zur Hemmung der Freisetzung, der Reifung und der Replikation von Retroviren. Am Beispiel der Humanen Immundefizienzviren (HIV) wird gezeigt, dass Proteasom-Inhibitoren die Freisetzung von Viruspartikeln sowie die Infektiosität der freigesetzten Viruspartikel und damit die Virusvermehrung blockieren. Die Proteasom-Inhibitoren beeinflussen die Aktivitäten des Ubiquitin/Proteasom-Pathway, insbesondere die enzymatischen Aktivitäten des 26S und des 20S Proteasom-Komplexes. Anwendungsgebiete sind die anti-retrovirale Therapie und Vorbeugung bei Infektionen mit Immundefizienz verursachenden Lentiviren bei Tieren und Menschen, insbesondere HIV-Infektionen und AIDS oder HIV-induzierte Demenz, auch in Kombination mit anderen anti-retroviralen Medikamenten.

## Beschreibung

Die Erfindung betrifft die Verwendung von Proteasom-Inhibitoren als Mittel zur Behandlung von Virus-Infektionen, insbesondere von Infektionen mit Retroviren. Gegenstand der Erfindung sind Mittel zur Hemmung der Freisetzung, der Reifung, der Infektiosität und somit der Replikation von Retroviren. Am Beispiel der Humanen Immundefizienzviren des Typs 1 und 2 (HIV-1/HIV-2) wird gezeigt, dass diese Mittel sowohl die Prozessierung der HIV-1- und der HIV-2-Gag-Proteine als auch die Freisetzung von HIV-1- und HIV-2-Viruspartikeln sowie die Infektiosität der freigesetzten Viruspartikel und dadurch die HIV-Virusreplikation blockieren. Die Proteasom-Inhibitoren beeinflussen die Aktivitäten des Ubiquitin/Proteasom-Pathway, insbesondere die enzymatischen Aktivitäten des 26S und des 20S Proteasom-Komplexes. Anwendungsgebiete sind die anti-retrovirale Therapie und die Vorbeugung bei Infektionen mit Immundefizienz verursachenden Lentiviren bei Tieren und Menschen, insbesondere HIV-Infektionen und AIDS oder HIV-induzierte Demenz, auch in Kombination mit anderen anti-retroviralen Medikamenten.

### Charakteristik des bekannten Standes

### 0. Einleitung

Seit Anfang der 80er Jahre hat die Pandemie des erworbenen Immunmangelsyndroms (Aquired Immunodeficiency Syndrome, AIDS) Millionen von HIV-infizierten Menschen mit einer heimtückischen, multisystemischen und ultimativ bislang unheilbaren Krankheit konfrontiert. Zum gegenwärtigen Zeitpunkt ist weder eine wirksame Strategie zur Vakzinierung gegen HIV bekannt, noch sind andere Mechanismen der Stimulation einer nach wie vor wenig verstandenen natürlichen Immunität gegenüber einer HIV-Infektion für eine breite Anwendung applizierbar. Für die Therapie einer bereits etablierten HIV-Infektion oder zum Schutz vor einer systemischen Manifestierung einer HIV-Infektion unmittelbar nach Aufnahme des Virus sind verschiedene anti-retrovirale Medikamente im Einsatz. Diese stellen im wesentlichen Substanzen dar, welche die viralen Enzyme Reverse Transkriptase (RT) sowie Protease (PR) hemmen. Neben dem Problem der Unverträglichkeit besteht die hauptsächliche Limitierung dieser Medikamente in der enormen, bis 10⁶-Mal höheren Mutationsrate (verglichen mit der Replikation humaner DNA) von HIV. Der dadurch bedingte Polymorphismus führt unweigerlich und in relativ kurzer Zeit zum Auftreten von HIV-Mutanten, die gegenüber einzelnen oder sogar kombinierten anti-HIV-Therapeutika, insbesondere der HAART-Therapie (highly active antiretroviral therapy - Patentschrift WO 00/33654), resistent sind. Das Ziel der weiteren HIV-Forschung besteht damit in der Identifizierung von zellulären Angriffspunkten ("Targets") für eine anti-retrovirale Therapie. Dies betrifft zelluläre Faktoren, Enzyme oder komplexe Mechanismen, die für die Replikation von HIV in der Wirtszelle essentiell sind und selektiv manipuliert werden können, ohne die Vitalität des Gesamtorganismus wesentlich zu beeinträchtigen. Diesen Anspruch erfüllt die überraschende Feststellung, welche in dieser Erfindung beschrieben wird, und zwar, dass die Mittel, die Proteasom-Inhibitoren, späte Prozesse in der Replikation von HIV-1 und HIV-2 inhibieren und dadurch die Freisetzung und die Formierung von infektiösen Nachkommenviren verhindern (Literaturverzeichnis hinter den Ausführungsbeispielen).

### 1. Funktion des Ubiquitin/Proteasom-Pathway

Proteasomen sind multikatalytische und multi-Subunit Enzymkomplexe, die ca. 1% des Gesamt-Zellproteins darstellen und als die hauptsächliche proteolytische Komponente im Zellkern und Zytosol aller eukaryontischen Zellen vorkommen. Die wesentliche Funktion von Proteasomen ist die Proteolyse von missgefalteten, nicht-funktionellen oder für den schnellen Abbau bestimmten, in der Regel regulatorischen Proteinen. Eine weitere Funktion des proteasomalen Abbaus einer Vielzahl von zellulären oder viralen Proteinen ist die Generierung von Peptidliganden für Major Histocompatibilitäts (MHC) Klasse-1-Moleküle, welche für die T-Zellvermittelte Immunantwort notwendig ist (für Review siehe Rock und Goldberg, 1999).

Proteasom-Targets werden in der Regel für den proteasomalen Abbau durch die Anheftung von oligomeren Formen von Ubiquitin (Ub) markiert. Ub ist ein hochkonserviertes, 76 Aminosäuren langes Protein, das kovalent an Targetproteine *via* Isopeptidbindung zwischen dem COOH-Terminus und der ε-NH₂-Gruppe von Lysin-Seitenketten gekoppelt wird, entweder am Targetprotein oder an Ub-Moleküle, die bereits an das Targetprotein geheftet sind. Das Ergebnis der Konjugation von Ub-Molekülen ist die Formierung von sogenannten poly-Ub-Ketten. Allgemein sind Multimere von vier Ub-Molekülen notwendig, um als Signal für den Abbau durch das Proteasom zu fungieren. Die Ubiquitinierung selbst ist reversibel, und Ub-Moleküle können durch eine Vielzahl von Ub-Hydrolasen von dem Targetmolekül wieder entfernt werden. Die Verbindung zwischen der Ubiquitinierung von Targetproteinen und der proteasomalen Proteolyse wird allgemein als Ubiquitin-Proteasom-System (UPS) bezeichnet (für Review siehe Hershko und Chiechanover, 1998; Baumeister *et al.,* 1998).

Das 26S Proteasom ist ein 2.5 Megadalton (MDa) großer Multienzymkomplex, welcher aus ca. 31 Untereinheiten besteht (für Review siehe Voges *et al.,* 1999). Die proteolytische Aktivität des Proteasom-Komplexes wird durch eine core-Struktur, dem 20S Proteasom, realisiert. Das 20S Proteasom bildet einen aus 14 nicht identischen Proteinen (im Molekulargewichtsbereich von 21 bis 31 kDa) bestehenden komplizierten Multienzymkomplex, der in zwei α-und zwei β-Ringen in einer αββα-Reihenfolge angeordnet ist (für Review siehe Voges *et al.,* 1999). Die Substratspezifität des 20S Proteasom umfasst drei wesentliche proteolytische Aktivitäten: Trypsin-, Chymotrypsin- und Postglutamyl-Peptid hydrolysierende- (PGPH), oder auch Kaspase- ähnliche Aktivitäten, welche in den β-Untereinheiten Z, Y und Z lokalisiert sind. Die enzymatische Aktivitäten des 20S Proteasoms werden durch Anlagerung der 19S regulatorischen Untereinheiten gesteuert, welche zusammen das aktive 26S Proteasom-Partikel bilden. Die 19S regulatorischen Untereinheiten sind bei der Erkennung von poly-ubiquitinierten Proteinen sowie bei der Entfaltung von Targetproteinen beteiligt. Die Aktivität des 26S Proteasom ist ATP-abhängig und degradiert fast ausschließlich nur poly-ubiquitinierte Proteine. Die katalytisch aktiven β-Untereinheiten des 20S-Proteasoms (X, Y und Z) können durch γ-Interferon induzierbare MHC-kodierte Untereinheiten ausgetauscht werden, die dann das sogenannte "Immuno-Proteasom" bilden (Gaczynska *et al.,* 1993).

### 1.1. Bedeutung des Ubiquitin-Proteasom-Systems in der Pathogenese klinisch relevanter Krankheiten

Die enge Verknüpfung des UPS (Ubiquitin-Proteasom-System) mit zellulären Mechanismen erklärt die Bedeutung dieses Systems für zahlreiche pathologische Mechanismen, von denen bislang nur ein geringer Teil bekannt ist (für Review siehe Schwartz und Ciechanover, 1999). Eine zentrale Funktion übt das UPS bei Erkrankungen des Immunsystems aus. Zum einen ist der 26S Proteasom-Komplex die hauptsächliche Protease in der MHC-I-Antigenprozessierung, und zum anderen kann die Aktivität des Proteasoms selbst sowohl durch γ-Interferon induzierbare katalytische β-Untereinheiten manipuliert werden. Viele entzündliche und immunologische Krankheiten stehen im Zusammenhang mit dem Transkriptionsfaktor NF-κB, welcher verschiedene Gen-Funktionen in der Immunantwort reguliert. Die Aktivierung von NF-κB, die durch Ubiquitinierung und spezifische Spaltung eines Vorläuferproteins durch das Proteasom gesteuert wird, führt zur erhöhten Expression von verschiedenen Zytokinen, Adhäsionsmolekülen, entzündlichen und Stress-Response-Proteinen sowie Immunrezeptoren (für Review siehe Chiechanover *et al.,* 2000; Schwartz und Ciechanover, 1999).

### 1.2. Proteasom-Inhibitoren

Verschiedene Substanzklassen sind als Proteasom-Inhibitoren bekannt. Es sind zum einen chemisch modifizierte Peptidaldehyde wie der Tripeptidaldehyd N-carbobenzoxyl-L-leucinyl-L-leucinyl-L-leucinal (zLLL), das auch als MG132 bezeichnet wird sowie das um den Faktor 10 wirksamere Borsäure-Derivat MG232. Das zLLL und davon abgeleitete Derivate blockieren das Proteasom reversible durch Ausbildung einer transienten Hemiacetal-Struktur mit der katalytisch aktiven Threonin-Hydroxyl-Seitenkette in Position 1 der β-Untereinheit des 26S Proteasoms (für Review siehe Coux *et al.,* 1996). Ähnlich zu zLLL wurde eine weitere Klasse von modifizierten Peptiden als Proteasom-Inhibitoren, Peptid-Vinyl-Sulfone, beschrieben (Bogyo *et al.,* 1997).
Natürlich vorkommende Substanzen, isoliert aus Mikroorganismen, sind Lactacystin (LC) (Fenteany *et al.,* 1995) aus Streptomyceten sowie Epoxomicin aus Aktinomyzeten (Meng *et al.,* 1999a,b). LC ist ein hoch spezifischer und wirksamer Proteasom-Inhibitor, welcher das Proteasom durch Transesterifizierung und Alkylierung der Threonin-Seitenkette in der β-Untereinheit irreversibel inaktiviert (Fenteany *et al.,* 1995). LC ist daher ein irreversibler, kovalent wirkender Proteasom-Inhibitor, welcher hauptsächlich die Chymotrypsin- und die Trypsin-ähnlichen Aktivitäten des 26S Proteasom-Partikels blockiert (Fenteany *et al.,* 1995). LC hat keine Peptid-Grundstruktur, sondern besteht aus einem γ-Lactam-Ring, einem Cystein und einer Hydroxy-butyl-Gruppe. LC selbst inhibiert nicht das Proteasom. Vielmehr wird in wässriger Lösung der N-Acetyl-Cystein-Rest hydrolysiert. Das Resultat ist die Bildung eines Clastolactacystein β-Lactons. Diese Lacton-Struktur ist in der Lage, Zellmembranen zu penetrieren. Nach Zellaufnahme kommt es zum nukleophilen Angriff des β-Lacton-Rings und anschließender Transesterifizierung der Threonin¹-Hydroxyl-Gruppe der β-Untereinheit.
Ein weiterer Proteasom-Inhibitor ist das natürlich vorkommende Epoxyketon Epoxomicin. Hinsichtlich der Spezifität für das 26S Proteasom und Wirksamkeit ist Epoxomicin der bislang wirksamste von allen bekannten natürlich vorkommenden Proteasom-Inhibitoren (Meng *et al.,* 1999a,b). Weiterhin zeichnet sich Epoxomicin durch eine vergleichsweise geringe Toxizität in Zellkulturen aus (Hanada *et al.,* 1992).

Eine weitere und sehr potente Klasse an synthetischen Proteasom-Inhibitoren sind Borsäure-Peptid-Derivate, insbesondere die Verbindung Pyranozyl-Phenyl-Leuzinyl-Borsäure mit dem Namen "PS-341". PS-341 ist sehr stabil unter physiologischen Bedingungen und ist bioverfügbar nach intravenöser Applikation (Adams und Stein, 1996; Adams *et al.,* 1998). Borsäure-Peptid-Derivate sind allgemein bekannt als Inhibitoren verschiedenster eukaryotischer Proteasen, wie zum Beispiel Thrombin, Elastase, Dipeptidylprotease IV (für Review siehe Adams und Stein, 1996). Die besondere Wirksamkeit von PS-341 als Proteasom-Inhibitor wird durch die sehr stabile Bindung zwischen Borsäure- und Hydroxyl-Gruppe der katalytisch aktiven Seitenkette von Thr¹ in der aktiven β-Untereinheit des 20S-Proteasoms mit einer inhibitorischen Konstante (Ki) von 0.6 nM realisiert (Adams und Stein, 1996). Außer dem Proteasom ist bislang keine zelluläre Protease bekannt, welche durch PS-341 beeinflusst wird.
Andere, zu PS-341 ähnliche Borsäure-Peptid-Derivate wurden als Proteasom-Inhibitoren beschrieben, wie zum Beispiel Benzoyl-Phenylanalin-Borsäure-Leucin (Gardner *et* al*.,* 2000). Ebenfalls wurde ein stark potenter Proteasom-Inhibitor mit der Bezeichnung "PS-273" (Morpholin-CONH-(CH-Naphtyl)-CONH-(CH-isobutyl)-B(OH)₂) beschrieben, welcher eine ähnliche Grundstruktur wie PS-341 hat, jedoch N-terminal eine Morpholin-Sturktur aufweist, dadurch mehr hydrophob ist und vermutlich somit leichter Membranen penetrieren kann als PS-341 (Adams *et al*.*,* 1999).

### 1.3. Klinische Applikation von Proteasom-Inhibitoren

Der Proteasom-Komplex übt essentielle Zellfunktionen aus und ist für die Zellvitalität unverzichtbar. Die permanente Hemmung der Proteasom-Aktivität kann daher zu Veränderungen in der Regulation des Zellzyklus, der Transkription, der gesamten zellulären Proteolyse sowie der MHC-I Antigenprozessierung führen (für Review siehe Hershko und Ciechanover, 1998). Vollständige Inhibierung der Proteasom-Aktivität führt allgemein zu Zellzyklusarrest und Zelltod. Eine dauerhafte Inhibierung aller enzymatischen Aktivitäten des Proteasoms ist mit dem Leben einer Zelle und somit des Gesamtorganismus nicht vereinbar.
Es zeigt sich jedoch, dass neuartige reversibel wirkende Proteasom-Inhibitoren selektiv einzelne proteolytische Aktivitäten des 26S Proteasoms inhibieren, ohne dabei andere zelluläre Proteasen zu beeinflussen. Die Zytotoxizität solcher Inhibitoren ist daher wesentlich geringer im Vergleich zu den relativ unspezifisch wirkenden Proteasom-Inhibitoren, wie zum Beispiel dem Peptidaldehyd zLLL. Diese Tatsache erlaubt sowohl die *in vivo* Anwendung solcher neuartigen Proteasom-Inhibitoren (Meng *et al.,* 1999a) als auch die Etablierung von permanenten Zelllinien, welche relativ hohe Konzentrationen an Proteasom-Inhibitoren tolerieren (Glas *et al.,* 1998; Geier *et al.,* 1999).
Der Anspruch, dass bestimmte Proteasom-Inhibitoren in einem bestimmten Dosis-Regime *in vivo* verträglich sein können, wurde mehrfach gezeigt. Beispielsweise wurde die Selektion von Maus-Thymus-Zelllinien beschrieben, welche die ständige Anwesenheit von 10 µM des Proteasom-Inhibitors z-Leuzinyl-Leuzinyl-Leuzinyl-vinylsulfon (NLVS) tolerieren und dabei normales Zellwachstum und Zellmetabolismus mit gleichzeitig eingeschränkter MHC-I-Antigenpräsentation besitzen (Glas *et al.,* 1998). Ähnliche Ergebnisse wurden mit dem sehr potenten Proteasom-Inhibitor LC erzielt, der bis zu 6 µM in Zellkultur toleriert wurde (Geier *et al.*, 1999).
Epoxomicin, ein Epoxy-β-aminoketon-modifiziertes Peptid, wurde als eine völlig neuartige Klasse von Proteasom-Inhibitoren aus *Aktinomyceten* isoliert (Hanada *et al.,* 1992). Epoxomicin besitzt eine starke zytotoxische Wirkung gegen verschiedene *in vitro* kultivierte Tumorzelllinien und zeigte im Maus-Modell *in vivo* inhibitorische Aktivität gegen Melanom- und Leukämie-Modelltumore (Hanada *et al.,* 1992).
Die Bedeutung von Proteasom-Inhibitoren als ein neues therapeutisches Prinzip hat in den vergangenen Jahren eine zunehmende Aufmerksamkeit erfahren, insbesondere bei der Behandlung von Krebs und entzündlichen Erkrankungen (für Review siehe Murray und Norbury, 2000; Rivett und Gardner, 2000 - Kategory XXX = Literatur-Stellen nach dem Prioritätsdatum). Bislang ist der Einsatz von Proteasom-Inhibitoren für die breite klinische Anwendung am Menschen noch nicht zugelassen. Es mehren sich jedoch Berichte in der Fachliteratur, dass in jüngster Zeit die pharmazeutische Industrie intensiv an der Entwicklung von neuen Medikamenten auf der Basis von *in vivo*-verträglichen Proteasom-Inhibitoren arbeitet. Einige Beispiele seien hierzu angeführt: Die Firma "Millennium Pharmaceuticals, *Inc."* (Cambridge, MA 02139, USA) arbeitet nach Übernahme der Firma "ProScript, *Inc."* an der Entwicklung von Proteasom-Inhibitoren für entzündungshemmende, immunmodulatorische und antineoplasitsche Therapien, insbesondere an Borsäure-Derivaten von Di-Peptiden. Dabei spielen die Verbindungen PS-341 (Gardner *et al.,* 2000 Kategory XXX), PS-519 und PS-273 (Adams *et al.,* 1998, 1999) eine besondere Rolle.
Die orale Applikation von PS-341 hat im Ratten-Modell eine entzündungshemmende Wirkung in Streptokokken-induzierter Polyarthritis und Leber-Entzündung (Palombella *et al.,* 1998). Im Maus-Modell zeigt PS-341 anti-neoplastische Wirkung gegen Lungenkarzinom und hat außerdem eine additive Wirkung in Verbindung mit Zytostatika (Teicher *et al.,* 1999). *In vitro* Versuche demonstrieren eine sehr gute Wirksamkeit gegenüber soliden humanen Ovarien- und Prostata-Tumorzellen (Frankel *et al.,* 2000). Phase I klinische Studien an PS-341 beweisen eine gute Bio-Verfügbarkeit und pharmakokinetisches Verhalten (Lightcap *et al.,* 2000 Kategory XXX).
PS-341 ist der bislang einzige klinisch erprobte Proteasom-Inhibitor. Dazu wurden Phase I und Phase II klinische Studien in Patienten mit unterschiedlichen Krebserkrankungen, wie zum Beispiel hämatologischen Malignancies als soliden Tumoren bereits abgeschlossen. Die Informationen dazu wurden in verschiedenen Pressemitteilungen von Millennium Pharmaceuticals *Inc.* vorgestellt:
- Zum Beispiel wurden Ergebnisse über Phase-II-klinische Studien in Multiple Myeloma-Patienten berichtet (Presse-Mitteilung von Millennium Pharmaceuticals, *Inc.* von 01.03.01: "Millennium Initiates Phase II Clinical Trials of LDP-341 in Multiple Myeloma." Publiziert auf der Webseite
   http:biz.yahoo.com/prnews/010301/neth003.html, Kategory XXX).
- Erste vorklinische Studien über die Wirkung von Proteasom-Inhibitoren PS-341 als neue anti-Krebsbehandlung in Myelom-Patienten wurde auf dem 42. Meeting der Amerikanischen Hämatologischen Gesellschaft, Dezember 2000, San Francisco, CA, USA, vorgestellte (Presse-Mitteilung von Millennium Pharmaceuticals, *Inc.* von 04.12.00: Millennium's LDP(PS)-341 inhibits growth and induces death of cancer cells, apperars to overcome chemotherapy resitance. Publiziert auf der Webseite
   http:biz.yahoo.com/prnews/010301/neth003.html, Kategory XXX).
- Auf dem Meeting der Amerikanischen Gesellschaft für Klinische Onkologie, im Mai 2000, wurden Daten über Phase-I-klinische Studien mit PS-341 in Patienten mit fortgeschrittenen malignen Tumoren (einschließlich Melanoma, Nierenkarzimon, Lungenkarzinom, ProstataKrebs, Ovarien-Krebs, Blasen-Krebs, Cervix-Krebs, Endometrialen und Gallen-Tumoren) vorgestellt. Es wurde keine Dosis-limitierende Toxizität durch die Behandlung mit PS-341 beobachtet. Aufgrund der erstaunlichen Wirkung von PS-341 in Bezug auf anti-neoplastische Wirkung und Induktion von Apoptose konnten therapeutische Effekte in verschiedenen Tumor-Patienten beobachtet werden (Presse-Mitteilung von Millennium Pharmaceuticals, Inc. von 23.05.00: "Millennium presents clinical trial data on LDP-341 for advanced malignancies." Publiziert auf der Webseite
   http://www.mlnm.com.releases.pr052300_1.shtml).
- Weitere Informationen über das klinische Protokoll von Phase-I-Studien in Patienten mit fortgeschrittenen Tumoren (solide Tumore als auch Lymphoma), welche auf Standard-Chemotherapie nicht mehr angesprochen haben, wurden im "CancerNet" online publiziert ("Phase I study of PS-341 in patients with advanced solid tumors or lymphomas". Publiziert am 11.09.00 auf der Webseite http://cancernet.nci.nih.gov/, Kategory XXX).
- Ergebnisse über Phase-1-klinische Studien mit PS-341 in Patienten mit akuter Leukämie, Myelodysplastischem Syndrom und chronischer Myelider Leukämie wurden bekannt, dabei insbesondere die synergistische Wirkung von PS-341 mit Standard-Chemotherapeutika (Presse-Mitteilung von Millennium Pharmaceuticals, *Inc."* vom 09.11.00: "Phase I study of PS-341 in acute leukemias, myelodysplastic syndromes and chronic myeloid leukemia in blast phase." Publiziert online in Leukemia Insights Newsletter auf der Webseite
   http://www3.manderson.org./leukemia/insight/letter52.html).

In einem Maus-Modell konnte gezeigt werden, dass der Proteasom-Inhibitor PS-519 (ein β-Lacton-Derivat), entwickelt von der Firma Millennium Pharmaceuticals, *Inc.,* eine starke antiinflammatorische Wirkung ausübt, und zwar in beiden, der verzögerten als auch der übersensitiven Entzündungsreaktion. In niedrigen Dosen ist PS-519 auch wirksam in Kombination mit Steroiden. PS-519 wurde daher als ein neues Medikament für die Asthma-Behandlung vorgeschlagen (Elliott *et al.,* 1999). Eine weitere Anwendung für PS-519 ergibt sich im Infarkt-Modell: Die inflammatorische Reaktion nach cerebralen Verletzungen wurde durch PS-519 dramatisch reduziert. Danach scheint PS-519 ebenfalls ein interessantes Pharmakon für die Behandlung von Gehirnschlag zu sein (Phillips *et al.,* 2000, Kategory XXX).
Da Proteasom-Inhibitoren einen essentiellen Pathway im Zellmetabolismus treffen, ist ein strenges Dosis-Regime notwendig, um toxische Neben-Effekte zu unterdrücken. Im Rahmen der Entwicklung von *in vivo* verträglichen Proteasom-Inhibitoren wurden verschiedene Peptid-Borsäure-Derivate getestet, welche sowohl in der Zellkultur als auch im Tiermodell anti-Tumor Wirkung zeigten (Adams und Stein, 1996; Adams *et al.,* 1998, 1999, 2000). *In vitro* besitzt PS-341 eine selektive zytotoxische Aktivität gegen ein breites Spektrum an humanen Tumorzelllinien (Adams *et al.,* 1999). Diese Aktivität ist mit der Akkumulation von p21 und Zellzyklus-Arrest in der G2-M-Phase mit nachfolgender Apoptose verbunden (Adams *et al.,* 1999). Direkte Injektion von PS-341 bewirkte das Absterben von 70% der untersuchten Tumoren im Maus-Modell. Nach intravenöser Verabreichung von PS-341 verteilte sich die Substanz in allen Organen und Geweben und hatte anti-neoplastische Aktivität in humanen Xenograft-Modellen (Adams *et al.,* 1999).
Über den Einsatz von Proteasom-Inhibitoren jeglicher Substanzklasse mit dem Ziel, virale Infektionen zu blockieren, wurde bislang nicht berichtet. Ein Anspruch auf Blockierung/Hemmung des Replikationszyklus von HIV-1, HIV-2 oder anderen Lentiviren oder gar der Behandlung von AIDS-Patienten mittels Reagenzien, die das UPS beeinflussen, regulieren oder blockieren, wurde in der Literatur bisher nicht erhoben.

### 1.4. Verbindung zwischen dem Ubiqutin/Proteasom-Pathway und dem Replikationszyklus von Retroviren

### 1.4.1. Biologie von Retroviren

Die Familie der Retroviren, zu der auch die Humanen Immundefizienzviren (HIV) gehören, zählt zu der großen Gruppe von eukaryontischen retrotransposablen Elementen (für Review siehe Doolittle *et al.,* 1990). Diese zeichnen sich durch die Fähigkeit aus, unter Verwendung des Enzyms Reverse Transkriptase RNA-Genome in DNA-Intermediate umzuschreiben. Retroviren werden in fünf Subfamilien untergliedert: (i) Spumaviren; (ii) Mammalian C-Typ Oncoviren; (iii) BLV (Bovine Leukemia Virus)/HTLV (Human T-Cell Leukemia Virus) Leukämieviren; (iv) eine heterogene Gruppe aus RSV (Rous Sarcoma Virus), A, B und D-Typ Viren; sowie (v) Lentiviren (für Review siehe Doolittle *et al.,* 1990).
Lentiviren replizieren vorrangig in Lymphozyten und ausdifferenzierten Makrophagen und verursachen in der Regel lang andauernde und meist unheilbare Krankheiten. Retroviren beinhalten mindestens drei charakteristische Gene: *gag* (gruppenspezifisches Antigen), *pol* (Polymerase) und *env* (Hüllproteine). Außer Struktur- und enzymatisch aktiven Virusproteinen kodieren verschiedene Retroviren zusätzliche, in der Regel kleine Proteine mit regulatorischen Funktionen. Neben HIV zählen zu der Subfamilie der Lentiviren SIV (Simian Immunodeficiency Virus), EIAV (Equine Infectious Anemia Virus), BIV (Bovine Immunodeficiency Virus), FIV (Feline Immunodeficiency Virus) und Visna-Virus. HIV wiederum wird in die zwei Subtypen HIV-1 und HIV-2 untergliedert (für Review siehe Doolittle *et al.,* 1990).

### 1.4.2. Replikationszyklus von HIV

Der Replikationszyklus von HIV beginnt mit der Bindung des Virus an verschiedene Zellrezeptoren, unter denen das Glykoprotein CD4 als der primäre Rezeptor sowie verschiedene zellspezifische Chemokin-Rezeptoren als Co-Rezeptoren nach Bindung an CD4 fungieren. Nach Viruseintritt wird das virale RNA-Genom mittels Reverser Transkriptase (RT), RNase H und Polymerase in doppelsträngige DNA umgeschrieben, welche dann in Assoziation mit dem Präintegrationskomplex in den Zellkern transportiert und als Provirusgenom in chromosomale DNA mittels viraler Integrase eingebaut wird. Nach Transkription und Translation werden Gag/Gag-Pol-Polyproteine sowie Hüllproteine an die Zellmembran transportiert, wo die Assemblierung von Virionen erfolgt. Nach Budding und Abschnürung reifen Viruspartikel durch proteolytische Prozessierung der Gag/Gag-Pol-Polyproteine (für Review siehe Swanstrom und Wills, 1997).

### 1.4.3. Assemblierung, Freisetzung und Reifung von HIV-Partikeln

Die Hauptkomponenten der HIV-Strukturproteine werden in Form von drei Polyproteinen translatiert: Gag und Gag-Pol für die inneren Core-Proteine und viralen Enzyme sowie Env für die viralen Hüllproteine. Im Falle von HIV-1 resultiert die vollständige proteolytische Prozessierung des Gag Polyproteins Pr55 in der Formierung des Matrix (MA), des Capsid (CA) sowie des Nucleocapsids (NC) und des C-terminalen p6^{*gag*} Proteins. HIV-1-Virionen werden generell als unreife nicht-infektiöse Viruspartikel von der Plasmamembran abgeschnürt; dieser Prozess wird als Virusbudding bezeichnet. Unmittelbar nach oder auch während des Buddings beginnt mit der Aktivierung von Protease (PR) die proteolytische Prozessierung von Gag und Gag-Pol-Polyproteinen. Die proteolytische Reifung der Virionen geht einher mit morphologischen Veränderungen. Charakteristisch dabei ist die Kondensierung des inneren Cores, welche in der Formierung eines für das reife Virus typischen kegelförmigen Core-Zylinders resultiert (für Review siehe Swanstrom und Wills, 1997).

### 1.4.4. Ubiquitin/Proteasom-Pathway und Retrovirus-Replikation

Informationen über die Bedeutung des UPS für bestimmte Abschnitte der HIV-Replikation sind ebenfalls bekannt: Das System wird zum einen für die Proteolyse von neusynthetisierten Virusrezeptor-CD4 genutzt. Dieser Pathway wird durch das HIV-1-spezifische Protein Vpu vermittelt, das CD4 aus der Membran des endoplasmatischen Retikulum (ER) in die proteasomale Degradation im Zytoplasma leitet (Schubert *et al.,* 1998). Weiterhin liegen Hinweise darauf vor, dass nach Viruseintritt Proteasomen Gag-Proteine abbauen und dadurch die Infektiosität eindringender HIV-Partikel reduzieren (Schwartz *et al.,* 1998). Außerdem wurden mono-ubiquitinierte Formen von Gag für HIV-1 und Mo-MuLV Gag-Proteine beschrieben (Ott *et al.,* 1998).
Obwohl die katalytischen Aktivitäten des 26S Proteasoms vollständig verschieden von der sehr spezifischen Aspartat-Protease-Aktivität der HIV-1/HIV-2-viralen Proteasen sind, wurde beobachtet, dass ein spezifischer Inhibitor der HIV-1-Protease mit der Bezeichnung "Ritonavir" (jedoch kein anderer der bislang bekannten HIV-Protease-Hemmer) die Chymotrypsin-Aktivität des 20S Proteasoms *in vitro* (Schmidtke *et al.,* 1999) sowie die Proteasom-vermittelte MHC-I-Antigenexpression *in vivo* (André *et al.,* 1998) hemmen kann. Ausgehend von diesem in der wissenschaftlichen Literatur beschriebenen Phänomen wurde von Bryant *et al.* (2000) eine Patentanmeldung hinterlegt, die auf die Verwendung von HIV-1-Protease-Inhibitoren als Proteasom-Inhibitoren in der Behandlung von Krebs, von entzündlichen Erkrankungen und von nicht-HIV verwandten Infektionen gerichtet ist (WO 00/33654). Der Gegenstand der vorliegenden Anmeldung wird davon nicht berührt.

Folgende Patentschriften, welche die vorliegende Erfindung nicht unmittelbar betreffen, wurden veröffentlicht: Eine Erfindung, welche Mittel zur Interferenz von HBV-Infektionen auf der Basis von HBV-Protein X Wechselwirkung mit Proteasom-Untereinheiten beschreibt (US 5872206); ein Verfahren zur Bestimmung der Proteasom-Aktivität in biologischen Proben (WO 00/23614); die Verwendung von Proteasom-Inhibitoren als Mittel zur Behandlung von Krebs, Entzündungen und Autoimmunerkrankungen (WO 99/22729); die Verwendung von Inhibitoren des UPS als Mittel zur Behandlung von Entzündungen und Autoimmunerkrankungen (WO 99/15183).

### Wesen der Erfindung

Der Erfindung liegt die Aufgabe zugrunde, Mittel zur Verfügung zu stellen, die zur Behandlung von Virus-Infektionen geeignet sind und welche insbesondere die Freisetzung und Reifung von infektiösen Retroviren, vor allem von Immundefizienzviren, wie zum Beispiel HIV-1 und HIV-2, hemmen.
Die Aufgabe wurde durch den Einsatz von mindestens einem Proteasom-Inhibitor gelöst. Es sind erfindungsgemäß Mittel zur Behandlung von Virus-Infektionen entwickelt worden, die als wirksame Komponenten Proteasom-Inhibitoren in pharmazeutischen Zubereitungen enthalten. Die Erfindung bezieht sich auf Virus-Infektionen insbesondere solcher Viren, die im Rahmen des Replikationszyklus von der Zelloberfläche freigesetzt werden. Gemäß einer bevorzugten Ausführungsform der Erfindung werden als Proteasom-Inhibitoren Substanzen eingesetzt, welche die Aktivitäten des Ubiquitin/Proteasom-Pathway hemmen, regulieren oder anderweitig beeinflussen.
Es ist auch möglich, dass als Proteasom-Inhibitoren Substanzen eingesetzt werden, die speziell die enzymatischen Aktivitäten des kompletten 26S Proteasom-Komplexes und der freien, nicht mit regulatorischen Untereinheiten assemblierten 20S katalytisch aktiven Proteasom-Struktur beeinflussen. Diese Inhibitoren können entweder eine oder mehrere oder alle drei hauptsächlichen proteolytischen Aktivitäten des Proteasoms (die Trypsin-, die Chymotrypsinund die Postglutamyl-Peptid hydrolysierenden Aktivitäten) innerhalb des 26S- oder auch des 20S-Proteasom-Komplexes hemmen.
Eine Variante der Erfindung besteht darin, als Proteasom-Inhibitoren Substanzen einzusetzen, die von Zellen höherer Eykaryoten aufgenommen werden und nach Zellaufnahme mit der katalytischen beta-Untereinheit des 26S-Proteasoms in Wechselwirkung treten und dabei alle oder einzelne der proteolytischen Aktivitäten des Proteasom-Komplexes irreversibel oder reversibel blockieren.
Als eine weitere Form der Erfindung kommen Mittel zum Einsatz, welche die Aktivitäten der Ubiquitin-konjugierenden wie auch der Ubiquitin-hydrolysierenden Enzyme hemmen. Dazu gehören auch zellulären Faktoren, die mit Ubiquitin - als Mono- oder auch als Poly-Ubiquitin - in Wechselwirkung treten. Poly-Ubiquitinierung gilt allgemein als ein Erkennungssignal für die Proteolyse durch das 26S-Proteasom, und die Beeinflussung des Ubiquitin-Pathway kann ebenfalls die Aktivität des Proteasoms regulieren.
Erfindungsgemäß werden als Proteasom-Inhibitoren auch Substanzen eingesetzt, die in verschiedenen Formen *in vivo* oral, intravenös, intramuskulär, subkutan, in verkapselter Form mit oder ohne Zellspezifität-tragende Veränderungen, oder anderweitig verabreicht werden, aufgrund der Anwendung eines bestimmten Applikations- und Dosis-Regimes eine geringe Zytotoxizität und/oder hohe Selektivität für bestimmte Zellen und Organe aufweisen, keine oder unbedeutende Nebenwirkungen auslösen, eine relativ hohe metabolische Halbwertszeit und eine relativ geringe Clearence-Rate im Organismus aufweisen.
Als Proteasom-Inhibitoren werden des weiteren Substanzen eingesetzt, die in natürlicher Form aus Mikroorganismen oder anderen natürlichen Quellen isoliert werden, durch chemische Modifikationen aus natürlichen Substanzen hervorgehen oder total-synthetisch hergestellt werden oder durch gentherapeutische Verfahren *in vivo* synthetisiert oder durch gentechnische Verfahren in vitro oder in Mikroorganismen hergestellt werden. Dazu gehören:
a) natürlich vorkommende Proteasom-Inhibitoren:
   - Epoxomicin (Epoxomycin) und Eponemycin,
   - Aclacinomycin A (auch bezeichnet als Aclarubicin),
   - Lactacystin und dessen chemisch modifizierte Varianten, insbesondere die Zellmembranpenetrierende Variante "Clastolactacystein β-Lacton",
b) synthetisch hergestellte :
   - modifizierte Peptidaldehyde wie zum Beispiel N-carbobenzoxy-L-leucinyl-L-leucinyl-L-leucinal (auch bezeichnet als MG132 oder zLLL), dessen Borsäure-Derivat MG232; N-carbobenzoxy-Leu-Leu-Nva-H (bezeichnet als MG115); N-Acetyl-L-Leuzinyl-L-Leuzinyl-L-Norleuzinal (bezeichnet als LLnL); N-carbobenzoxy-Ile-Glu(OBut)-Ala-Leu-H (auch bezeichnet als PSI);
   - Peptide, die C-terminal α,β-Epoxyketone (auch bezeichnet als Epoxomicin / Epoxomycin oder Eponemycin), Vinyl-sulphone (zum Beispiel Carbobenzoxy-L-Leucinyl-L-Leucinyl-L-Leucin-vinyl-sulfon oder 4-Hydroxy-5-iodo-3-nitrophenylactetyl-L-Leucinyl-L-Leucinyl-L-Leucinvinyl-sulfon, auch bezeichnet als NLVS ), Glyoxal oder Borsäure-Reste (zum Beispiel Pyrazyl-CONH(CHPhe)CONH(CHisobutyl)B(OH)₂), auch bezeichnet als "PS-431" oder Benzoyl(Bz)-Phe-boroLeu, Phenacetyl-Leu-Leu-boroLeu, Cbz-Phe-boroLeu); Pinacol-Ester - zum Beispiel Benzyloxycarbonyl (Cbz)-Leu-Leu-boroLeu-Pinacol-Ester - tragen; und
   - als besonders geeignete Verbindungen werden Peptide und Peptid-Derivate eingesetzt, welche C-terminal Epoxyketon-Strukturen tragen, hierzu zählen beispielsweise Epoxomicin (Molekülformel: C₂₈H₈₆N₄O₇) und Eponemycin (Molekülformel: C₂₀H₃₆N₂O₅);
   - chemisch modifizierte Derivate auf der Basis von natürlich vorkommenden, insbesondere ein β-Lacton-Derivat mit der Bezeichnung PS-519 (1*R*-[1*S*, 4*R*, 5*S*]]-1-(1-Hydroxy-2-methylpropyl)-4-propyl-6-oxa-2-azabicyclo[3.2.0]heptane-3,7-dione, Molekülformel: C₁₂H₁₉NO₄), welches sich von dem natürlichen Proteasom-Inhibitor Lactacystin ableitet;
   - bestimmte Dipeptidyl-Borsäure-Derivate, insbesondere Verbindungen, welche sich von dem Pyranozyl-Phenyl-Leuzinyl-Borsäure-Derivat mit dem Namen "PS-341" (N-Pyrazinecarbonyl-L-Phenylalanin-L-leuzin-Borsäure, Molekülformel: C₁₉H₂₅BN₄O₄) ableiten. Hierzu zählen weiterhin die Verbindungen "PS-273" (Morpholin-CONH-(CH-Naphtyl)-CONH-(CH-isobutyl)-B(OH)₂) und dessen Enantiomer PS-293, die Verbindung PS-296 (8-Quinolyl-sulfonyl-CONH-(CH-Naphthyl)-CONH(-CH-isobutyl)-B(OH)₂); die Verbindung PS-303 (NH₂(CH-Naphtyl)-CONH-(CH-isobutyl)-B(OH)₂); die Verbindung PS-321 (Morpholin-CONH-(CH-Naphthyl)-CONH-(CH-Phenylalanin)-B(OH)₂); die Verbindung PS-334 (CH₃-NH-(CH-Naphthyl-CONH-(CH-Isobutyl)-B(OH)₂); die Verbindung PS-325 (2-Quinol-CONH-(CH-*homo*-Phenylalanin)-CONH-(CH-isobutyl)-B(OH)₂); die Verbindung PS-352 (Phenyalanin-CH₂-CH₂-CONH-(CH-Phenylalanin)-CONH-(CH-isobutyl)1-B(OH)₂); die Verbindung PS-383 (Pyridyl-CONH-(CH*p*F-Phenylalanin)-CONH-(CH-isobutyl)-B(OH)₂. Alle diese Verbindungen wurden bereits beschrieben, unter anderem in Adams *et al.* (1999).
Als besonders geeignete Verbindungen haben sich, neben Epoxomicin und Eponemycin, die Proteasom-Inhibitoren PS-519, PS-341 und PS-273 (entwickelt von der Firma Millennium Pharmaceuticals Inc., Cambridge, MA 02139, USA) erwiesen. Diese Proteasom-Inhibitoren sind sehr potent, sehr spezifisch für das Proteasom, blockieren keine anderen zellulären Proteasen und haben daher so gut wie keine Nebenwirkungen. Die Proteasom-Inhibitoren PS-341 und PS-519 wurden außerdem sowohl in Tiermodellen für vorklinische als auch in Menschen (Krebspatienten) für klinische Studien getestet.
Mit den Proteasom-Inhibitoren werden erfindungsgemäß Mittel zur Verfügung gestellt, die überraschenderweise durch die Blockierung von späten Prozessen der Retrovirus-Replikation die Produktion von infektiösen Nachkommen-Viren beeinträchtigen und damit die Ausbreitung der Infektion im Organismus verhindern.
Im Rahmen der vorliegenden Erfindung wird überraschenderweise festgestellt, dass Proteasom-Inhibitoren späte Prozesse im Replikationszyklus von Retroviren hemmen. Dabei wurde spezifisch festgestellt, dass sich die erfindungsgemäße Verwendung von Proteasom-Inhibitoren dazu eignet, die Assemblierung und Freisetzung von Virionen von der Zelloberfläche zu hemmen. Dabei tritt eine Hemmung der proteolytischen Prozessierung der Gag-Strukturproteine durch die virale Protease ein. Ebenfalls ist die Infektiosität der freigesetzten Virionen reduziert.

Die Hemmung folgender Retroviren ist möglich: Spumaviren, Mammalian C-Typ Oncoviren, BLV (Bovine Leukemia Virus), HTLV (Human T-Cell Leukemia Virus), Leukämieviren, RSV (Rous Sarcoma Virus) Viren oder Lentiviren. Als Beispiele für Leukämieviren kommen BLV, HTLV-I oder HTLV-II in Frage, Beispiele für Lentiviren sind Humanes Immundefizienzvirus Type 1 (HIV-1), Humanes Immundefizienzvirus Type 2 (HIV-2), Affen-Immundefizienzvirus (SIV), Katzen-Immundefizienzvirus (FIV) oder Rinder-Immundefizienzvirus (BIV).
Gegenstand der Erfindung ist - ebenfalls unter Verwendung von Proteasom-Inhibitoren - die Bekämpfung/ Behandlung von Erkrankungen/ pathologischen Erscheinungen, die durch Infektionen mit Retroviren verursacht wurden. Die Erkrankungen/pathologischen Erscheinungen können durch Infektionen mit Leukämieviren, humanen T-Zell Leukämieviren HTLV-I und HTLV-II oder durch Infektionen mit Lentiviren verursacht werden.
Ein weiteres Anwendungsgebiet der Erfindung ist die Bekämpfung/ Behandlung von AIDS mittels Proteasom-Inhibitoren, sowohl in der frühen symptomlosen als auch in der fortgeschrittenen Krankheitsphase. Sie, die Proteasom-Inhibitoren, können auch in Kombination mit anderen anti-retroviralen Medikamenten eingesetzt werden, z.B. mit Blockern von viraler RT und PR. Auch die Kombination mit anti-retroviralen Therapien basierend auf gentherapeutischen Interventionen ist möglich. Eine weitere Verwendung ergibt sich durch die Kombination mit intrazellulärer Immunisierung, wie z.B. dem Einbringen von anti-HIV-1/HIV-2 wirksamen Genen in Stammzellen und/oder in periphere CD4⁺ Lymphozyten.
Eine Verhinderung des Krankheitsausbruches und eine Reduzierung der Infektionsausbreitung im Organismus (Reduzierung von Viruslast) von symptomlosen HIV-1/HIV-2-seropositiven und HIV-1/HIV-2-infizierten Personen ist erfindungsgemäß ebenfalls möglich. Weiterhin können Proteasom-Inhibitoren zur Behandlung / Bekämpfung / Verhinderung von HIV-induzierter Demenz, insbesondere zur Verhinderung der HIV-Infektion von Neuronen, Glia und Endothelzellen in Kapillaren des Gehirns eingesetzt werden. Eine weitere Verwendung ist die Verhinderung der Etablierung einer systemischen HIV-1/HIV-2-Infektion unmittelbar nach Kontakt mit infektiösem Virus (zum Beispiel bei Nadel-Stich-Verletzungen mit HIV-kontaminiertem Blut oder Blutprodukten).
Die Verwendung von Proteasom-Inhibitoren in der Grundlagenforschung zum Verständnis der Retrovirusassemblierung, der Wirkungsweise der viralen Proteasen, bei Studien der Gag-Prozessierung, bei der Entwicklung von weiteren Substanzen, welche die Gag-Prozessierung beeinflussen, bei Untersuchungen zum Verständnis zellulärer Mechanismen involviert in späte Prozesse der Retrovirus-Assemblierung kann ebenfalls erreicht werden.

Die Prinzip-Lösung der Aufgabe wird am Beispiel von HIV-1 und HIV-2 gezeigt. Es wird dargestellt, dass unmittelbar nach Zugabe von verschiedenen Substanzklassen von Proteasom-Inhibitoren die Produktion von infektiösen Viruspartikeln gehemmt wird. Erfindungsgemäß wird dieses Phänomen sowohl in HIV-1-infizierten permanenten Kulturen von CD4⁺ humanen T-Zellen als auch in Kulturen von humanen Fibroblasten (HeLa-Zellen) transfiziert mit infektiöser proviraler DNA HIV-1 und HIV-2 beobachtet und hier näher beschrieben. Aufgrund dieser neuartigen Aktivitäten von Proteasom-Inhibitoren ist davon auszugehen, dass die Applikation von *in vivo* verträglichen Proteasom-Inhibitoren die Infektionsausbreitung von HIV im Organismus unterdrücken oder vollständig eliminieren kann.
Erfindungsgemäß wird gezeigt, dass der hemmende Effekt von Proteasom-Inhibitoren auf die HIV-Replikation auf folgenden Mechanismen beruht:
1) Blockierung/Reduktion der proteolytischen Prozessierung der Gag-Polyproteine durch die HIV-1-PR (Protease);
2) Blockierung/Reduktion der Freisetzung und des Budding von neuen Virionen an der Zellmembran;
3) Blockierung/Reduktion der Infektiosität von freigesetzten Virionen;
4) Blockierung/Reduktion der Infektionsausbreitung von HIV-1 in Kultur CD4⁺ T-Zellen.
Diese Mechanismen können direkt oder indirekt mit dem Phänomen in Zusammenhang stehen, dass Ubiquitin-Ligasen mit L(late assembly)-Domänen von HIV-1-Gag-Proteinen in Wechselwirkung treten (Strack et al., 2000, Kategorie XXX) und dass nach Inaktivierung des UPS die Mono-Ubiquitinierung von L-Domänen enthaltenden HIV-1-Gag-Proteinen blockiert wird (Schubert et al., 2000 und Ott et al, 2000, Kategorie XXX).
Zur Lösung der Aufgabe wurden im Rahmen der Erfindung verschiedene proteinchemische, molekular-virologische und morphologische Studien an HIV-1 durchgeführt. Erfindungsgemäß wird der durch Proteasom-Inhibitoren ausgelöste Defekt in der Gag-Prozessierung mittels biochemischer Methoden dargestellt. Dazu wurde eine metabolische pulse-Markierung von HIV-Proteinen mittels radioaktiver Aminosäuren, gefolgt von einer Inkubation (chase) in nichtradioaktivem Medium, durchgeführt. Die dabei gewonnenen Informationen ermöglichen die Darstellung des hemmenden Effektes von Proteasom-Inhibitoren auf Gag-Prozessierung und Budding von HIV-Virionen innerhalb kurzer Zeit-Kinetiken, die Teilabschnitten eines HIV-Replikationszyklus entsprechen.
Erfindungsgemäß wird dargestellt, dass die hemmende Wirkung von Proteasom-Inhibitoren auf HIV-Assemblierung und Freisetzung nicht die enzymatische Aktivität der HIV-1-PR trifft. Durch *in vitro* Prozessierungsstudien an isolierten Gag- und PR-Molekülen von HIV-1 wird gezeigt, dass verschiedene Substanzklassen von Proteasom-Inhibitoren keinerlei Einfluss auf die enzymatische PR-Aktivität ausüben.
Ferner wird erfindungsgemäß die durch Wirkung der Proteasom-Inhibitoren reduzierte Infektiosität freigesetzter unreifer HIV-Virionen mittels End-Punkt-Titrationsstudien in CD4⁺ T-Zellkulturen dargestellt. Dabei wird gezeigt, dass allein eine Inkubation mit Proteasom-Inhibitoren für sechs Stunden (entspricht etwa einem Drittel eines HIV-Replikationszyklus in der Targetzelle) zu einer 10-fachen Reduktion im Virus-Titer und zu einer 50-fachen Reduktion der spezifischen Infektiosität des freigesetzten Viruspartikels führt.
Erfindungsgemäß wird der Einfluss von Proteasom-Inhibitoren auf die Morphologie von HIV-1-Virionen im Assemblierungs- und Budding-Prozess an der Zellmembran untersucht. Zur Lösung dieser Aufgabe wird hochauflösende Transmissions-Elektronenmikroskopie an HIV-1-infizierten CD4⁺ T-Zellen durchgeführt. Dabei wird festgestellt, dass die Behandlung mit Proteasom-Inhibitoren für einen Zeitraum von etwa 5 Stunden zu folgenden Veränderungen in der Virusmorphologie führt:
1) Der Arrest von assemblierenden Virionen in der Budding-Phase ist signifikant erhöht;
2) die Ablösung der Virionen von der Zelloberfläche ist gestört, und es kommt zur Ausbildung von Virus-Membran-Verbindungen ("Stalk-formation");
3) die absolute Zahl von Viruspartikeln an der Zelloberfläche ist reduziert;
4) die relative Zahl von unreifen, zellfreien Virionen ist erhöht.

Erfindungsgemäß wird der inhibitorische Effekt von Proteasom-Inhibitoren auf die Virusreplikation in Kulturen HIV-1-infizierter CD4⁺ T-Zellen demonstriert. Die Zugabe von nanoM-Konzentrationen an verschiedenen Substanzklassen von Proteasom-Inhibitoren verhindert die Infektionsausbreitung und bewirkt das Ausbleiben einer produktiven Virusreplikation.
Das in der Erfindungsbeschreibung dargestellte Prinzip der Verwendung von Proteasom-Inhibitoren zur Blockierung einer HIV-Infektion ist neuartig in Hinsicht auf die Verwendung einer bereits bekannten Substanzklasse (den Proteasom-Inhibitoren) für eine neue Aktivität (der Blockierung von Gag-Prozessierung und Freisetzung von Retroviren).
Gleichzeitig ist die Verwendung von Proteasom-Inhibitoren auch neuartig hinsichtlich des Anwendungsprinzips. Bislang sind keine Substanzen / Prinzipien / Methoden bekannt, welche späte Prozesse der HIV-Replikation beeinflussen, ohne dabei Mutationen im Virus selbst als Voraussetzung zu haben.
Weiterhin ist neu, dass die Verwendung von Proteasom-Inhibitoren zur Blockierung von HIV und anderen Retroviren nicht das Virus selbst, sondern Mechanismen trifft, die in allen Wirtszellen des Virus konserviert sind. Im Vergleich zu bisherigen anti-retroviralen Methoden, die essentielle Komponenten des Virus per se betreffen, ist die Wahrscheinlichkeit der Entstehung von Resistenzmechanismen bei Applikation von Proteasom-Inhibitoren in der anti-retroviralen Therapie um Größenordnungen geringer. Die Neuartigkeit dieses Wirkprinzips von Proteasom-Inhibitoren zeigt sich auch in der Tatsache, dass Proteasom-Inhibitoren ein breites Wirkungsspektrum gegenüber unterschiedlichen Isolaten von HIV-1 und HIV-2 besitzen. Der inhibitorische Effekt wurde im Rahmen der Erfindung mit gleicher Intensität bei verschiedenen primären wie auch Zellkultur-adaptierten T-Zelltropen und Makrophagen-tropen HIV-Isolaten beobachtet.
Neuartig ist weiterhin das Prinzip der Wirkung von Proteasom-Inhibitoren, die zwar nicht den Viruseintritt, wohl aber die Produktion von infektiösen Viruspartikeln von bereits infizierten Zellen verhindern. Dadurch sollte wesentlich die Menge an infektiösen Virionen (Viruslast) und somit die Infektionsausbreitung *in vivo* reduziert werden können. Die mittlere Überlebenszeit einer akut HIV-infizierten T-Zelle beträgt wenige Tage. Zudem ist bekannt, dass die Hemmung der Virusfreisetzung und die damit verbundene Akkumulation von zum Teil toxischen HIV-Proteinen (insbesondere den Env-Hüllproteinen) zu einem verstärkten zytopathischen Effekt und dadurch zum schnelleren Absterben der infizierten Zelle führt. Neben der Neuinfektion sollte die Wirkung von Proteasom-Inhibitoren auch zu einem schnelleren Absterben von bereits infizierten Zellen führen.

In der Summe dieser neuartigen Mechanismen lässt sich feststellen, dass die verminderte Freisetzung von noch dazu wenigen oder gar nicht infektiösen Viruspartikeln im Netto-Effekt bei gleichzeitigem Zelltod der Virus-produzierenden Zellen im Falle einer *in vivo* Anwendung von Proteasom-Inhibitoren die Menge an infektiösen Virionen im peripheren Blut und gleichzeitig die Zahl infizierter Produzentenzellen von HIV im Gesamt-Organismus reduziert wird. Dies macht die Anwendung von Proteasom-Inhibitoren allein oder in Kombination mit bereits in der anti-retroviralen Therapie verwendeten Enzymhemmern attraktiv.

### Proteasom-Inhibitoren reduzieren die Infektiosität von HIV-1-Viruspartikeln

Im Rahmen der Erfindung wird erstmalig gezeigt, dass unmittelbar nach Inaktivierung des Proteasom-Pathway durch Behandlung von HIV-1-infizierten T-Zellen die Freisetzung von Viruspartikeln reduziert wird. Des weiteren wird die spezifische Infektiosität der freigesetzten Virionen erniedrigt und dadurch der spezifische Titer an neuproduzierten Viruspartikeln dramatisch reduziert (siehe hierzu Ausführungsbeispiel 1).
Erfindungsgemäß werden hierzu CD4⁺ T-Zellen mit HIV-1 infiziert und zum Zeitpunkt der maximalen Virusproduktion (ca. 80% der Zellkultur befindet sich in der akuten Infektionsphase) mit Proteasom-Inhibitoren für verschiedene Zeiträume, bis maximal 4.5 Stunden behandelt. Zu bestimmten Zeitpunkten nach der Behandlung werden virushaltige Zellkulturüberstände geerntet. Die Menge an freigesetzten Virionen wird mittels anti-Capsid Antigen-ELISA bestimmt und die spezifische Infektiosität der neu produzierten Virionen mittels Endpunkt-Titration ermittelt.
Erfindungsgemäß wird mit diesem Ergebnis eine neuartige Aktivität von Proteasom-Inhibitoren festgestellt. Diese kann nicht auf eine rein unspezifische Beeinträchtigung des Zellmetabolismus durch Abschaltung des UPS zurückgeführt werden, und zwar aus folgenden Gründen:
- Dieser Effekt wird sehr frühzeitig wirksam, zu einem Zeitpunkt, zu dem potentielle negative Effekte der Proteasom-Inhibition auf Zellprozesse wie zum Beispiel Protein-synthese und - faltung sowie die Funktionen von Chaperonen noch nicht wirksam werden können.
- Die freigesetzten Virionen haben eine deutlich reduzierte Infektiosität, was im Rahmen der weiteren Erfindungsbeschreibung mit einem spezifisch durch Proteasom-Inhibitoren ausgelösten Defekt in der proteolytischen Reifung von HIV-Partikeln erklärt wird.
- Toxische Effekte wie unspezifische Zelllyse, Apoptose oder die unspezifische Freisetzung von zellulären und viralen Proteinen wurden unter den hier beschriebenen experimentellen Bedingungen der Behandlung mit Proteasom-Inhibitoren nicht festgestellt.
- Die hemmende Wirkung von Proteasom-Inhibitoren ist ausschließlich auf die Beeinflussung zellulärer Prozesse zurückzuführen, die für die Virusassemblierung, Freisetzung und die Virus-Reifung notwendig sind, nicht aber auf chemische Veränderungen der freigesetzten Virionen selbst. Diese Tatsache reflektiert die erfindungsgemäß gemachte Beobachtung, dass zellfreie HIV-1-Virionen, die in Zellen mit aktivem Proteasom-Pathway produziert werden, nicht in ihrer Infektiosität beeinträchtigt wurden, wenn man diese mit relativ hohen Konzentrationen an Proteasom-Inhibitoren vor der Titration behandelt.
Zusammengefasst kann hiermit festgestellt werden, dass Proteasom-Inhibitoren durch die Beeinflussung zellulärer Prozesse die Produktion von infektiösen HIV-1-Partikeln stört. Der Mechanismus dieses Effektes wird im folgenden näher erläutert.

### Elektronenmikroskopische Analyse von HIV-1-infizierten Zellen

Im Rahmen der Erfindung wird die spezifische Wirkung von Proteasom-Inhibitoren auf Assemblierung und Budding von HIV-1-Virionen mit Hilfe der Transmissionselektronenmikroskopie untersucht (siehe hierzu Ausführungsbeispiel 2). Erfindungsgemäß wird mit dieser Methode die neuartige Wirkung von Proteasom-Inhibitoren auf die Virusmorphologie deutlich gemacht. Hierzu werden akut infizierte T-Zellen mit Proteasom-Inhibitoren für 5 Stunden behandelt. Die Zellen werden in Zellulose-Kapillaren eingeschlossen, inkubiert, fixiert und für Dünnschnitte verwendet. Diese Methode hat den wesentlichen Vorteil, dass Virionen in den Zellulose-Kapillaren zurückgehalten werden und daher keine Konzentrierung und damit Veränderung der Virusmorphologie durch Zentrifugation notwendig ist.
Erfindungsgemäß werden bei dieser Untersuchung wesentliche Phänomene der Wirkung von Proteasom-Inhibitoren auf die Virusmorphologie beobachtet:
- Eine relative Reduktion in der Zahl von extrazellulären Viruspartikeln und Buddingstrukturen an der Zellmembran;
- eine relative Zunahme in der Zahl von unreifen Viruspartikeln, die mit der Zellmembran verhaftet bleiben und sich nicht vollständig abschnüren können.
Diese elektronenmikroskopischen Untersuchungen bestätigen die im Rahmen der Erfindung gemachten biochemischen und virologischen Beobachtungen, dass die Behandlung mit Proteasom-Inhibitoren die Freisetzung und die Infektiosität neuer Virionen reduziert. Gleichzeitig zeigen diese elektronenmikroskopischen Untersuchungen an, dass die proteolytische Reifung und damit die Reifung von Viruspartikeln durch die Behandlung mit Proteasom-Inhibitoren negativ beeinflusst werden. Wesentlicher Prozess der HIV-Reifung ist die proteolytische Spaltung der Gag-Polyproteine durch die virale Protease. Im Rahmen der weiteren Untersuchungen wird gezeigt, dass eben dieser Prozess der proteolytischen Reifung durch Proteasom-Inhibitoren gehemmt wird.

### Proteasom-Inhibitoren reduzieren die Kinetik von Gag-Prozessierung und Virusbudding von HIV-1 und HIV-2

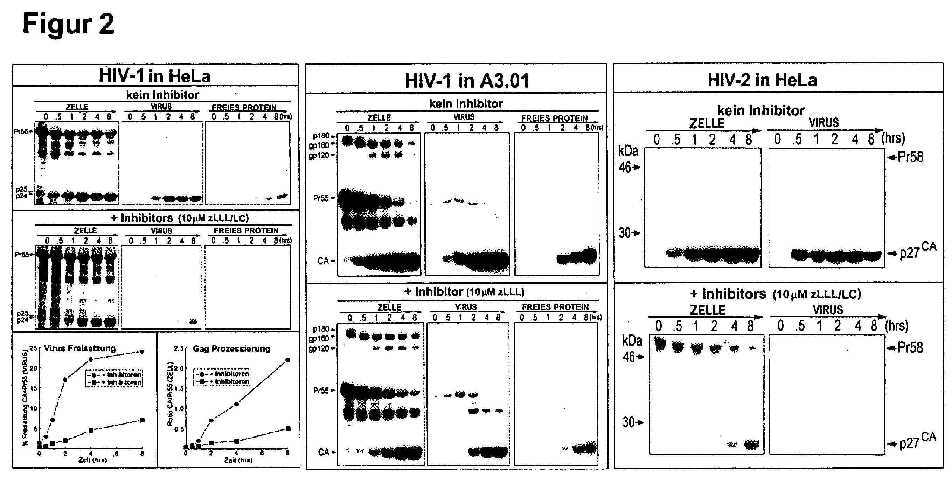

Im Rahmen der Erfindung wird mittels pulse/chase-Analysen nachgewiesen, dass Proteasom-Inhibitoren die Kinetik von Gag-Prozessierung und Virusfreisetzung / Budding wesentlich reduzieren (siehe hierzu Ausführungsbeispiel 3). Erfindungsgemäß werden hierzu Zellen (entweder T-Zellen infiziert mit HIV-1 oder HeLa-Zellen transfiziert mit infektiöser proviraler DNA von HIV-1 oder HIV-2) zum Zeitpunkt der maximalen Expression der viralen Proteine mit [³⁵ S]-Methionin in der Zelle metabolisch für einen relativ kurzen pulse-Zeitraum von ca. 30 min markiert. Anschließend werden die Zellen in Abwesenheit von radioaktiv markierten Aminosäuren inkubiert. Zu verschiedenen Zeitpunkten innerhalb des chase-Zeitraums werden äquivalente Proben von Zellen, dem Medium und der durch Zentrifugation isolierten Virionen gewonnen. Der Transport, die Prozessierung und die Assemblierung der radioaktiv markierten viralen Proteine werden insgesamt über einen chase-Zeitraum von 8 Stunden verfolgt. Die einzelnen HIV-Proteine werden durch Immunpräzipitation mittels HIV-spezifischer Antikörper und AIDS-Patientenseren aus dem intrazellulären, dem extrazellulären und den Virusassozierten Fraktionen isoliert, in der SDS-PAGE aufgetrennt und anschließend durch Image-Analyse quantifiziert. In parallelen Ansätzen eines jeweiligen Experimentes werden Zellen mit Proteasom-Inhibitoren behandelt, während im Kontroll-Ansatz die Zellen unbehandelt bleiben. Um eine möglichst vollständige Blockierung der Proteasom-Aktivität zu erzielen, werden die Zellen mit jeweils 25 microM zLLL und LC behandelt. Die Inhibitoren werden ca. 30 min vor Beginn der pulse-Markierung zu dem Zellkulturmedium gegeben. Diese kurze Vorinkubation beeinträchtigt nicht die Protein-Biosynthese und bewirkt auch keine wesentlichen Veränderungen in der Konzentration an zellulären Chaperonen (Schubert *et al.,* 2000, Kategorie XXX). Die relative Menge an markiertem Gag-Polyprotein Pr55 und dem Hauptprozessierungsprodukt p24 Capsid (CA) wird für jeden Zeitpunkt der Probenentnahme in den Zell-, Medium-, und Virus-Fraktionen ermittelt. Davon ausgehend wird die Rate der Virusfreisetzung (entspricht der Menge an radioaktiv markiertem Gag, welches innerhalb von 8 Stunden nach Synthese in der Virus-Fraktion auftritt) und Gag-Prozessierung (Rate der Umwandlung von radioaktiv markierten Gag Pr55 in CA) ermittelt.
Im Rahmen der Erfindung wird dabei eine ca. 6-fache Reduktion in der Virusfreisetzungskinetik innerhalb der ersten zwei Stunden nach pulse-Markierung in Zellen mit inaktivierten Proteasomen beobachtet (Fig. 2). Parallel dazu wird ebenfalls eine ähnlich starke Reduktion in der Kinetik der Gag-Prozessierung beobachtet, die als der Quotient von CA zu Pr55 für jeden Zeitpunkt des chases ermittelt wurde (Fig. 2). Dieser Prozessierungseffekt scheint mehrere Schritte der Reifung von Pr55 zu beeinflussen. Die vollständige Spaltung von HIV-1 Pr55 resultiert allgemein in der Entstehung der reifen Gag-Proteine MA, CA, NC und P6^{*gag*} sowie zwei kleinerer Spacer-Peptide, welche die einzelnen Domänen miteinander verbinden. Mehrere dieser Spaltprozesse scheinen im Prozess der Gag-Prozessierung durch die Einwirkung von Proteasom-Inhibitoren gehemmt zu sein, da das Auftreten von Intermediaten der Gag-Prozessierung wie zum Beispiel MA-CA (p41), p39 (Ca-NC) oder CA mit einem 14 Aminosäure langen Spacer (p25^{CA}) nach Blockade der Proteasom-Aktivität beobachtet werden. Im Unterschied zur Gag-Prozessierung beeinflussen die Proteasom-Inhibitoren nicht die Prozessierung der Env-Hüllproteine, auch wird nicht die Expression und die Stabilität anderer viraler Proteine beeinträchtigt.
Zusammengefasst wird festgestellt, dass Proteasom-Inhibitoren die Gag-Prozessierung und die Freisetzung von Viruspartikel blockieren. Das Ausmaß der Hemmung ist abhängig von der Zeit der Vorinkubation mit Proteasom-Inhibitoren vor dem Start des pulse/chase-Experimentes. Nach ca. 5 Stunden Vorinkubation ist die Prozessierung von Gag-Proteinen und die Freisetzung von Viruspartikeln fast vollständig blockiert.
Dieser neuartige Effekt von Proteasom-Inhibitoren scheint einen generellen Mechanismus der Assemblierung, des Budding und der Reifung von Retroviren zu beeinflussen. Dieser Effekt ist nicht spezifisch auf ein bestimmtes HIV-1-Isolat beschränkt. Vergleichende Analysen von Makrophagen-tropen HIV-1-Isolaten zeigen ähnliche Effekte, wie sie für T-Zell-trope HIV-1-Isolate beobachtet werden. Im Sinne der Erfindung wird ein hemmender Effekt von Proteasom-Inhibitoren auch auf die Gag-Prozessierung und die Virusfreisetzung von verschiedenen HIV-2-Isolaten beobachtet (Fig. 2, Panel HIV-2 in HeLa).
Es wird weiterhin gezeigt, dass die im Rahmen der Erfindung beschriebenen Phänomene einer spezifischen Inhibierung des 26S Proteasom-Komplexes bedürfen. Die Testung von Peptidaldhyden, welche ähnlich zLLL zytosolische Kaspasen, aber nicht das Proteasom inhibieren, üben keine hemmende Wirkung auf HIV-1 Gag-Prozessierung und Virusfreisetzung aus.

Aufgrund der neuartigen Aktivität von Proteasom-Inhibitoren kann festgestellt werden, dass
- Proteasom-Inhibitoren sowohl die Prozessierung von Gag-Proteinen als auch die Freisetzung und das Budding von neuen Virionen von der Zelloberfläche blockieren;
- diese hemmende Eigenschaft von Proteasom-Inhibitoren nicht auf einzelne Isolate von HIV-1 beschränkt ist, sondern auch für andere Lentiviren, wie zum Beispiel HIV-2, gilt;
- diese neuartige Aktivität nicht primär auf unspezifisch wirkenden negativen Effekten von Proteasom-Inhibitoren auf zelluläre und virale Prozesse basiert, da dieser Effekt selektiv für Gag-Prozessierung und Virusfreisetzung im Prozess der Virusassemblierung und Reifung wirksam wird;
- dieses Phänomen der selektiven Inhibierung der Aktivität des 26S Proteasom-Komplexes bedarf.
Da die Gag-Prozessierung zusammen mit einer effizienten Freisetzung von Viruspartikeln wesentlicher Bestandteil des Retrovirusreplikationszyklus und damit essentiell für die Produktion von infektiösen Virionen ist, kann davon ausgegangen werden, dass die hier beschriebene neuartige Funktion von Proteasom-Inhibitoren geeignet ist, die Infektionsausbreitung von HIV-1 und HIV-2 (den zwei Erregern der AIDS-Pandemie) *in vivo* zu unterdrücken. Da HIV-infizierte Zellen allgemein nur eine begrenzte Lebensdauer besitzen und zudem durch Blockierung der Virusfreisetzung der Zelltod infizierter Zellen noch beschleunigt wird, kann diese neuartige Aktivität von Proteasom-Inhibitoren bei *in vivo* Applikation sowohl die Neuinfektion nicht-infizierter Zellen verhindern, als auch das Absterben bereits infizierter Zellen induzieren und somit eine vollständige Eradikation (Auslöschung) der Infektion bewirken. Auch ist für HIV-1 bekannt, dass eine effiziente Gag-Prozessierung Voraussetzung für eine effiziente Virusfreisetzung ist. Die beiden neuartigen Effekte von Proteasom-Inhibitoren auf späte Prozesse der Virusreplikation sind somit direkt miteinander verbunden und können sich dadurch gegenseitig verstärken, jedoch nicht gegenseitig ausschließen.

### Proteasom-Inhibitoren beeinflussen nicht die enzymatische Aktivität der HIV-1-Protease

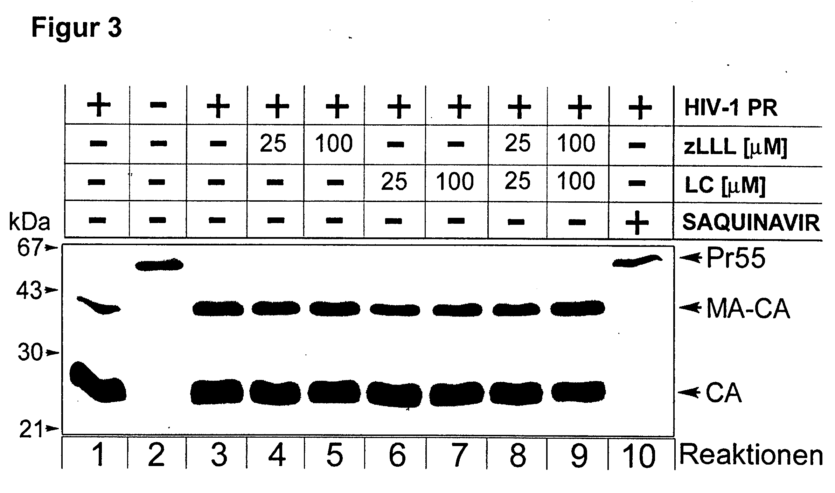

Zum mechanistischen Verständnis der im Rahmen der Erfindung gezeigten neuartigen Effekte ist es wesentlich, die spezifische Wirkung von Proteasom-Inhibitoren auf die virale Protease von HIV näher zu verstehen. Die einfachste Erklärung für die in dieser Erfindung beschriebenen neuartigen Phänomene wäre eine direkte Hemmung der HIV-Protease durch Proteasom-Inhibitoren. Dieser Zusammenhang erscheint jedoch unwahrscheinlich, da beide Protease-Komplexe vollkommen verschiedene enzymatische Mechanismen ausüben: Während die HIV-1-Protease als Dimer wirksam wird und dabei den Mechanismus von Aspartat-Proteasen ausübt, stellt das Proteasom ein multi-Enzym-Komplex mit mehreren aktiven Zentren dar. Ungeachtet der Tatsache, dass die proteolytischen Aktivitäten und die katalytischen Mechanismen von HIV-Proteasen und dem Proteasom-Komplex grundsätzlich verschieden sind, erscheint eine Beeinflussung der viralen Protease durch Proteasom-Inhibitoren zumindest theoretisch möglich, da in der Literatur berichtet wurde, dass der HIV-1-spezifische Protease-Hemmer Ritonavir die Chymotrypsin-Aktivität des 20S Proteasoms inhibiert (André *et al.,* 1998; Schmidtke *et al.,* 1999).
Im Rahmen der Erfindung wird daher ein möglicher Einfluss von Proteasom-Inhibitoren auf die enzymatische Aktivität der HIV-1-Protease durch Studien der *in vitro* Prozessierung von Gag-Polyprotein untersucht (siehe hierzu Ausführungsbeispiel 4). Erfindungsgemäß wird dazu rekombinantes Pr55 in Insektenzellen exprimiert und aus freigesetzten Virus-ähnlichen Partikeln gereinigt. Enzymatisch aktive HIV-1-Protease wird in *E. coli* exprimiert und durch Chromatographie gereinigt. Die spezifische Aktivität der Protease wird mittels Titration der aktiven Zentren ermittelt. Pr55 und Protease werden in ein Enzym-Substrat-Verhältnis von 1:25 versetzt und für eine definierte Zeit unter Bedingungen inkubiert, bei denen nur ca. 50% des Substrates Pr55 proteolytisch prozessiert werden. Diese Reaktionsbedingungen erlauben den sensitiven Nachweis von geringfügigen Effekten auf die Enzymaktivität der Protease. Selbst unter diesen sensitiven Reaktionsbedingungen und unter sehr hohen Inhibitorkonzentration von bis zu 100 microM wird keinerlei inhibitorische Wirkung von beiden Proteasom-Inhibitoren, LC und zLLL, auf die Gag-Prozessierung festgestellt (Figur 3).
Zusammengefasst zeigen die im Rahmen der Erfindung gemachten Ergebnisse, dass Proteasom-Inhibitoren die enzymatische Aktivität der HIV-Protease nicht beeinflussen und daher die virale Protease nicht das direkte Target der Inhibitoren sein kann. Die Untersuchung des zugrunde liegenden Mechanismus erlaubt die Schlussfolgerung, dass zelluläre Prozesse das Target der negativen Wirkung von Proteasom-Inhibitoren sind, welche für Gag-Prozessierung und Virusfreisetzung von HIV essentiell sind. Aufgrund der im Rahmen der Erfindung erstmals beschriebenen neuartigen Beobachtung kann somit festgestellt werden, dass im Unterschied zu bereits angewendeten anti-retroviralen Therapien (Hemmer der viralen Enzyme Protease und Reverse Transkriptase) kein viraler Faktor das direkte Target dieser neuartigen Wirkung ist. Aufgrund der Tatsache, dass ein zellulärer Mechanismus das Target der Inhibitoren darstellt, wäre bei einer *in vivo* Applikation von Proteasom-Inhibitoren zur Unterdrückung der HIV-Replikation in HIV-Infizierten die Gefahr der Resistenzentwicklung von HIV gegenüber Proteasom-Inhibitoren vergleichsweise gering oder überhaupt nicht gegeben.

### Proteasom-Inhibitoren hemmen die HIV-1-Virusreplikation in Zellkultur

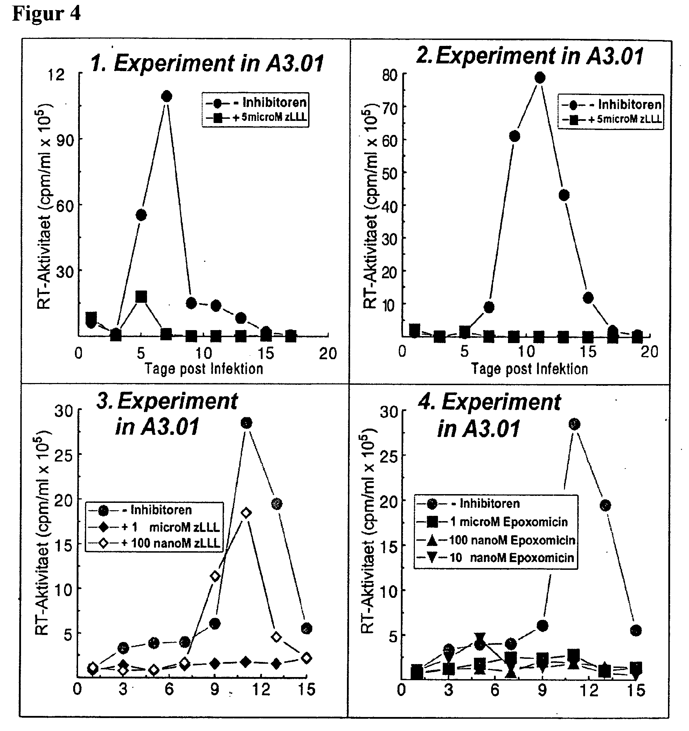

Nachdem erfindungsgemäß gezeigt werden konnte, dass Proteasom-Inhibitoren späte Prozesse der Virusfreisetzung und Gag-Prozessierung im Retrovirus-Replikationszyklus hemmen, war es wichtig, diesen negativen Effekt auch für die Infektionsausbreitung und damit den vollständigen Replikationszyklus von HIV zu demonstrieren. Aufgrund der im Rahmen der Erfindung beobachteten Phänomene, dass Proteasom-Inaktivierung eine verminderte Freisetzung von Virionen mit einer verminderten Infektiosität bewirkt, ist anzunehmen, dass die Behandlung mit Proteasom-Inhibitoren ebenfalls in einer verminderten Infektionsausbreitung in einer HIV-infizierten Kultur resultiert.
Erfindungsgemäß werden dazu parallele Kulturen einer CD4⁺ T-Zelllinie (zum Beispiel A3.01) mit einer definierten infektiösen Dosis von HIV-1 infiziert. Die Zellen werden nach Infektion in Medium mit dem oder ohne den Proteasom-Inhibitor zLLL in einer mittleren Konzentration von 5 microM über den gesamten Zeitraum der ca. 2 Wochen andauernden Kultur behandelt (siehe hierzu Ausführungsbeispiel 5). Die Produktion neuer Virionen wird durch Messung der Akkumulation von Virus-assoziierter Reverser Transkriptase-Aktivität im Zellkulturüberstand ermittelt.
Erfindungsgemäß wird dabei festgestellt, dass in Gegenwart von zLLL entweder gar keine (Fig. 4, Panel 1. Experiment in A3.01) oder nur eine sehr verminderte (Fig. 4, Panel 2. Experiment in A3.01) produktive Infektion in der Kultur etabliert werden kann. Der Abbruch der RT-Akkumulation nach ca. einer Woche Inkubation in Kulturen mit aktiver Virusreplikation ist auf die allgemein bekannte Tatsache zurückzuführen, dass mit dem Zeitpunkt der maximalen Virusreplikation die Zahl der Synzitia-Formierungen zunimmt, dadurch der HIV-induzierte Zelltod stärker ist als die Rate der Zellteilung und in Folge dessen die Gesamtkultur zum Stillstand kommt.
Um die Wirksamkeit unterschiedlicher Konzentrationen von verschiedenen Proteasom-Inhibitoren hinsichtlich Blockade der Virusreplikation zu untersuchen, werden erfindungsgemäß parallele Kulturen von infizierten Zellen mit verschiedenen Konzentrationen an zLLL (Fig. 4, Panel 3. Experiment in A3.01) und Epoxomicin (Fig. 4, Panel 4. Experiment in A3.01) behandelt. Dabei zeigt sich ein Dosis-abhängiger Effekt auf die HIV-1-Replikation: Während 100 nanoM zLLL einen relativ schwach hemmenden Effekt ausübt, bewirkt 1 microM zLLL die vollständige Hemmung der Replikation, so wie zuvor bereits für 5 microM zLLL beobachtet. Im Vergleich zu zLLL ist der sehr spezifische Proteasom-Inhibitor Epoxomicin sehr viel potenter in seiner Wirkung auf die Proteasom-Aktivität. Dies widerspiegelt sich in der erfindungsgemäß erhaltenen Beobachtung, dass 100 nanoM Epoxomicin eine vollständige sowie 10 nanoM Epoxomicin eine fast vollständige Blockade der HIV-1-Replikation bewirken (Fig. 4, Panel 4. Experiment in A3.01).

Die Blockierung der Virusreplikation in Zellkulturen kann mechanistisch als Ergebnis der im Rahmen der Erfindung beschriebenen neuartigen Aktivitäten von Proteasom-Inhibitoren wie folgt begründet werden:
- In Gegenwart der Proteasom-Inhibitoren werden weniger Virionen freigesetzt,
- die wenigen freigesetzten Virionen habe eine geringe oder gar keine Infektiosität.
Dadurch wird die Neuinfektion von Zellen in der Kultur eingeschränkt, und die Ausbreitung der Infektion ist daher vermindert oder bricht vollständig ab.
Das Wesen der Erfindung liegt in der Verwendung bekannter Mittel zu einem neuen Zweck und in einer Kombination bekannter Elemente, den Proteasom-Inhibitoren, und einer neuen Wirkung - ihrem Einsatz zur Beeinflussung von Retroviren - die in ihrer neuen Gesamtwirkung einen Vorteil und den erstrebten Erfolg ergeben, der darin liegt, dass nunmehr Mittel zur Hemmung der Freisetzung und Reifung von Retroviren zur Verfügung stehen.
Die Erfindung richtet sich ferner auf die Verwendung von Proteasom-Inhibitoren zur Herstellung von Mitteln zur Hemmung der Freisetzung, Reifung und Replikation von Retroviren. Dazu gehört ihre Verwendung zur Herstellung von Arzneimitteln zur Behandlung und Prophylaxe von AIDS und damit verwandter pathologischer Erscheinungen einer HIV-Infektion, wie zum Beispiel der HIV-induzierten Demenz, der HIV-induzierten Störungen im Lipidstoffwechsel, speziell dem HLS-Syndrom (HIV-associated lipodystrophy syndrome) sowie von HIV-induzierten Störungen der Nierenfunktionen, speziell dem HIVAN-Syndrom (HIV associated nephropathy).
Die Proteasom-Inhibitoren können auch in Kombination mit anderen Medikamenten und sonstigen Therapieschemata eingesetzt werden, z.B. Interferon alpha/beta/gamma und Varianten hiervon (zum Beispiel pegylierte Interferone), Interleukine, Nukleosidanaloga (Lamivudine, Cidovir, Ribavirin und andere), Steroide, Plasma-Austausch, Thymosin alpha 1, Impfstoffe, passive und aktive Vakzinierung, therapeutische und prophylaktische Vakzinierung, Glycyrrhizin, Stammzell-Transplantation, Organtransplantationen, Nahrungstherapie, Immunsuppressiva, Cyclosporine und Derivate hiervon, Amanditin und Derivate, Interleukine und andere Cytokine, nicht Proteasom-selektive Protease-Inhibitoren, Azathoprin, Hämodialyse, sowie hoch aktive antiretrovirale Therapie (highly active antiretroviral therapy, "HAART") bei Co-Infektionen von Hepatitis-B-Virus (HBV) mit Humanen Immundefizienzviren (HIV). Da Proteasom-Inhibitoren auch anti-virale Wirkung auf HIV ausüben, ist eine Behandlung von HBV/HIV-Koinfektionen, insbesondere in Kombination mit einer HAART-Therapie, ein Anwendungsschwerpunkt der Erfindung.

Generell beinhalten späte Prozesse des Replikatonszyklus die Neusynthese von viralen Strukturproteinen in der infizierten Zelle, die korrekte Faltung und Modifizierung sowie den Transport der Strukturprotein zu dem Ort der Virusassemblierung, gefolgt von Virusfreisetzung an der Zellmembran und der proteolytischen Prozessierung der Gag-Polyproteine im reifenden Viruspartikel durch die virale Protease. Am Beispiel der Humanen Immundefizienzviren wie auch an Hepatitis-B-Viren wurde gezeigt, dass die verschiedenen Inhibitoren des 26S Proteasoms sowohl die Freisetzung von Viruspartikeln als auch die Infektiosität der freigesetzten Viruspartikel reduzieren. Die biochemischen Untersuchungen zeigen, dass Proteasom-Inhibitoren spezifisch die Reifung und proteolytische Prozessierung der Gag-Proteine blockieren und bei Hepatitis-B-Viren auch die posttranslationale Modifikation des Nukleokapsids ändern und die Sekretion des e-Antigens reduzieren. Morphologische Untersuchungen zeigen, dass sich im Falle von HIV-1 in Gegenwart von Proteasom-Inhibitoren unreife Viruspartikel an der Zellmembran konzentrieren. Virologische Untersuchungen zeigen, dass Proteasom-Inhibitoren die Ausbreitung einer HIV-1-Infektion wie auch eine HBV-Infektion in der Zellkultur verhindern. Mechanistische Untersuchungen zeigen weiterhin, dass Proteasom-Inhibitoren keinen direkten Effekt auf die virale Protease von HIV-1 ausüben, sondern zelluläre Mechanismen beeinflussen, welche für die effiziente Reifung und Freisetzung von Viruspartikeln notwendig sind.
Die Verwendung von Proteasom-Inhibitoren stellt eine neuartige Methode zur Intervention viraler Replikationszyklen dar. Da das Target dieser Methode konservierte zelluläre Mechanismen, dass heißt die enzymatische Aktivität des Proteasom-Komplexes selbst darstellt, sind bei einer *in vivo* Applikation von Proteasom-Inhibitoren keine durch virale Mutationen vermittelte Resistenzmechanismen zu erwarten.
Die Erfindung betrifft des weiteren die Verwendung in der Grundlagenforschung, z.B. die Analyse der Assemblierung, Freisetzung und Reifung von Retroviren, speziell späte Prozesse im HIV-Replikationszyklus; ferner
- die Grundlagenforschung und angewandten Forschung,
- das Verständnis der Retrovirusassemblierung,
- das Verständnis der Wirkungsweise der viralen Proteasen,
- das Verständnis der Gag-Prozessierung von Retroviren,
- das Verständnis zellulärer Mechanismen, involviert in den späten Prozess der Retrovirus-Assemblierung, insbesondere von Faktoren des Ubiqutin-Proteasom-Systems, von Ubiquitinbindenden Faktoren,
- Fakoren, welche an ubiquitinierte Gag-Proteine von Retroviren binden,
- Fakoren, welche an mono-ubiquitinierte L(late assembly)-Domänen von retroviralen Gag-Proteinen binden,
- zelluläre Faktoren, welche die mono-Ubiquitinierung von L-Domänen von Retroviren steuern, regulieren, beeinflussen und / oder hemmen,
- zelluläre Faktoren, welche die mono-Ubiqutinierung von L-Domänen in Gag-Proteinen von Retroviren durch De-Ubiquitinierung rückgängig machen,
- zelluläre Faktoren, welche späte Prozesse der Virusassemblierung, speziell der Ablösung von Viruspartikeln von der Zellmembran in Abhängigkeit der mono-Ubiquitinierung von L-Domänen in retroviralen Gag-Proteinen steuern, regulieren, beeinflussen und / oder hemmen sowie
- das Verständnis bei der Entwicklung von weiteren Substanzen, welche die retrovirale Gag-Prozessierung sowie die Assemblierung und die Freisetzung von Retroviren durch Beeinflussung der Wechselwirkung von retroviralen Gag-Proteinen mit dem Ubiquitin-Proteasom-System steuern, regulieren, beeinflussen und / oder hemmen können.

Weitere Anwendungsgebiete der vorliegenden Erfindung sind:
- die Vorbeugung und Behandlung/Therapie von Erkrankungen, die durch Retrovirusinfektion verursacht werden, speziell die anti-retrovirale Therapie und Vorbeugung bei Infektionen mit Immundefizienz verursachenden Lentiviren, vor allem die erworbene Immundefizienz bei Tieren und Menschen, wie die Behandlung von HIV-infizierten Individuen - symptomloser wie auch AIDS-Patienten, die Vorbeugung einer HIV-Infektion und die Verhinderung einer HIV-Infektion unmittelbar nach Kontakt mit HI-Viren;
- die Entwicklung von neuen Pharmaka, speziell auf der Basis von Substanzen, die das 26S Proteasom inhibieren, wie Proteasom-Inhibitoren, die *in vivo* applizierbar sind und eine geringe Toxizität aufweisen und dabei gleichzeitig die Replikation von Retroviren im Organismus unterdrücken und die Infektionsausbreitung verhindern;
- die Applikation von retroviralen Vektoren für den Einsatz bei Gentransfermethoden in der Gentherapie, speziell der Anwendung von Proteasom-Inibitoren zur Verhinderung der Entstehung und Ausbreitung rekombinanter und/oder ungewollter replikationskompetenter Retroviren nach Gentransfer;
- die Virologie, Zellbiologie, Gentherapie, Pharmakologie, Naturstoffchemie, Peptidchemie, molekulare HIV- und angewandte AIDS-Forschung.

Die Erfindung soll anhand von Ausführungsbeispielen näher erläutert werden, ohne auf diese Beispiele beschränkt zu sein.

### Ausführungsbeispiele

### Beispiel 1: Behandlung von HIV-1 infizierten Zellen mit Proteasom-Inhibitoren reduziert die Infektiosität von freigesetzten Viruspartikeln.

Kulturen von humanen CD4⁺ T-Zellen, A3.01 wurden mit HIV-1_{NL4-3} infiziert und für ca. 7 Tage in RPMI kultiviert. Ca. 80% des Zellkulturmediums wurde alle zwei Tage erneuert. Zur Bestimmung der Menge an freigesetzten Viruspartikeln wurden Proben der Zellkulturüberstände auf Aktivität von Virus-assoziierter RT untersucht. Weiterhin wurde die Infektionsausbreitung durch indirekte Immunfluoreszenz mit anti-CA Antikörpern verfolgt. Der Zeitpunkt der maximalen Infektionsausbreitung in der Kultur wurde anhand der RT-Akkumulation sowie der Synzytia-Formierung ermittelt. Zum Zeitpunkt der maximalen Virusproduktion (ca. 7 Tage nach Infektion) wurden frische nicht infizierte A3.01 Zellen der infizierten Kultur im Verhältnis 1:1 gemischt und für weitere 24 h inkubiert. Dies ermöglicht eine quasi-Synchronisation der Infektion und eine maximale Zahl an infizierten Zellen mit maximaler HIV-Expression zu einem gegebenen Zeitpunkt. Die Zellen wurden in parallele Kulturen aufgeteilt, mit PBS gewaschen und für 4.5 Stunden mit 40 microM zLLL im Medium behandelt. Das Waschen der Zellen und die Kultivierung in frischem Medium war notwendig, um die neuproduzierten Viren während der 4.5 Stunden Erntezeit zu verfolgen (+zLLL "0hr"). Es bestand die Annahme, dass eine gewisse, der HIV-1 Assemblierung entsprechende, ca. eine Stunde lange lack-Phase besteht zwischen dem Beginn der Behandlung mit Proteasom-Inhibitoren und der Freisetzung defekter Virionen. Um die Virus-Produktion von Zellen mit inaktivierten Proteasomen zum Beginn der Erntezeit zu verfolgen, wurden parallele Kulturen für eine (+zLLL "-1hr") oder 6 Stunden (+zLLL "-6hr") vor dem Wasch-Schritt mit 40 microM zLLL vorbehandelt. Eine parallele Kultur wurde ohne Inhibitor inkubiert (kein zLLL). Die Virus-haltigen Zellkulturüberstände wurden gesammelt, filtriert, und die Menge an Gag-Proteinen wurde mittels eines standardisierten Capture-ELISA unter Verwendung von Epitop-differenten anti-CA-Antikörpern quantifiziert. Die daraus kalkulierte Virusfreisetzung ist als nanog p24^{CA}/ml Zellkulturmedium in Tabelle 1 dargestellt. Die Infektiosität der Kulturüberstände wurde mittels Endpunkttitration (detailliert in Beispiel 6d) ermittelt und ist als TC_{ID50} in Tabelle 1 angegeben. Die spezifische Infektiosität wurde als der Quotient von TC_{ID50} zur Gesamt-Menge an Gag ermittelt und ist als prozentualer Anteil der Infektiosität zur Kontroll-Kultur (kein zLLL = 100%) in Tabelle 1 angegeben. Dabei zeigt sich, dass sowohl die Freisetzung an Gag-Proteinen als auch die Infektiosität der freigesetzten Virionen nach zLLL-Behandlung gehemmt wird. Dieser Effekt nimmt mit der Zeit zu, nach 4.5 Stunden tritt eine 84%ige und nach weiteren 6 Stunden Vorbehandlung eine 98%ige Reduktion an infektiösen Virionen im Zellkulturmedium auf (Tabelle 1).

### Tabelle 1:

Proteasom-Inhibitoren verringern die Infektiosität von freigesetzten Viruspartikeln.

CD4+ T-Zellen (A3.01) wurden mit HIV-1NL4-3 infiziert und zum Zeitpunkt der maximalen Virusproduktion (ca. 7 Tage post Infektion) parallele Kulturen entweder ohne oder für 1 (+zLLL "-1hr") beziehungsweise 6 Stunden (+zLLL "-6hr") mit 40 microM zLLL behandelt. Danach wurden die Zellen gewaschen und für weitere 4.5 Stunden mit oder ohne 40 microM zLLL inkubiert. In einer parallelen Kultur wurden Zellen unmittelbar nach dem Waschen mit 40 microM zLLL behandelt (+zLLL "0hr"). Die Virus-haltigen Überstände wurden gesammelt, und die Menge an CA-Antigen wurde mittels ELISA quantifiziert. Die spezifische Infektiosität wurde als der infektiöse Virus-Titer per nanog CA ermittelt und im Verhältnis zu der nicht behandelten Kontroll-Kultur (100%) dargestellt.

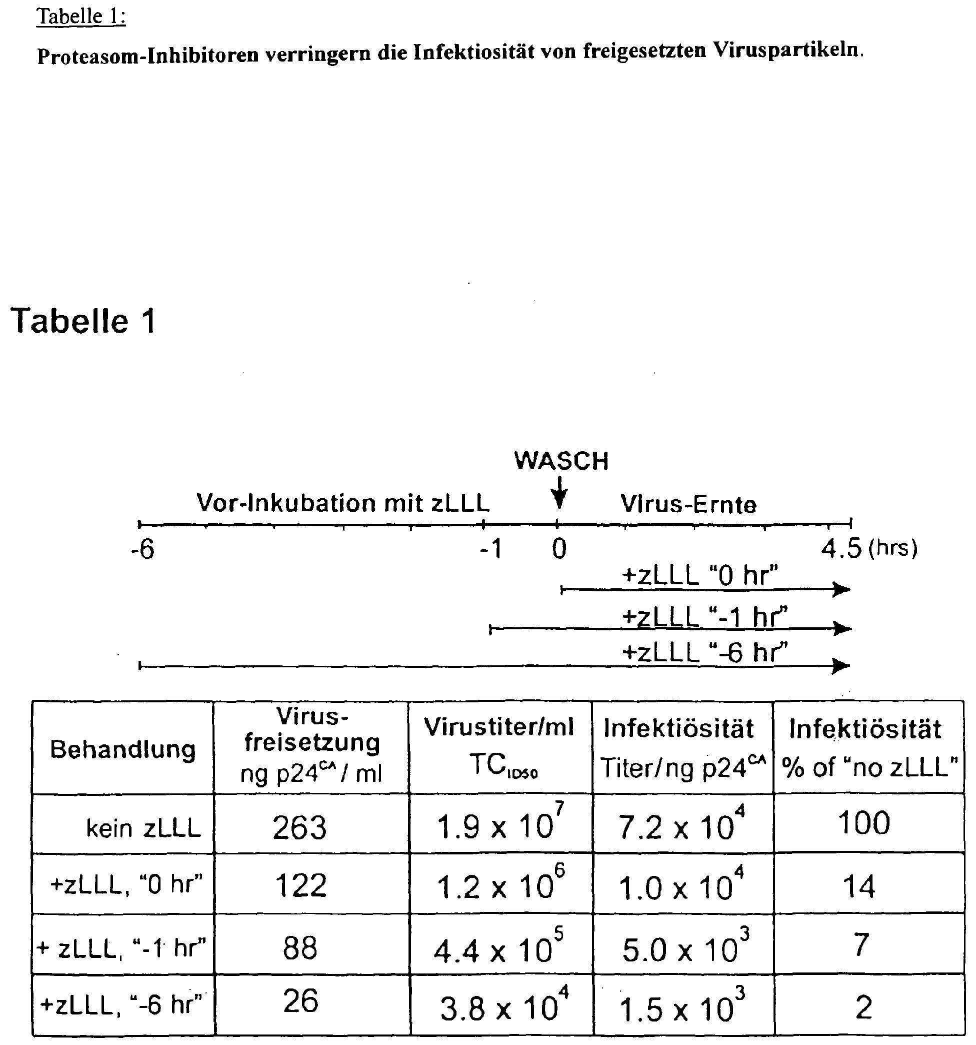

### Beispiel 2: Elektronenmikroskopische Analyse HIV-1 infizierter MT-4 Zellen nach Behandlung mit Proteasom-Inhibitoren.

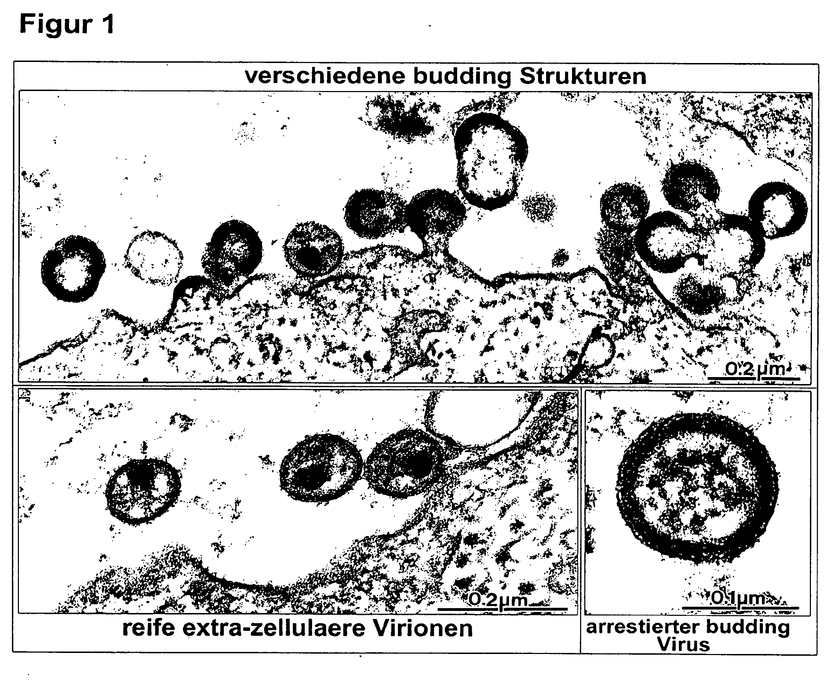

CD4⁺ T-Zellen, MT-4, wurden mit HIV-1_{NL4-3} infiziert und für ca. 4 Tage in RPMI kultiviert. Zum Zeitpunkt der maximalen Virusproduktion wurden die Zellen gewaschen, in Zellulose-Kapillaren gesaugt und für 5 Stunden mit 50 µM (microMol) zLLL behandelt. Die experimentellen Details der Fixierung, der Dünnschnittpräparation und der Transmissionselektronenmikroskopie sind in Beispiel 6e aufgeführt. Repräsentative Ausschnitte von elektronenmikroskopischen Aufnahmen sind in Figur 1 dargestellt.

### Beispiel 3: Proteasom-Inhibitoren hemmen Gag-Prozessierung und Virusfreisetzung von infizierten T-Zellkulturen und transfizierten HeLa-Zellen.

Zur biochemischen Analyse der hemmenden Wirkung von Proteasom-Inhibitoren auf die Kinetik der Gag-Prozessierung und Virusfreisetzung wurden pulse/chase-Analysen durchgeführt. Die experimentellen Details der Infektion, Kultivierung, DNA-Transfektion und pulse/chase-Experimente sind in Beispiel 6g angegeben. Hierzu wurden entweder HIV-1 infizierte CD4⁺ T-Zellkulturen oder Kulturen von HeLa-Zellen eingesetzt, welche mit proviraler DNA von HIV-1_{NL4-3} oder HIV-2_{ROD10} transfiziert wurden. In der Regel wurden parallele Kulturen zum Zeitpunkt der maximalen Expression von HIV-Proteinen für 30 min einer Methionin-Depletion unterzogen, anschließend für 30 min mit [³⁵S]-markierten Methionin metabolisch pulse-markiert und nachfolgend in einem chase-Medium mit einem Überschuss an nicht-radioaktiv markiertem Methionin für einen Zeitraum von 8 Stunden inkubiert. Die Behandlung mit Proteasom-Inhibitoren begann in der Regel mit dem Zeitpunkt der Methionin-Depletion und wurde über den gesamten Versuchszeitraum (Depletions-, pulse-, und chase-Phase aufrecht erhalten). Proteasom-Inhibitoren wurden entweder selektiv (10 microM zLLL, Figur 2, Panel "HIV-1 in HeLa") oder in Kombination (10 microM zLLL und 10 microM LC, Abbildungen 2, Panels "HIV-1 in HeLa" und "HIV-2 in HeLa") eingesetzt. Aliquote Zellkulturen wurden zu jedem Zeitpunkt des chases gewonnen und durch Zentrifugation in Zell-, Virus- und Zellkulturüberstand-Fraktionen aufgetrennt. Die radioaktiv markierten HIV-Proteine wurden mittels Standard-Immunpräzipitation unter Verwendung von AIDS-Patientenseren sowie Gagspezifischen Antikörpern unter Verwendung von bereits beschriebenen Methoden (Schubert *et al.*, 1998) isoliert, im SDS-PAGE aufgetrennt, und anschließend durch Fluorographie sichtbar gemacht. Die Quantifizierung erfolgte mittels Image-Analyse. In der Regel wurden die relativen Mengen an Gag-Polyprotein und dem Hauptprozessierungsprodukt CA (capsid) für jeden chase-Zeitpunkt jeweils in der Zell-, Virus- und der Zellkultur-Fraktion bestimmt. Die Kinetik von Virusfreisetzung wurde als der prozentuale Anteil von Gag-Proteinen in der Virus-Fraktion relativ zu der Gesamt-Menge an Gag (ermittelt in den Zell-, Virus-, und Zellkultur-Fraktionen) pro Zeitpunkt des chases dargestellt. Die Kinetik der intrazellulären Gag-Prozessierung wurde als der Quotient der Mengen von CA durch Pr55 über den gesamten chase-Zeitraum berechnet (Figur 2, Panel "HIV-1 in HeLa").

### Beispiel 4: In vitro Gag-Prozessierung von Pr55.

Rekombinantes HIV-1 Gag-Polyprotein Pr55 wurde in Insektenzellen und rekombinante HIV-1 Protease wurde in *E. coli* hergestellt. Die experimentellen Details der Expression, Reinigung, und der Bestimmung der enzymatischen Aktivität wie auch der Durchführung der *in vitro* Spaltreaktionen und Western blots sind im Beispiel 6f näher erläutert. Die Enzym-Substrat-Verhältnisse (Protease-Pr55-Verhältnis) wurde so gewählt, dass eine relativ langsame Substrat-Umsetzung auftrat. Nach 30 min Reaktion wurden ca. 50% von Pr55 gespalten. Unter diesen Bedingungen können selbst schwach hemmende Effekte auf die enzymatische Aktivität der Protease ermittelt werden. Unter diesen sensitiven Bedingungen konnte keine Inhibierung der Protease-Aktivität festgestellt werden, selbst nicht unter Bedingungen, bei denen beide Inhibitoren, zLLL und LC, in einer 10-fach höheren Konzentration als in der Zellkultur getestet wurden (Fig. 3, Reaktionen 4-9). Als Kontrolle: Der HIV-1 spezifische Proteasom-Inhibitor Saquinavir bewirkt einen vollkommenen Block der *in vitro* Prozessierung (Fig. , Reaktion 10).

### Beispiel 5: Proteasom-Inhibitoren hemmen HIV-1 Replikation in Zellkultur.

Parallel-Kulturen von CD4⁺ T-Zellen wurden in frischem RPMI-Medium inkubiert und mit einem definierten Virus-Stock von gereinigtem HIV-1_{NL4-3} infiziert. In der Regel wurde 1 RT-Unit pro Zelle zur Infektion eingesetzt. Einen Tag nach der Infektion wurden die Zellen in PBS gewaschen, mit frischem Medium versetzt, welches verschiedene Konzentrationen an den Proteasom-Inhibitoren zLLL (Fig. 4, Panels "1. bis 3. Experiment in A3.01 ") oder Epoxomicin (Fig. 4, Panel "4. Experiment in A3.01 ")
enthielt. Alle zwei Tage wurden Proben von Zellkulturüberständen gewonnen, eingefroren und später zur Bestimmung der RT-Aktivität eingesetzt. Gleichzeitig wurden 80% des Zellkultur-Mediums erneuert und mit frischen Proteasom-Inhibitoren versetzt. Die Proteasom-Inhibitoren wurden als 10 mM (milliMol) Stock-Lösungen in 75% Ethanol eingesetzt. Als Negativ-Kontrolle wurde eine parallele Kultur mit 20 microM Ethanol versetzt. Die Reverse Transkriptase-Aktivität wurde in den zellfreien Zellkulturüberständen bestimmt und gegen die Zeit der Zellkultur aufgetragen (Fig. 4).

### Beispiel 6: Material und Methoden.

### Beispiel 6a: Molekulare HIV-1-Klone.

Zur Herstellung von T-Zell-tropen Viren des molekularen Klons HIV-1_{NL4-3} wurde das bereits publizierte Plasmid pNL4-3 (Adachi *et al.,* 1986) verwendet und für die Herstellung von Makrophagen-tropen Viren das bereits publizierte Plasmid pNL4-3(AD8) (Schubert *et al.,* 1995) und pAD8 (Theodore *et al.,* 1995). Für die Expression von HIV-2_{ROD10} in HeLa-Zellen wurde das bereits publizierte Plasmid pROD10 (Bour *et al.,* 1996) verwendet.

### Beispiel 6b: Zellkultur.

CD4⁺ humane T-Zell-Lymphom-Zelllinien, H9, A3.01, C8166, und MT4, wurden in RPMI 1640 mit 10% (^{v}/ᵥ) fötalem Kälberserum, 2 milliM L-Glutamin, 100 U ml⁻¹ Penicillin und 100 mg (millig) ml⁻¹ Streptomycin kultiviert. Hela-Zellen (ATCC CCL2) wurden in Dulbeccos' modifizierten Eagle's Medium (DMEM) mit 10% fötalem Kälberserum, 2 milliM L-Glutamin, 100 U ml⁻¹ Penicillin, und 100 millig ml⁻¹ Streptomycin kultiviert.

### Beispiel 6c: Transfektion und Gewinnung von Virusstocks.

Für die Herstellung von Virus-Präparaten wurde Plasmid-DNA von molekularer HIV-1- oder HIV-2-DNA unter Anwendung der Kalzium-Phosphat-Präzipitationsmethode in HeLa-Zellen transfiziert. Dazu wurden konfluente Kulturen von HeLa-Zellen (5x10⁶ Zellen) mit 25 µg (microg) Plasmid DNA in Kalziumphosphat-Kristallen hergestellt, inkubiert und anschließend einem Glycerol-Schock unterzogen. Für die Gewinnung von konzentrierten Viruspräparaten wurden zwei Tage nach Transfektion die Zellkulturüberstände geerntet. Anschließend wurden die Zellen sowie deren Bestandteile durch Zentrifugation (1,000 x g, 5 min, 4°C) und Filtration (0.45 µm - microm Porengröße) abgetrennt. Viruspartikel wurden durch Ultra-Zentrifugation (Beckman SW55 Rotor, 1.5 hr, 35,000 rpm, 10°C) pelletiert und nachfolgend in 1 ml of DMEM Medium resuspendiert. Die Viruspräparate wurden durch Filtration sterilisiert (0.45 microm Porengröße) und portioniert eingefroren (-80°C). Einzelne Viruspräparate wurden durch Bestimmung der Reverse Transkriptase-Aktivität unter Verwendung von [³²P]-TTP Einbau in ein oligo(dT)-poly(A) Template standardisiert.

### Beispiel 6d: Infektiositätsassay.

Akute mit HIV-1_{NL4-3} infizierte A3.01-Zellen wurden mit 40 microM zLLL für verschiedene Zeiten behandelt, wie im Ausführungsbeispiel 1 angegeben. Zellfreier Zellkulturüberstand wurde geerntet und filtriert (Poren-Größe = 0.45 microM). Der relative Betrag an freigesetzten Virionen wurde mittels eines p24^{CA} -Antigen Capture ELISA quantifiziert. Zum Zweck der Virus-Titration wurden jeweils 8 parallele Kulturen von C8166 Zellen infiziert mit den jeweiligen Virus-Präparaten, in einer seriellen 10-fachen Verdünnung infiziert. Der Infektiositätstiter wurde bestimmt als 50% Zellkultur infektiöse Dosis (TCID₅₀), und zwar indem die Synzytia-Formierung je Kultur und Verdünnungsstufe am Tag 10 post infectionem ermittelt wurde. Die spezifische Infektiosität wurde für jede Probe standardisiert zu dem Betrag an ermittelten p24^{CA} Antigenen in jedem Virus-Inokulum.

### Beispiel 6e: Elektronenmikroskopie.

Akut mit HIV-1_{NL4-3} infizierte MT-4 Zellen wurden für 2.5 Stunden in frischem Medium mit bzw. in einer parallelen Kultur ohne Proteasom-Inhibitor (50 microM zLLL) kultiviert. Anschließend wurden die Zellen in Zellulose-Kapillaren gesaugt, die Kapillaren wurden verschlossen und für weitere 2.5 Stunden in frischem Medium mit oder ohne 50 microM zLLL weiter kultiviert. Die Kapillaren wurden gewaschen, und die Zellen wurden für eine Stunde in 2.5% Glutaraldehyd in Phosphat gepufferter Saline (PBS) inkubiert. Anschließend wurden die Kapillaren in PBS gewaschen und nachfixiert in 1% OsO₄ in PBS, erneut gewaschen in Wasser, gefärbt in 1% Uranyl-Acetat in Wasser, sowie abschließend dehydratisiert in einem steigenden Gradienten an serieller Verdünnung von Ethanol. Die gesamten Kapillaren wurden in ERL-Harz eingebettet für nachfolgende ultra-Dünnschnitte. Die Ultra-Dünnschnitte wurden gegengefärbt mit 2% Uranyl-Acetat und Blei-Zitrat. Mikro-Aufnahmen wurden mit einem Philips CM120 Transmissions-Elektronenmikroskop bei 80 kV aufgenommen.

### Beispiel 6f: In vitro Prozessierung von Gag-Proteinen.

Myristyliertes Pr55 von HIV-1 wurde aus Virus-ähnlichen Partikeln produziert, welche von Insektenzellen infiziert mit rekombinanten Baculovirus Gag12myr freigesetzt wurden. Die Virus-ähnlichen Partikel wurden durch Zentrifugation durch ein 20% Sucrose-Kissen gereinigt. Rekombinante HIV-1 Protease exprimiert in *E. coli* wurde mittels Gelfiltrations-Chromatographie an Superose12 gereinigt. Die Enzymkonzentration wurde mittels active-site-Titration ermittelt. Für Spaltreaktionen wurde allgemein 1 µM Pr55 mit 40 nM (nanoMol) PR in Reaktionspuffer (50 mM MES pH 6.5, 300 mM NaCl, 2 mM DTT, 1 mM EDTA, 0.1 % Triton-X 100) bei 37°C für 30 min inkubiert. Um eine maximal Sättigung von PR mit Inhibitoren zu garantieren, wurde PR mit den jeweiligen Inhibitor für 5 min vor dem Start der Spaltreaktionen vorinkubiert. Die Spaltreaktionen wurden durch Zugabe von SDS-sample Puffer und Kochen der Probe für 10 min gestoppt. Die Spaltprodukte wurde im 10% SDS-PAGE aufgetrennt und anschließend mittels Western blot nachgewiesen. Dazu wurden anti-CA spezifische Antikörper eingesetzt, die mittels anti-Kanichen-IgG-Peroxidase-Konjugat und Chemiluminiszenz-Reaktion nachgewiesen wurden.

### Beispiel 6g: Metabolische Markierung, pulse/chase-Analyse und Immunpräzipitation.

Für pulse/chase-Analysen wurden HIV-1 oder HIV-2 exprimierende Zellen, entweder infizierte T-Zellen oder transfizierte HeLa-Zellen, für 30 min in Methionin-freiem RPMI-Medium inkubiert (Methionin-Depletion). Danach folgte eine metabolische pulse-Markierung mit [³⁵S]-Methionin (spezifische Aktivität = 2 milliCi/ml) in RPMI für 30 min. Die Zellen wurden in PBS gewaschen, aliquotiert und in parallelen Kulturen in komplettem DMEM mit 10 milliM nicht radioaktivem Methionin weiter inkubiert (chase). Zu bestimmten Zeitpunkten des chases (in der Regel 0, 0.5, 1, 2, 4 und 8 Stunden nach pulse-Markierung) wurden die Zellen geerntet und unmittelbar danach auf Trockeneis eingefroren. Die zellfreien Virionen wurden isoliert durch Zentrifugation der Zellkultur-Überstände (bei 4°C und 18,000xg, für 100 min). Extrakte von Zell- und Viruspellets wurden mittels Detergenzien hergestellt und Aliquote von der Zell-, der Virus-, und der Zellkultur-Überstände, erhalten nach Zentrifugation, wurden mit Antikörpern behandelt, die zuvor an Protein-G-Sepharose gekoppelt wurden. In der Regel wurde Serum von HIV-1 oder HIV-2 seropositiven Individuen wie auch anti-CA Antikörper, hergestellt in Kaninchen, für die Immunpräzipitation eingesetzt. Die Immunpräzipitate wurden mit Detergenzhaltigen Puffern gewaschen, in SDS-Probenpuffer denaturiert und anschließend mittels Gelelektrophorese in 10% SDS-PAGE aufgetrennt. Die Gele wurden in Lösungen von 50% Methanol und 10% Essigsäure fixiert und anschließend für 1 Stunde in 1 M Salizylsäure behandelt. Die radioaktiv markierten Proteine wurden durch Fluorographie bei -80°C sichtbar gemacht. Die relative Menge der markierten Proteine, speziell der Gag-Polyproteine und der CA-Prozessierungsprodukte wurden mittels Phosphor-Image-Analyzer bestimmt.

### Beispiel 7: Effekt von Proteasom-Inhibitoren auf die Sekretion von HBV-spezifischen Antigenen in humanen Hepatoma-Zelllinien.

Primäre humane Hepatozyten wurden aus menschlichen Lebern gewonnen (Dandri *et* al*.,* 2001) und mit humanen Hepatitis-B-Viren (HBV) infiziert, welche aus dem Medium von HepG2.2.15-Zellen, die infektiöses humanes Hepatitis-B-Virus produziert (Sells *et al.,* 1987), geerntet wurden. Parallele Kulturen der HepG2-Zellen wurden für zwei Tage mit PI behandelt, oder nicht behandelt. Die HepG2-Zellen wurden vor der Behandlung mit replikationskompeteten HBV-Genomen wie beschrieben (Kalinina *et al.,* 2001) transfiziert. Im Gegensatz zu zahlreichen HBV Core-positiven primären Hepatozyten (analysiert durch indirekte Immunfluoreszenzfärbung mit anti-Core Antikörpern), welche auf eine erfolgreiche Infektion von primären humanen Hepatozyten mit Viren aus den nicht behandelten Zellen schließen lassen, wurde keine Corepositive primäre humane Hepatozyten nach Infektion mit Nachkommenviren aus Proteasom-Inhibitor-behandelten HepG2 Zellen gefunden. Im Ergebnis dieses Experimentes kann damit festgestellt werden, dass Proteasom-Inhibitor-Behandlung ähnlich der Wirkung auf DHBV ebenfalls die Freisetzung und Infektiosität von HBV blockiert.
Generell beinhalten späte Prozesse des Replikatonszyklus die Neusynthese von viralen Strukturproteinen in der infizierten Zelle, die korrekte Faltung und Modifizierung sowie den Transport der Strukturprotein zu dem Ort der Virusassemblierung, gefolgt von Virusfreisetzung an der Zellmembran und der proteolytischen Prozessierung der Gag-Polyproteine im reifenden Viruspartikel durch die virale Protease. Am Beispiel der Humanen Immundefizienzviren wie auch an Hepatitis-B-Viren wurde gezeigt, dass die verschiedenen Inhibitoren des 26S Proteasoms sowohl die Freisetzung von Viruspartikeln als auch die Infektiosität der freigesetzten Viruspartikel reduzieren. Die biochemischen Untersuchungen zeigen, dass Proteasom-Inhibitoren spezifisch die Reifung und proteolytische Prozessierung der Gag-Proteine blockieren und bei Hepatitis-B-Viren auch die posttranslationale Modifikation des Nukleokapsids ändern und die Sekretion des e-Antigens reduzieren. Morphologische Untersuchungen zeigen, dass sich im Falle von HIV-1 in Gegenwart von Proteasom-Inhibitoren unreife Viruspartikel an der Zellmembran konzentrieren. Virologische Untersuchungen zeigen, dass Proteasom-Inhibitoren die Ausbreitung einer HIV-1-Infektion wie auch eine HBV-Infektion in der Zellkultur verhindern. Mechanistische Untersuchungen zeigen weiterhin, dass Proteasom-Inhibitoren keinen direkten Effekt auf die virale Protease von HIV-1 ausüben, sondern zelluläre Mechanismen beeinflussen, welche für die effiziente Reifung und Freisetzung von Viruspartikeln notwendig sind.

### Legende zu den Figuren

Figur 1: Elektronenmikroskopische Analyse HIV-1-infizierter CD4⁺ T-Zellen nach Behandlung mit PI.
   MT-4-Zellen wurden mit HIV-1_{NL4-3} infiziert und zum Zeitpunkt der maximalen Virusproduktion (ca. 4 Tage post infectionem) mit 50 µM zLLL für 5 Stunden behandelt. Zellen wurde in Zellulose-Kapillaren fixiert und für Dünnschnitt-Mikroskopie präpariert. In Panel "verschiedene Buddingstrukturen" ist ein Überblick von infizierten Zellen mit Virus-Buddingstrukturen von reifen und unreifen Viren dargestellt. Panel "reife extrazelluläre Virionen" zeigt reife extrazelluläre HIV-1-Partikel, und Panel "arrestierter budding Virus" zeigt eine hoch auflösende Vergrößerung von unreifen Partikeln, welche noch mit der Zellmembran verbunden sind.
Figur 2: Proteasom-Inhibitoren hemmen Gag-Prozessierung und Virusfreisetzung von HIV-1 und HIV-2 in infizierten und transfizierten Zellen.
   (A) HeLa-Zellen wurden mit dem HIV-1-proviralen infektiösen DNA-Klon pNL4-3 transfiziert. Nach 24 Stunden wurden parallele Ansätze der transfizierten Zellen mit Proteasom-Inhibitoren (10 microM zLLL und 10 microM LC (+INHIBITOREN)) im Medium oder ohne Inhibitoren (NO INHIBITOR) behandelt und einem pulse/chase-Experiment unterzogen. Radioaktiv mit [³⁵S]-Methionin markierte virale Proteine wurden mittels Immunpräzipitation aus der zellulären Fraktion (ZELLE), der pelletierten Virus-Fraktion (VIRUS) sowie der nach Zentrifugation erhaltenen Virus-freien Überstand-Fraktion (FREIES PROTEIN) isoliert und in einem 10% SDS-PAGE aufgetrennt. Die radioaktiv markierten Proteine wurden anschließend durch Fluorographie sichtbar gemacht. Die relative Konzentration dieser Proteine wurde mittels Image-Analyse quantitativ ausgewertet. Repräsentative Ausschnitte der Fluorogramme im Molekulargewichtsbereich von CA und Gag-Polyprotein Pr55 (ca. 20 bis 60 kDa) sind in Panel "HIV-1 in HeLa" dargestellt. Positionen von dem Hauptprozessierungsprodukt p24^{CA} sowie einem intermediären Spaltprodukt, p25^{CA} sind extra markiert. Die Kinetik der Virusfreisetzung wurde als der prozentuale Anteil von Gag-Proteinen in der Virus-Fraktion relativ zu der Gesamt-Menge an Gag (in ZELL, VIRUS, und FREIES PROTEIN) pro Zeitpunkt des chases dargestellt. Die Kinetik der intrazellulären Gag-Prozessierung wurde als der Quotient der Mengen von CA durch Pr55 über den gesamten chase-Zeitraum dargestellt. Es tritt eine deutliche, ca. 6-fache Verzögerung in der Gag-Prozessierung sowie der Virusfreisetzung innerhalb des 8-stündigen chase-Zeitraumes auf. Ebenfalls ist deutlich die Akkumulation unvollständiger Spaltprodukte, wie zum Beispiel von p25^{CA}, in den ZELL- und den Virus-Fraktionen zu erkennen. In den pulse/chase-Experimenten zu dem Panel "HIV-1 in A3.01" wurden CD4⁺ T-Zellen mit HIV-1_{NL4-3} infiziert, und die Infektionsausbreitung in der Kultur wurde durch Bestimmung der RT-Aktivität im Zellkulturüberstand verfolgt. Zum Zeitpunkt der maximalen Virusreplikation, ca. 7 Tage nach der Infektion, wurden parallele Ansätze der Kultur mit Proteasom-Inhibitor (10 microM zLLL (+INHIBITOR)) im Medium oder ohne Inhibitor (NO INHIBITOR) behandelt und einem pulse/chase-Experiment gefolgt von Immunpräzipitation und SDS-PAGE unterzogen, ähnlich dem in Fig. 2, Panel "HIV-1 in HeLa" dargestellten Experiment. Die Positionen der Hauptstrukturproteine von Env (gp160 und gp120) sowie von Gag (Pr55 und CA) sind in den Fluorogrammen der jeweiligen SDS-PAGE-Analysen angezeigt. Es ist deutlich zu erkennen, dass selbst bei einer vergleichsweise niedrigen Konzentration von 10 microM zLLL eine deutliche Verzögerung der Kinetik der intrazellulären Gag-Prozessierung sowie der Freisetzung von Virus-assoziiertem und freiem Gag auftritt. Gleichzeitig tritt eine Akkumulation von intermediären Prozessierungsprodukten im Bereich von ca. 40 kDa auf, insbesondere in der VIRUS-Fraktion.
   In dem pulse/chase-Experiment zu der Figur 2, Panel "HIV-2 in HeLa", wurden HeLa-Zellen mit dem HIV-2-proviralen infektiösen DNA-Klon pHIV-2_{ROD10} transfiziert. Nach 24 Stunden wurden parallele Ansätze der transfizierten Zellen mit Proteasom-Inhibitoren (10 microM zLLL und 10 microM LC (+INHIBITOREN)) im Medium oder ohne Inhibitoren (NO INHIBITOR) behandelt und einem pulse/chase-Experiment gefolgt von Immunpräzipitation und SDS-PAGE unterzogen, ähnlich dem vorher in Figur 1 dargestellten Experiment. Repräsentative Ausschnitte der Fluorogramme im Molekulargewichtsbereich von CA und Gag-Polyprotein Pr55 (ca. 20 bis 60 kDa) der ZELL- und VIRUS-Fraktionen sind in Fig. 2, Panel "HIV-2 in HeLa", dargestellt. Positionen der Hauptprozessierungsprodukte p27^{CA} und dem Gag-Polyprotein Pr58 sind angezeigt. Eine starke Reduktion der intrazellulären und Virus-assoziierten Gag-Prozessierung sowie eine deutliche Reduktion der Virusfreisetzung ist zu beobachten.
Figur 3: Proteasom-Inhibitoren haben keinen Einfluss auf Gag-Prozessierung von Pr55 durch virale Protease *in vitro*.
   Rekombinantes Pr55 wurde aus Virus-ähnlichen Partikeln isoliert, welche von Insektenzellen infiziert mit rekombinanten Bacculovirus produziert wurden. Rekominante HIV-1-Protease wurde in *E*. *coli* exprimiert, gereinigt und die spezifische Aktivität durch aktive-site-Titration bestimmt. Die Mengen an Pr55 und Protease wurde in einem Enzyme-zu-Substrat-Verhältnis von 1:25 gemischt und für 30 min bei 37°C inkubiert. Die Reaktion wurde durch Zugabe von SDS-Probepuffer gestoppt. Die Spalt-Reaktionen wurden anschließend durch Western blot-Analyse mit CA-spezifischem Antiserum untersucht. Pr55, CA und intermediäres Prozessingprodukt MA-Ca wurden nach Antikörperbindung mittels Chemilumineszenz-Reaktion sichtbar gemacht. In den Reaktionen 3 bis 10 wurde die Protease vor Beginn der Spaltreaktion mit den jeweiligen Proteasom-Inhibitoren 5 min vor Zugabe des Substrates Pr55 vorinkubiert. In Reaktion 10 wurde der HIV-1-Protease spezifische Inhibitor Saquinavir zugegeben.
Figur 4: Proteasom-Inhibitoren hemmen HIV-1 Replikation in Zellkultur.
   Parallele Kulturen von A3.01-Zellen wurden mit vergleichbaren infektiösen Dosen an HIV-1_{NL4-3} infiziert und mit 5 µM (Panels "1. und 2. Experiment in A3.01 ") behandelt. In einem parallelen Versuch wurden Kulturen mit unterschiedlichen Konzentrationen an zLLL (0.1 und 1 µM, Panel "3. Experiment in A3.01 ") sowie Epoxomicin (10 nM bis 1 µM, Panel "4. Experiment in A3.01 ") behandelt. Die Sekretion von reverser Transkriptaseaktivität in das Zellkulturmedium wurde im Verlauf des Virus-Replikationsprofiles (ca. 2 Wochen Kultur) dargestellt.

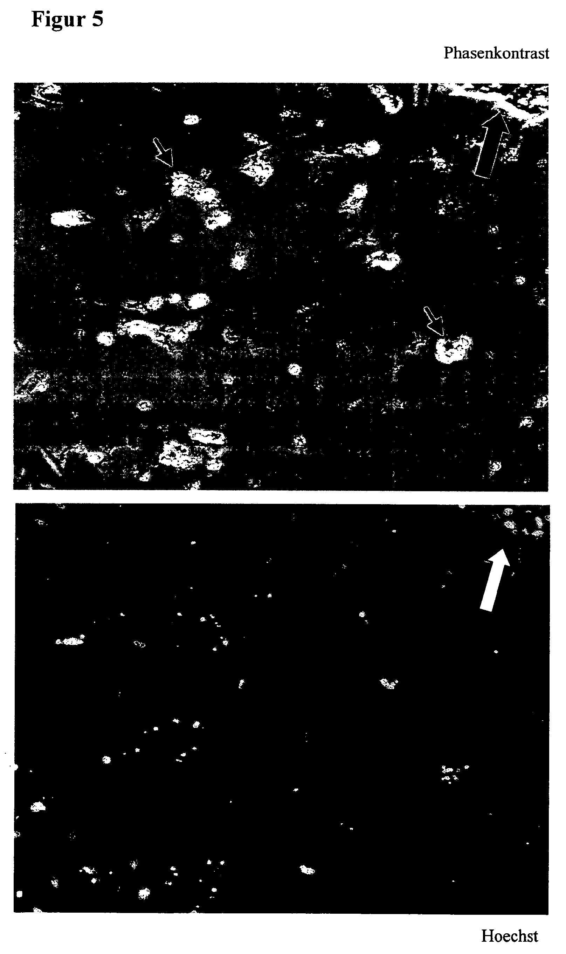

Figur 5: Nichtparenchymale Zellen einer primären Hepatozytenkultur sind sensitiver gegenüber den toxischen Effekten von Proteasom-Inhibitoren.
   Gezeigt ist eine primäre Hepatozytenkultur, welche mit 1 microM PI für 72 h behandelt worden ist. Anschließend wurden die Zellen fixiert und die Kerne mit Hoechst gefärbt (blau). Die korrespondierende Phasenkontrastaufnahme ist gegenüberstellt. Das obere Phasenkontrastbild zeigt eine ehemalige Insel aus Nicht-Hepatozyten, während das untere die gefärbten Kerne desselben Bildausschnittes wiedergibt. Die kleinen Pfeile zeigen auf die apoptotischen nichtparenchymalen Zellen, während der Blockpfeil auf eine intakte, kleine Hepatozytenkolonie zeigt.

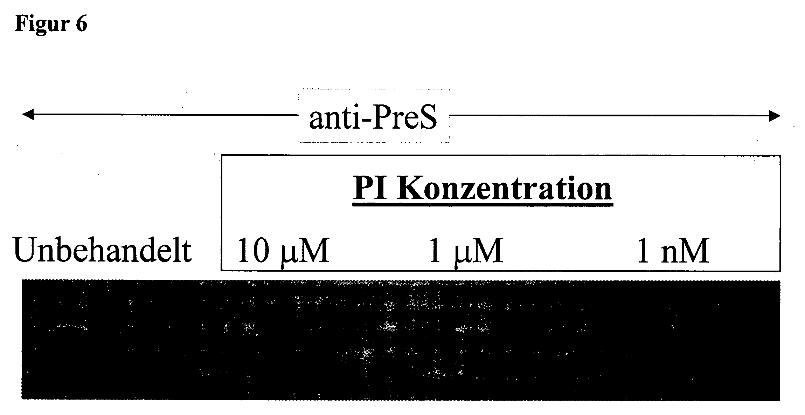

Figur 6: Proteasom-Inhibitoren hemmen konzentrationsabhängig die Freisetzung von PreShaltigen Viruspartikeln in chronisch DHBV-infizierten primären Entenhepatozyten - PreS dotblot- Nachweis.
   Eine 7 Tage alte, chronisch DHBV-infizierte primäre Entenhepatozytenkultur wurde für 48 h mit steigenden Konzentrationen von PI (1 nanoM, 1 microM und 10 microM) behandelt bzw. nicht behandelt. 200 microl der jeweiligen Überstände wurden aufgedottet. Die Menge der freigesetzten viralen Partikeln wurde mittels eines PreS-Antigen spezifischen dot-blots bestimmt.

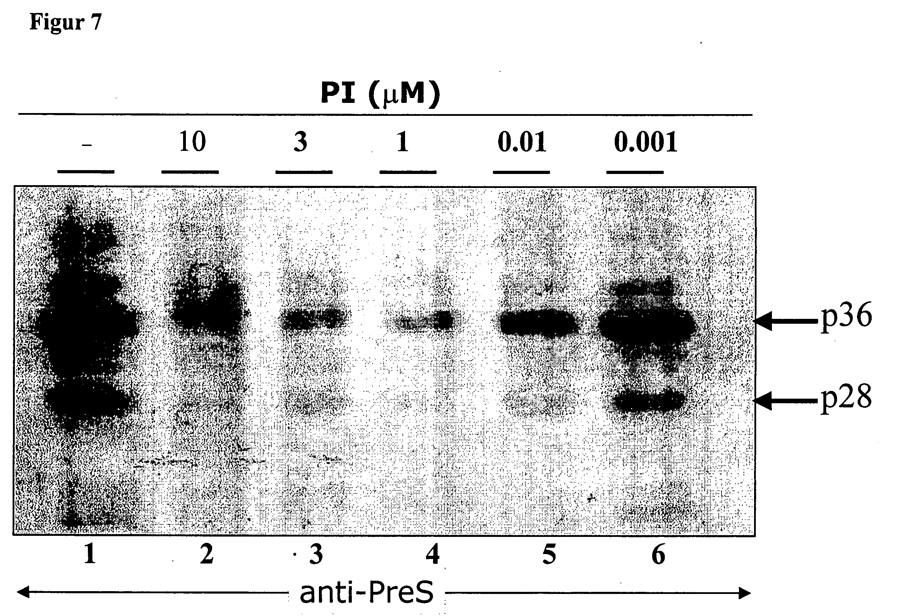

Figur 7: Proteasom-Inhibitoren hemmen konzentrationsabhängig die Freisetzung von PreShaltigen Viruspartikeln in chronisch DHBV-infizierten primären Entenhepatozyten - PreS Western blot-Nachweis.
   Eine 7 Tage alte, chronisch DHBV-infizierte primäre Entenhepatozytenkultur wurde für 48h mit steigenden Konzentrationen von PI (1 nanoM, 10 nanoM, 1 microM, 3 microM und 10 microM) behandelt bzw. nicht behandelt. Die 5 microL der jeweiligen Überstände wurden in 12.5% SDS-PAGE aufgetrennt. Die Darstellung der PreS-Protein-Banden (p36 und p28) erfolgte durch Western blot.

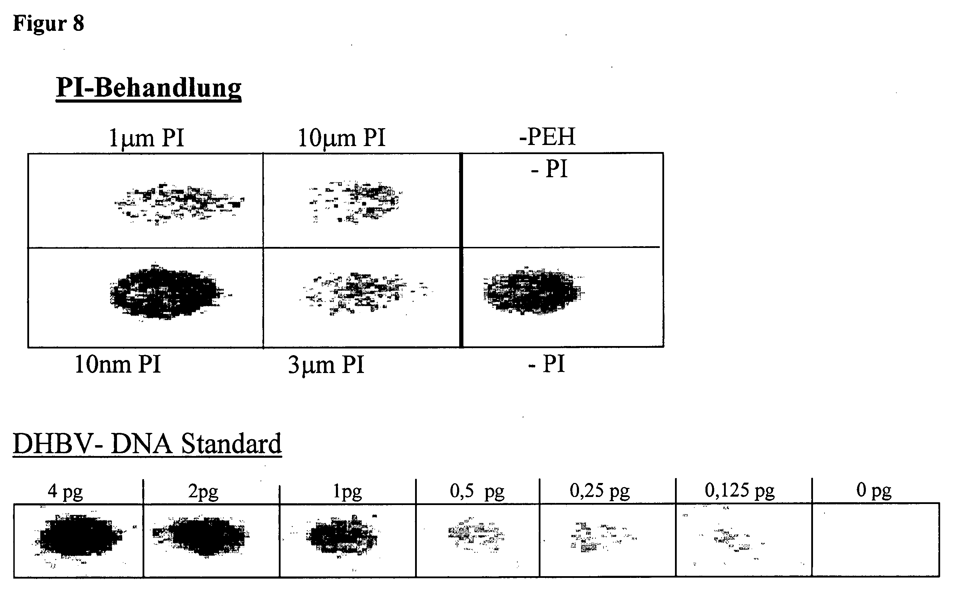

Figur 8: Proteasom-Inhibitoren hemmen konzentrationsabhängig die Freisetzung von DNAhaltigen Viruspartikeln in chronisch DHBV-infizierten primären Entenhepatozyten (PEH) - DNA-dot-blot-Nachweis.
   Eine 7 Tage alte chronisch DHBV-infizierte primäre Entenhepatozytenkultur wurde für 48 h mit steigenden Konzentrationen von PI (10 nanoM, 1 microM, 3 microM und 10 microM) behandelt bzw. nicht behandelt, der Überstand gesammelt und durch Zentrifugation geklärt. Danach wurde der Überstand auf Nitrozellulose aufgedottet und die Membran mit einer ³²P-markierten DHBV-DNA-Sonde hybridisiert und autoradiographiert. Dots von nicht infizierten PEHs sind mit - gekennzeichnet, dots von infizierten Hepatozyten mit +. Die Standardisierung des DNA-dotblots erfolgte durch Auftragung von bekannten Konzentrationen an klonierter DHBV-DNA (Standard DHBV-DNA).

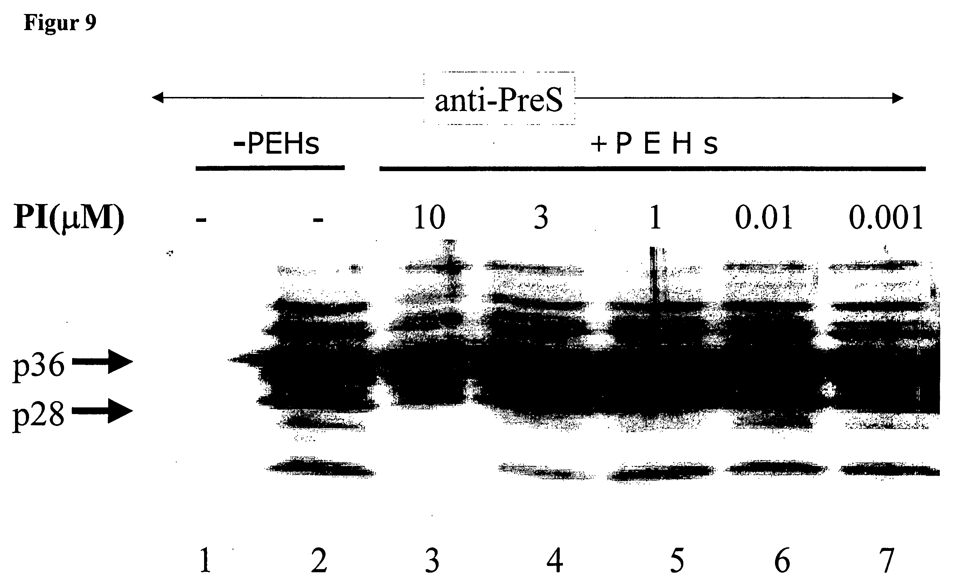

Figur 9: Proteasom-Inhibitoren haben keinen nennenwerten Einfluss auf die intrazelluläre PreS-Genexpression.
   7 Tage alte chronisch DHBV-infizierte primäre Entenhepatozytenkulturen (PEHs) wurden für 48 h mit steigenden Dosen PI behandelt. Zelllysate wurden in 12.5% SDS-PAGE aufgetrennt und zum Nachweis von PreS-Antigen geblottet. In Spuren 1 und 2 sind unbehandelte, nicht infizierte (-PEHs) bzw. chronisch DHBV-infizierte Hepatozyten (+PEHs) aufgetragen. Spuren 3 bis 7 entsprechen Aufträgen von mit unterschiedlichen Dosen an PI-behandelten Zellen. p36 und p28 entsprechen dem PreS-Protein voller Länge bzw. einem Degradationprodukt desselben.

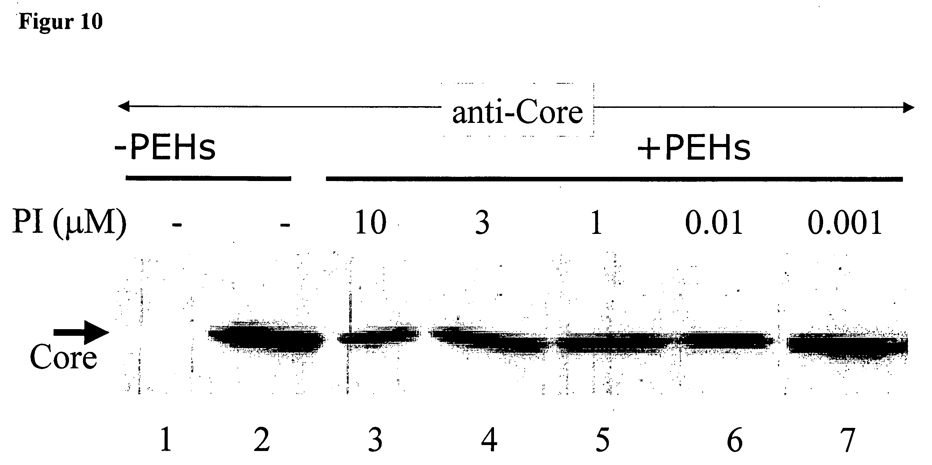

Figur 10: Proteasom-Inhibitoren haben keinen nennenswerten Einfluss auf die Core-Expression. 7 Tage alte chronisch DHBV-infizierte primäre Entenhepatozytenkulturen wurden für 48 h mit steigenden Dosen PI behandelt. Zelllysate wurden in 12.5% SDS-PAGE aufgetrennt und das Core-Protein (Pfeil) durch indirekte Chemolumineszenz dargestellt. In Spuren 1 und 2 sind nicht behandelte DHBV-negative (-PEHs) bzw. chronisch DHBV-infizierte Hepatozyten (+PEH) aufgetragen. Spuren 3 bis 7 entsprechen Aufträgen von mit unterschiedlichen Dosen an PI-behandelten Zellen.

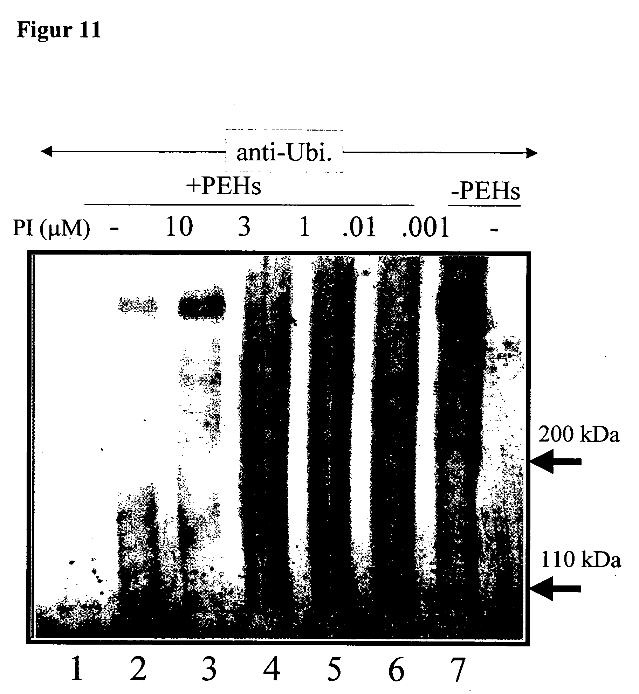

Figur 11: Akkumulation von poly-ubiquitinierten Proteinen in mit Proteasom-Inhibitoren behandelten primären Entenhepatozyten.
   Eine Woche alte, chronisch DHBV-infizierte primäre Entenhepatozyten wurden für 48 h mit steigenden Konzentrationen von PI (Spuren 2 bis 6) behandelt. Als Kontrolle wurden DHBV infizierte (+PEHs, Spur 1) und Virus-freie Zellen ohne PI kultiviert (-PEHs, Spur 7). Anschließend wurden Zelllysate in 6% SDS-PAGE aufgetrennt und mono- sowie poly-ubiquitinierte Proteine mittels Kaninchen-anti-Ubiquitin nachgewiesen. Rechts ist die jeweilige Position der Proteinmarkerbanden in kDA angegeben. Polyubi. zeigt die hochmolekularen poly-ubiquitinierten Proteine an.

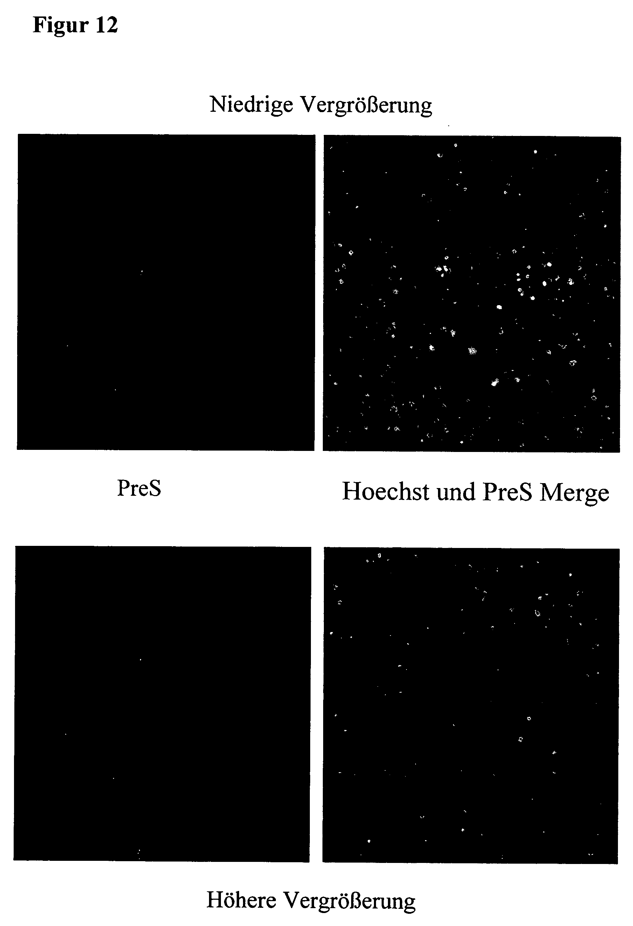

Figur 12: Überstände der mit Proteasom-Inhibitoren behandelten chronisch DHBV-infizierten primären Entenhepatozyten-Kulturen (PEH) enthalten keine infektiösen Nachkommenviren.
   7 Tage alte chronisch DHBV-infizierte primäre Entenhepatozyten wurden für 48 h mit 10 microM PI behandelt und anschließend der geerntete Zellkulturüberstand für die Neuinfektion von DHBV-negativen PEH-Kulturen benutzt. 60 h nach der Infektion wurden die Zellen gewaschen und fixiert. Abschließend erfolgte die Färbung der Zellen für PreS (grün). Die Darstellung der Zellkerne erfolgte mittels Hoechstfärbung (blau). Der obere bzw. untere Bereich der Figur ist eine niedrige bzw höhere Vergrößerung desselben Bildauschnittes.

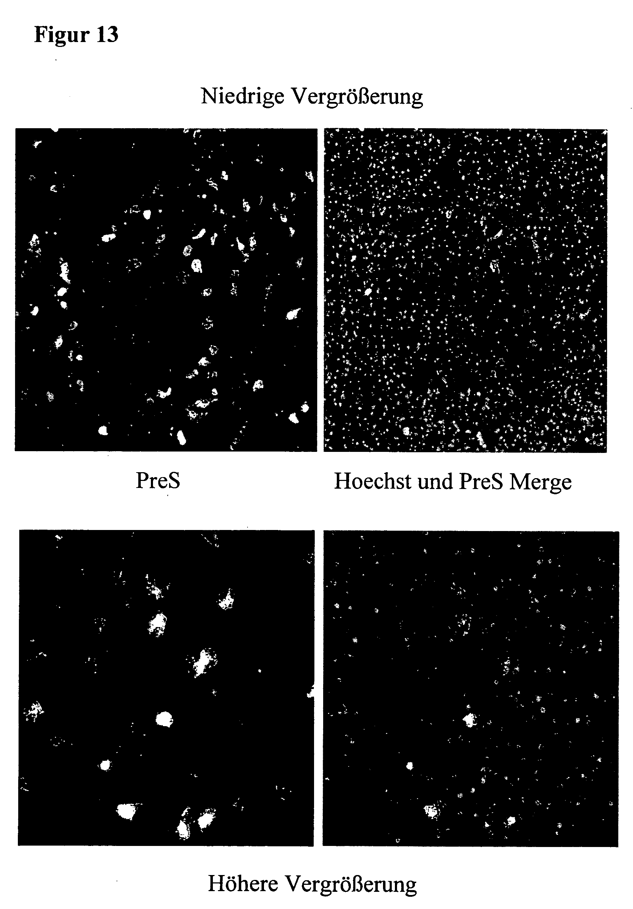

Figur 13: Nachkommenviren aus chronisch DHBV-infizierten primären Entenhepatozyten sind infektiös.
   Zellkulturüberstände einer 7 Tage alten chronisch infizierten primären Entenhepatozytenkultur wurden gesammelt und für die Neu-Infektion von DHBV-negativen primären Entenhepatozyten benutzt. 60 h nach der Infektion wurden die Zellen fixiert. Abschließend erfolgte die Färbung der Zellen für PreS (grün). Die Darstellung der Zellkerne erfolgte mittels Hoechstfärbung (blau). Der untere Bereich der Figur ist die höhere Vergrößerung desselben oben gezeigten Bildauschnittes.

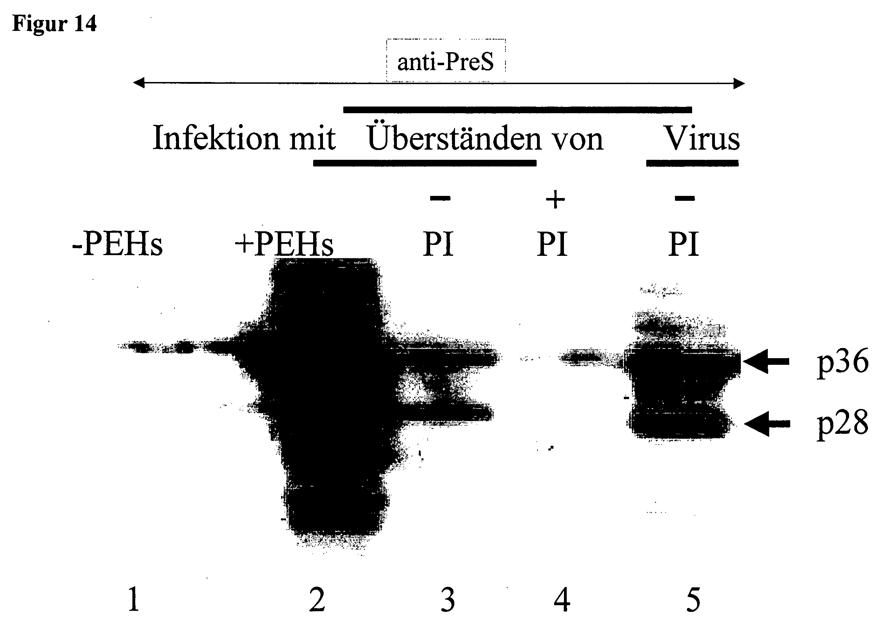

Figur 14: Überstände der mit Proteasom-Inhibitoren behandelten DHBV-infizierten Hepatozyten enthalten keine infektiöse Nachkommenviren -- Nachweis durch PreS-blot.
   7 Tage alte chronisch DHBV-infizierte primäre Entenhepatozyten (PEH) wurden für 48 h mit 10 microM PI behandelt und anschließend der Überstand gesammelt, durch Zentrifugation geklärt und für de novo-Infektion von PEHs benutzt. 60 h nach der Infektion wurden die Zellen lysiert und in 12.5% SDS-PAGE aufgetrennt. Die Darstellung der PreS-Proteinbanden erfolgte durch Western blot. In Spuren 1 und 2 sind DHBV-negative bzw. chronisch DHBV-infizierte PEH aufgetragen. Spur 3 und 4 entsprechen PEHs, welche mit Überständen aus nicht behandelten bzw. PI-behandelten Kulturen infiziert wurden. Aufgetragen in Spur 5 sind PEHs, welche mit einer MOI von 20 infiziert wurden.

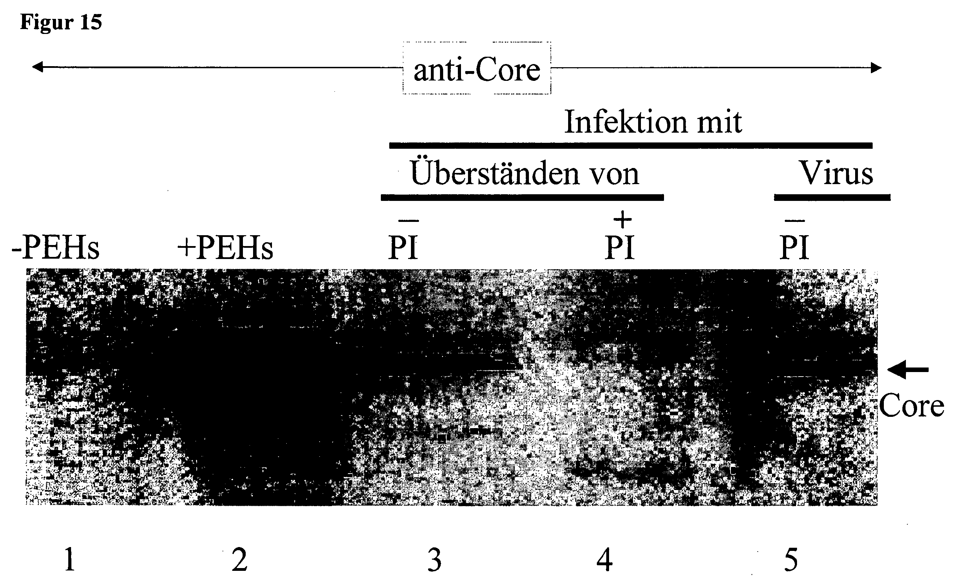

Figur 15: Überstände der mit Proteasom-Inhibitoren behandelten DHBV-infizierten Hepatozyten enthalten keine infektiösen Nachkommenviren - Nachweis durch Core blot.
   7 Tage alte chronisch DHBV-infizierte primäre Entenhepatozyten (PEH) wurden für 48 h mit 10 microM PI behandelt und anschließend der Überstand gesammelt, durch Zentrifugation geklärt und für Neuinfektion von PEH-Kulturen benutzt. 60 h nach der Infektion wurden die Zellen lysiert und das Lysat durch 12.5% SDS-PAGE aufgetrennt. Die Darstellung des Core-Proteins erfolgte durch Western blot. In Spuren 1 und 2 sind DHBV-negative bzw. chronisch DHBV-infizierte PEH aufgetragen. Spur 3 und 4 entsprechen PEH, welche mit Überständen aus nicht behandelten bzw. PI-behandelten Kulturen infiziert wurden. Aufgetragen in Spur 5 sind PEHs, welche mit einer MOI von 20 infiziert worden sind.

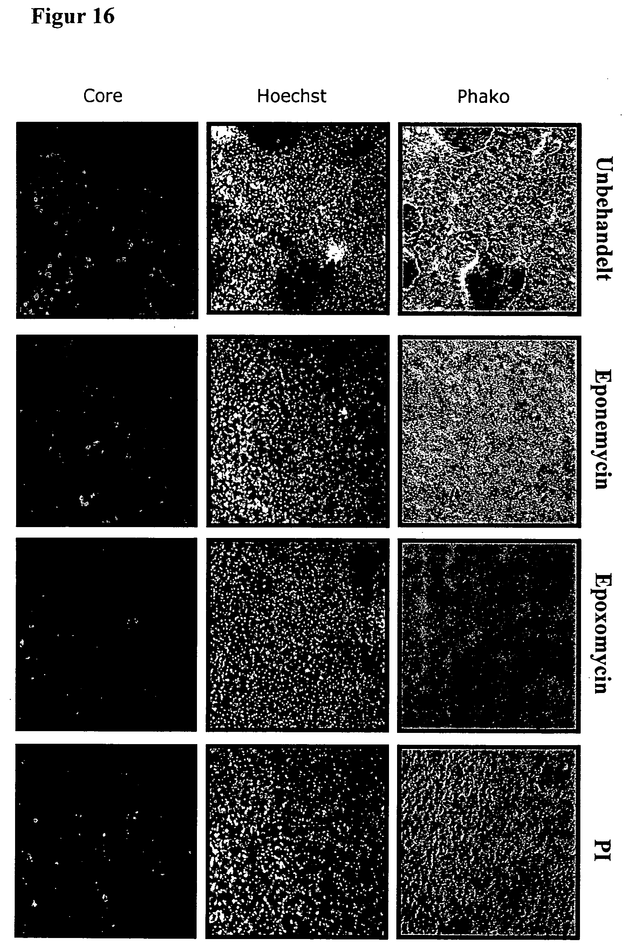

Figur 16: Hemmung der Primär- und Sekundärinfektion durch Behandlung mit Proteasom-Inhibitoren von primären Entenhepatozyten (PEH) infiziert mit DHBV.
   Vier Tage alte Kulturen von PEH wurden mit einer MOI von 20 für 16 h infiziert. Anschließend wurden die Zellen gewaschen und im neuen Medium für weitere drei Tage kultiviert. Am 3. Tag nach der Infektion wurden die Zellen entweder ohne Proteasom-Inhibitoren kultiviert (unbehandelt) oder mit jeweils 1 microM Eponemycin, Epoxomicin oder PI für weitere 3 Tage behandelt. Am 6. Tag der Infektion wurden die Zellen gewaschen und fixiert. Anschließend erfolgte die Färbung der Zellen für Core-Protein (grün). Die Zellkerne wurden mit Hoechst gefärbt (blau). Die Phasenkontrastaufnahmen der jeweiligen Bildausschnitte sind ebenfalls zu sehen.

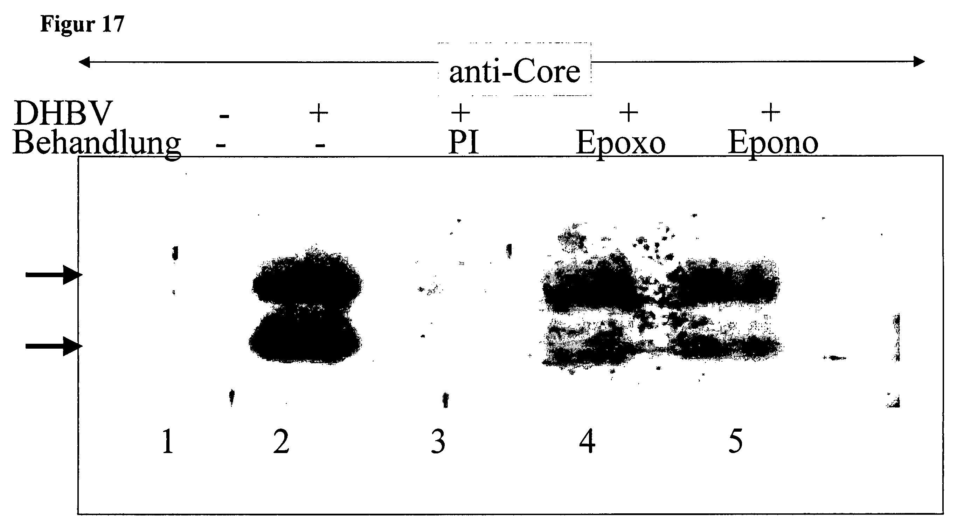

Figur 17: Proteasom-Inhibitoren hemmen die Freisetzung des e-Antigens in DHBV-infizierten primären Entenhepatozyten.
   Vier Tage alte primäre Entenhepatozyten (PEH) wurden mit einer MOI von 20 für 16 h infiziert. Anschließend wurden die Zellen gewaschen und im neuen Medium für weitere drei Tage kultiviert. Am 3. Tag nach der Infektion wurden die Zellen mit jeweils 1 microM PI (Spur 3) Epoxomicin (Spur 4), Eponemycin (Spur 5) für weitere drei Tage behandelt. Am 6. Tag nach der Infektion wurde der Überstand gesammelt, durch Zentrifugation geklärt. Ein Aliquot der Überstände wurde in 12.5% SDS-PAGE aufgetrennt. Die Darstellung der e-Antigen-Protein-Banden (Pfeile) erfolgte durch indirekte Chemoluminescenz. In Spuren 1 und 2 sind nicht behandelte negative bzw. chronisch DHBV-infizierte PEH, in Spuren 3 PI, 4 Epoxomicin und 5 Eponemycin behandelte, DHBV-infizierte PEH aufgetragen.

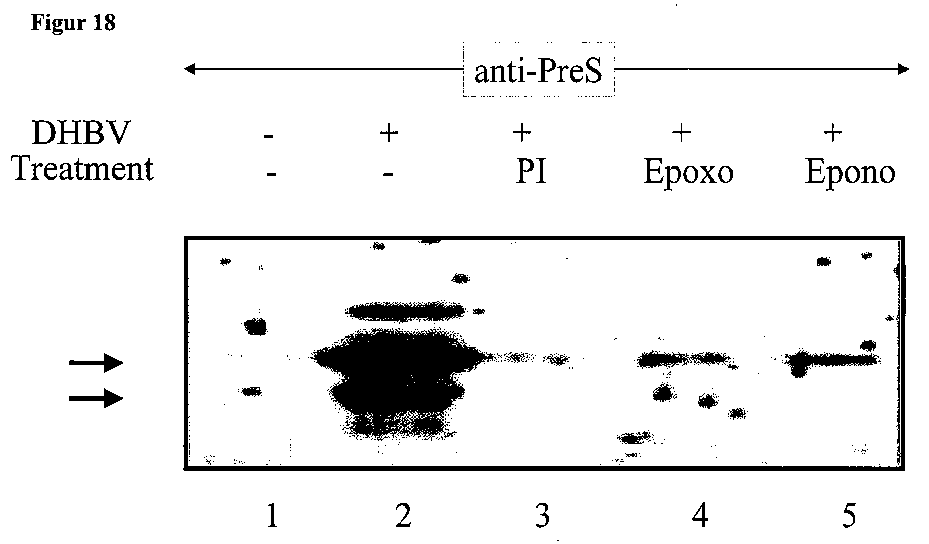

Figur 18: Proteasom-Inhibitoren hemmen die Freisetzung des PreS-Antigens in DHBV-infizierten primären Entenhepatozyten.
   Vier Tage alte Hepatozyten-Kulturen wurden mit einer MOI von 20 für 16 h infiziert. Anschließend wurden die Zellen gewaschen und im neuen Medium für weitere drei Tage kultiviert. Am 3. Tag nach der Infektion wurden die Zellen mit jeweils 1 microM PI (Spur 3), Epoxomicin (Spur 4) und Eponemycin (Spur 5) für weitere drei Tage behandelt. Am 6. Tag nach der Infektion wurde der Überstand gesammelt und in 12.5% SDS-PAGE aufgetrennt. Die Darstellung der PreS-Protein-Banden (p36 und p28) erfolgte im Western blot. In Spuren 1 und 2 sind nicht behandelte negative bzw. chronisch DHBV-infizierte PEH aufgetragen, DHBV-infizierte PEH wurden aufgetragen in Spur 3 (PI), in Spur 4 (Epoxomicin), und in Spur 5 (Eponemycin).

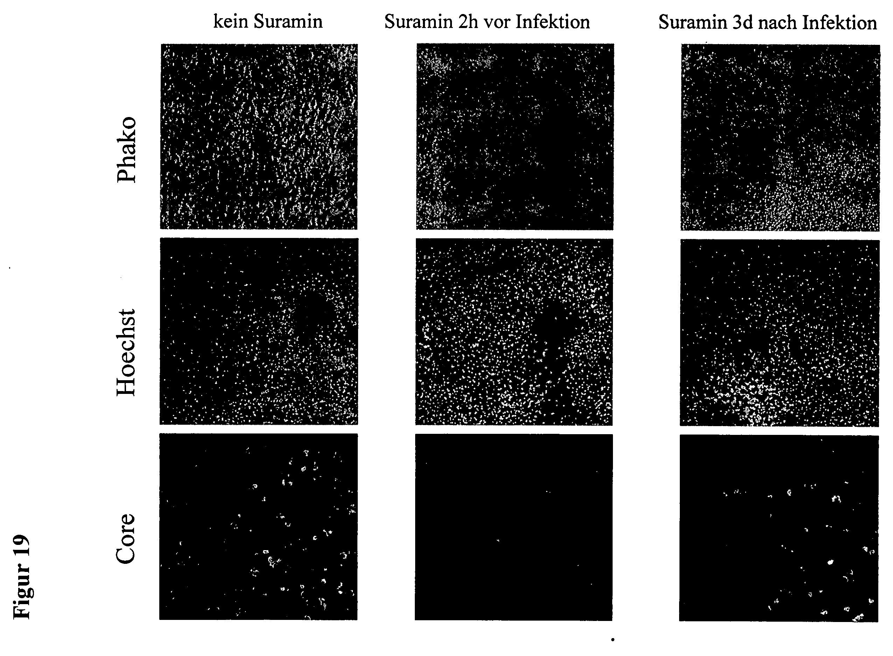

Figur 19: Suramin hemmt sowohl die primäre als auch die sekundäre DHBV-Infektion in primären Entenhepatozyten.
   Vier Tage alte PEH wurden 2 h vor der Infektion mit 100 microg/ml Suramin behandelt (Suramin Präinfektion) oder zunächst unbehandelt belassen (kein Suramin und Suramin 3d post Infektion). Anschließend wurden alle Zellen mit einer MOI von 20 infiziert. Nach 16 h Inkubation wurden die Kulturen gewaschen und mit neuem Medium weiter kultiviert. Am 3. Tag nach der Infektion wurde das Medium erneut gewechselt und die Zellen in "kein Suramin" und "Suramin prä Infektion" wurden für weitere drei Tage ohne und die Zellen in "Suramin 3d post Infektion" mit 100 microg/ml Suramin behandelt. Am 6. Tag der Infektion wurden die Zellen gewaschen und fixiert. Anschließend erfolgte die Färbung der Zellen für Core-Protein (Core). Die Zellkerne wurden mit Hoechst gefärbt (Hoechst). Die Phasenkontrastaufnahmen der jeweiligen Bildausschnitte sind ebenfalls gezeigt (Phasenkontrast).

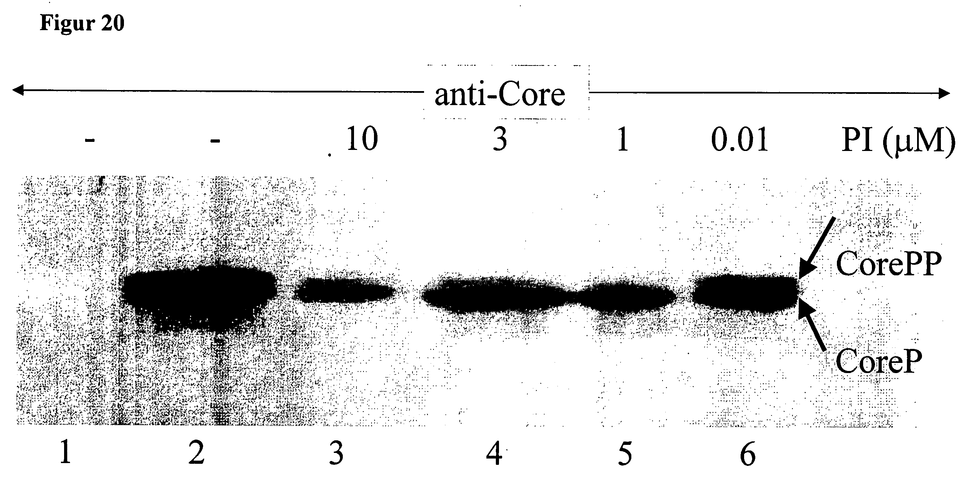

Figur 20: Proteasom-Inhibitoren induzieren die Hypophosphorylierung des intrazellulären Core-Proteins in chronisch DHBV-infizierten primären Hepatozyten.
   7 Tage alte chronisch DHBV-infizierte primäre Entenhepatozytenkulturen wurden für 48 h mit steigenden Dosen PI behandelt und anschließend lysiert. Die Lysate wurden in 12.5% SDS-PAGE aufgetrennt und das Core-Protein durch indirekte Chemolumineszenz dargestellt. In Spuren 1 und 2 sind nicht behandelte negative bzw. chronisch DHBV-infizierte PEH aufgetragen. Spuren 3 bis 6 entsprechen den mit PI behandelten Zellen. CorePP entspricht hyperphosphoryliertem und CoreP hypophosphoryliertem Core-Protein.

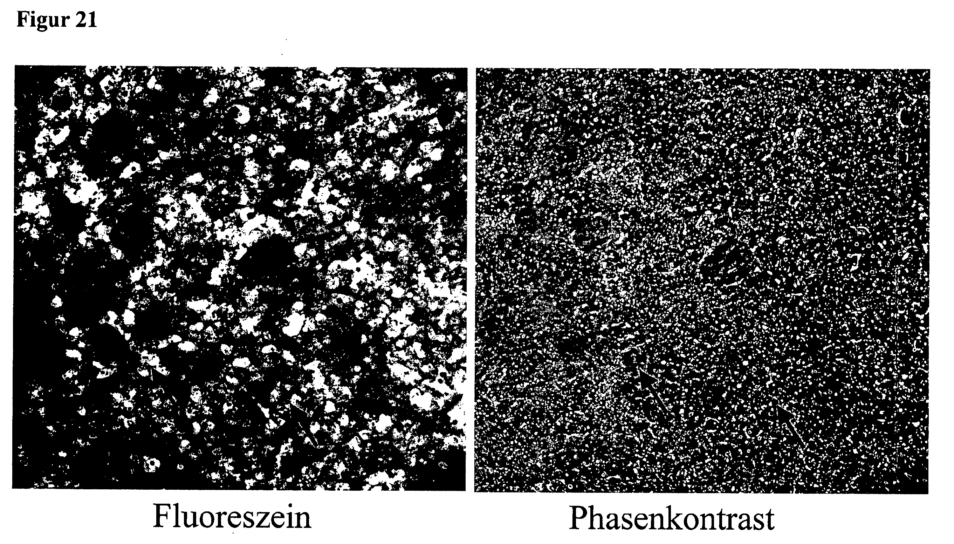

Figur 21: Behandlung von primären Entenhepatozyten mit Proteasom-Inhibitoren schränkt nicht deren Vitalität und Funktionalität ein.
   7 Tage alte primäre Zellkulturen aus chronisch DHBV-infizierten Entenlebern wurden 48 h lang mit steigenden Konzentration von PI (10 microM, 3 microM und 1 microM) behandelt. Am Ende dieser Behandlungsperiode wurden die Zellen für 5 min mit FDA-haltigem Williams E Medium (5 microg/ml) bei 37°C inkubiert. Anschließend wurden die Zellen mit PBS gewaschen und die erfolgreiche Konversion des FDA in Fluoreszein in Hepatozyten mittels eines Epifluoreszensmikroskops beurteilt. Zellen mit grüner Färbung im Zytoplasma wurden als vital und funktionell angenommen. Die jeweiligen Fluoreszenz- (Fluoreszein) und die korrespondierenden Phasenkontrastaufnahmen (Phasenkontrast) mit der Angabe der benutzten PI-Dosis werden gezeigt. Die weißen Pfeile mit Spitze zeigen beispielhaft auf Nester von nichtparenchymalen Zellen, welche im Vergleich zu Hepatozyten keine bzw. nur beschränkte FDA-Aufnahme und Konversion zeigen.

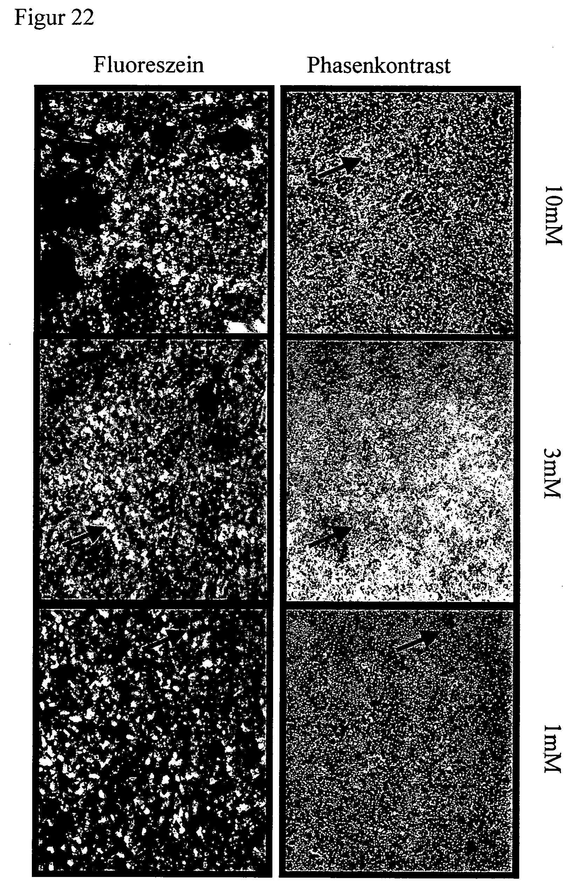

Figur 22: Aufnahme und Konversion des Vitalmarkers Fluoreszeindiazetat in Fluoreszein erfolgt bevorzugt in Hepatozyten.
   Eine Woche alte primäre Zellkulturen aus chronisch DHBV-infizierten Entenlebern wurden für 5 min mit FDA-haltigem Williams E Medium (5 microg/ml) bei 37°C inkubiert. Anschließend wurden die Zellen mit PBS gewaschen und die erfolgreiche Konversion des FDA in Fluoreszein in Hepatozyten mittels eines Epifluoreszenzmikroskops beurteilt. Zellen mit grüner Färbung im Zytoplasma wurden als vital und funktionell angenommen (Fluoreszein). Die zu dem selben Bildausschnitt korrespondierende Phasenkontrastaufnahme ist ebenfalls gezeigt (Phasenkontrast). Die weißen Pfeile mit Spitze zeigen beispielhaft auf Nester von nichtparenchymalen Zellen, welche im Vergleich zu den Hepatozyten keine bzw. nur beschränkte FDA-Aufnahme und Konversion zeigen.

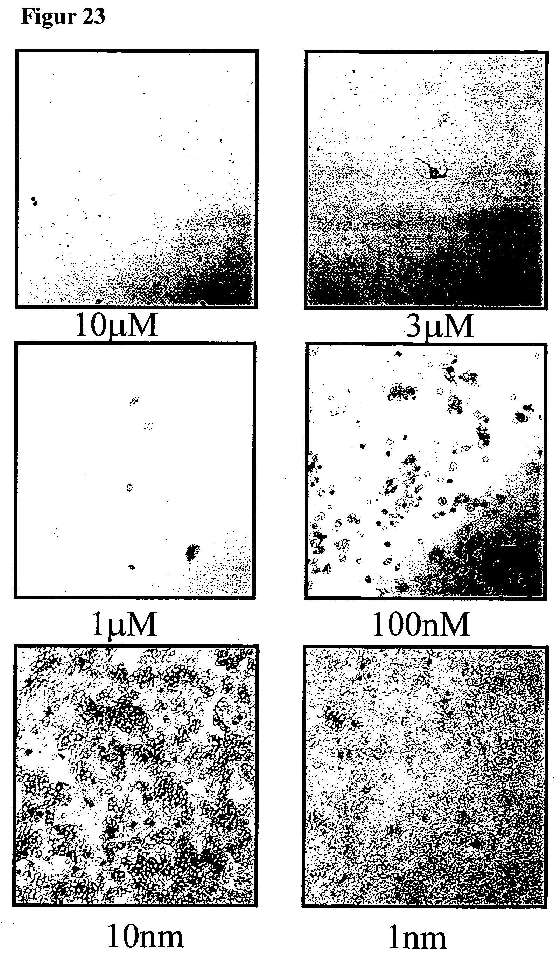

Figur 23: Behandlung von Hepatomazellen (HepG2) mit Proteasom-Inhibitoren schränkt deren Vitalität und Funktionalität ein.
   HepG2-Zellen wurden 48 h lang mit steigenden Konzentration (10 microM, 3 microM und 1microM, 100 nanoM, 10 nanoM und 1 nanoM) von PI (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration des PI ist in den entsprechenden Phasenkontrastaufnahmen angegeben.

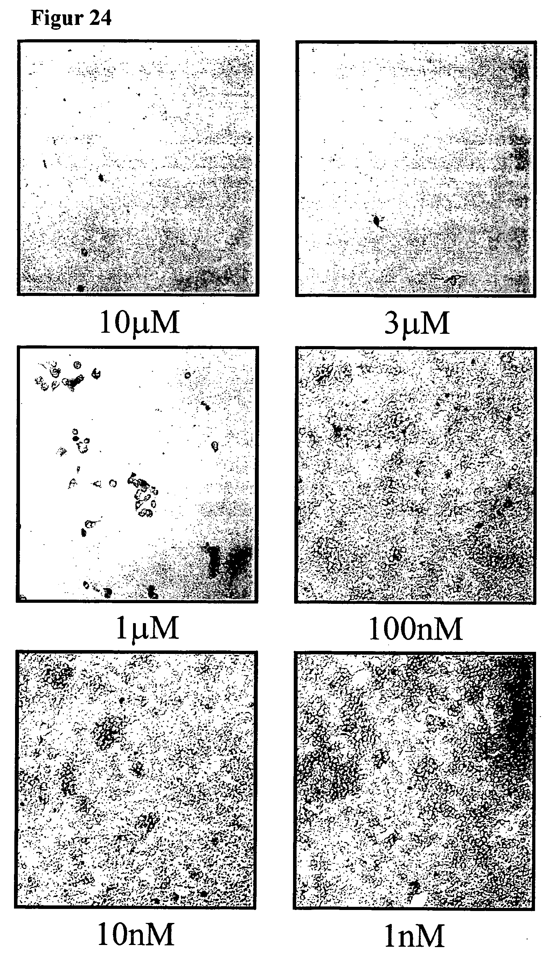

Figur 24: Behandlung von Hepatomazellen (HepG2) mit Eponemycin schränkt deren Vitalität und Funktionalität ein.
   HepG2-Zellen wurden 48 h lang mit steigenden Konzentration (10 microM, 3 microM und 1microM, 100 nanoM, 10 nanoM und 1 nanoM) von Eponemycin (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration des Eponemycin ist in den entsprechenden Phasenkontrastaufnahmen angegeben.

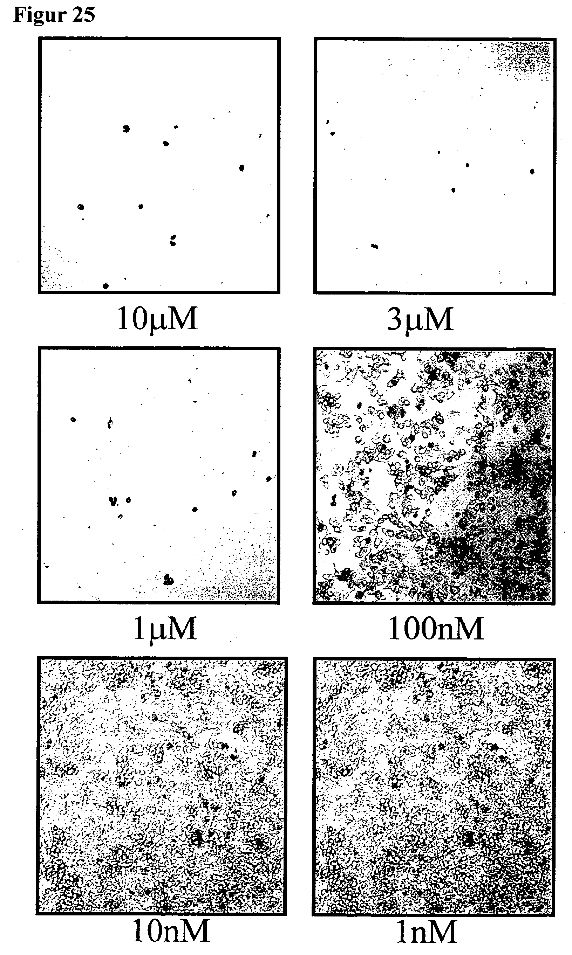

Figur 25: Behandlung von humanen Hepatomazellen, HepG2 mit Epoxomicin schränkt deren Vitalität und Funktionalität ein.
   HepG2-Zellen wurden 48 h lang mit steigenden Konzentration (10 microM, 3 microM und 1microM, 100 nanoM, 10 nanoM und 1 nanoM) von Epoxomicin (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration von Epoxomicin ist in den entsprechenden Phasenkontrastaufnahmen angegeben.

### Literatur

Adachi, A; Gentleman, HE; König, S, Folks; T, Willey, R; Rabson, A; Martin, MA (1986). Production of acquired immunodeficiency syndrome-associated retrovirus in human and nonhuman cells transfected with an infectious molecular clone. *J. Virol.* **59**:284-291.
Adams, J; Behnke, M; Chen, S; Cruickshank, AA; Dick, LR; Grenier, L; Klunder, JM; Ma, YT; Plamondon, L; Stein, RL (1998). Potent and selective inhibitors of the proteasome: dipeptidyl boronic acids. *Bioorg. Med. Chem. Lett.* **8**:333-338.
Adams, J; Palombella, VJ; Sausville, EA; Johnson, J; Destree, A; Lazarus, DD; Maas, J.; Pien, CS; Prakash, S; Elliott, PJ (1999). Proteasome inhibitors: a novel class of potent and effective antitumor agents. *Cancer Res.* **59**:2615-2622.
Adams, J; Palombella, VJ; Elliott, PJ (2000). Proteasome inhibition: a new strategy in cancer treatment. *Investigational New drugs* **18**:109-121.
Adams, J; Stein, R (1996). Novel inhibitors of the proteasome and their therapeutic use in inflammation. *Annu. Rep. Med. Chem.* **31**:279-288.
André, P; Gröttrup, M; Klenerman, P; de Giuli, R; Booth, BL Jr; Cerundolo, V; Bonneville, M; Jotereau, F; Zinkernagel, RM; Lotteau, V (1998). An inhibitor of HIV-1 protease modulates proteasome activity, antigen presentation, and T cell responses. *Proc. Natl. Acad. Sci.* USA **95**:13120-13124.
Baumeister, W; Walz, J; Zuhl, F; Seemuller, E (1998). The proteasome: paradigm of a selfcompartmentalizing protease. *Cell* **92**:367-380.
Bogyo, M; McMaster, JS; Gaczynska, M; Tortorella, D; Goldberg, AL; Ploegh, H (1997). Covalent modification of the active site threonine of proteasomal beta subunits and the Escherichia coli homolog HsIV by a new class of inhibitors. *Proc. Natl. Acad. Sci.* USA **94**:6629-6634.
Bour, S; Schubert, U; Peden, K; Strebel, K (1996). The envelope glycoprotein of human immunodeficiency virus type 2 enhances particle release: a Vpu-like factor? *J. Virol.* **70**:820-829.
Breiner, KM; Urban, S; Glass, B; Schaller, H (2001). Envelope protein-mediated downregulation of hepatitis B virus receptor in infected hepatocytes. *J. Virol.* **75**:143-50.
Bruns, M; Miska, S; Chassot, S; Will, H (1998). Enhancement of hepatitis B virus infection by noninfectious subviral particles. *J. Virol.* **72**:1462-1468.
Bryant, JL; Gallo RC; Weichold FF (2000). Use of protease inhibitors to modulate cellular pathways, immunity and therapies associated therewith. Patent application WO 0033654.
Chen, HS; Kew, MC; Hornbuckle, WE; Tennant, BC; Cote, PJ; Gerin, JL; Purcell, RH; Miller, RL (1992). The precore gene of the woodchuck hepatitis virus genome is not essential for viral replication in the natural host. *J. Virol.* **66**:5682-5684.
Coux, O; Tanaka, K; Goldberg, AL (1996). Structure and functions of the 20S and 26S proteasomes. *Annu. Rev. Biochem.* **65**:801-847.
Dandri, M; Burda, MR; Torok, E; Pollok, JM; Iwanska, A; Sommer, G; Rogiers, X; Rogler, CE; Gupta, S; Will, H; Greten, H; Petersen, J (2001). Repopulation of mouse liver with human hepatocytes and in vivo infection with hepatitis B virus. Hepatology 33:981-988.
Doolittle, RF; Feng, DF; McClure, MA; Johnson, MS (1990). Retrovirus phylogeny and evolution. *Curr. Top. Microbiol. Immunol.* **157:**1-18.
Elliott, PJ; Pien, CS; McCormack, TA; Capman, ID; Adams, J (1999). Proteasome inhibition: a novel mechanism to combat asthma. *J. Allergy Clin. Immunol.* **104**:294-300.
Fenteany, G; Standaert, RF; Lane, WS; Choi, S; Corey, EJ; Schreiber, SL (1995). Inhibition of proteasome activities and subunit-specific amino-terminal threonine modification by lactacystein. *Science* **268**:726-731.
Fernholz, D; Wildner, G; Will, H (1993). Minor envelope proteins of duck hepatitis B virus are initiated at internal pre-S AUG codons but are not essential for infectivity. *Virology* **197:**64-73.
Frankel, A; Man, S; Elliott, P; Adams, J; Kerbel, RS (2000). Lack of multicellular drug resistance observed in human ovarian and prostate carcinoma treated with the proteasome inhibitor PS-341. *Clin. Cancer Res.* **6:**3719-3728.
Gaczynska M, Rock KL, Goldberg AL (1993). Gamma-interferon and expression of MHC genes regulate peptide hydrolysis by proteasomes. *Nature* **365**:264-267.
Gardner, RC; Assinder, SJ; Christie, G; Mason, GG; Markwell, R; Wadsworth, H; McLaughlin, M; King, R; Chabot-Fletcher, MC; Breton, JJ; Allsop, D; Rivett, AJ (2000). Characterization of peptidyl boronic acid inhibitors of mammalian 20 S and 26 S proteasomes and their inhibition of proteasomes in cultured cells. *Biochem. J.* **346**:447-454.
Geier, E; Pfeifer, G; Wilm, M; Lucchiari-Hartz, M; Baumeister, W; Eichmann, K; Niedermann, G (1999). A giant protease with potential to substitute for some functions of the proteasome. *Science* **283**:978-981.
Glas, R; Bogyo, M; McMaster, JS; Gaczynska, M; Ploegh, HL (1998). A proteolytic system that compensates for loss of proteasome function. *Nature* **392**:618-622.
Hanada, M; Sugawara, K; Kaneta, K; Toda, S; Nishiyama, Y; Tomita, K; Yamamoto, H; Konishi, M; Oki, T (1992). Epoxomicin, a new antitumor agent of microbial origin. *J. Antibiot.* (Tokyo) **45**:1746-1752.
Hershko, A; Ciechanover, A (1998). The ubiquitin system. *Annu. Rev Biochem.* **67**:425-479.
Higashitsuji, H; Itoh, K; Nagao, T; Dawson, S; Nonoguchi, K; Kido, T; Mayer, RJ; Arii, S; Fujita, J (2000). Reduced stability of retinoblastoma protein by gankyrin, an oncogenic ankyrinrepeat protein overexpressed in hepatomas. *Nat. Med.* **6**:96-99.
Hu, Z; Zhang, Z; Doo, E; Coux, O; Goldberg, AL; Liang, TJ (1999). Hepatitis B virus X protein is both a substrate and a potential inhibitor of the proteasome complex.
*J. Virol.* **73**:7231-40.
Lightcap, ES; McCormack, TA; Pien, CS; Chau, V; Adams, J; Elliott, PJ (2000). Proteasome inhibition measurements: clinical application. *Clin. Chem.* **46**:673-683.
Meng, L; Kwok, BH; Sin, N; Crews, CM (1999a). Eponemycin exerts its antitumor effect through the inhibition of proteasome function. *Cancer Res.* **59**:2798-2801.
Meng, L; Mohan, R; Kwok, BH; Elofsson, M; Sin, N; Crews, CM (1999b). Epoxomicin, a potent and selective proteasome inhibitor, exhibits in vivo antiinflammatory activity. *Proc. Natl. Acad. Sci.* USA. **96**(18):10403-10408.
Moradpour, D; Grabscheid, B; Kammer, AR; Schmidtke, G; Gröttrup, M; Blum, HE; Cerny, A (2001). Expression of hepatitis C virus proteins does not interfere with major histocompatibility complex class I processing and presentation in vitro. *Hepatology* **33**: 1282-1287.
Murray, RZ; Norbury, C (2000). Proteasome Inhibitors as anti-cancer agents. *Anticancer Drugs* **11**:407-417.
Ott, DE; Coren, LV; Copeland, TD; Kane, BP; Johnson, DG; Sowder, RC 2nd; Yoshinaka, Y; Oroszlan, S; Arthur, LO; Henderson, LE (1998). Ubiquitin is covalently attached to the p6Gag proteins of human immunodeficiency virus type-1 and simian immunodeficiency virus and the p12Gag protein of moloney murine leukemia virus. *J. Virol.* **72:**2962-2968.
Palombella, VJ; Conner, EM; Fuseler, JW; Destree, A; Davis, JM; Laroux, FS; Wolf, RE; Huang, J; Brand, S; Elliott, PJ; Lazarus, D; McCormack, T; Parent, L; Stein, R; Adams, J; Grisham, MB (1998). Role of the proteasome and NF-kappaB in streptococcal cell wall-induced polyarthritis. *Proc. Natl. Acad. Sci. USA* **95**:15671-15676.
Phillips, JB; Williams, AJ; Adams, J; Elliott, PJ; Tortella, FC (2000). Proteasome inhibitor PS-519 reduces infarction and attenuates leukocyte infiltration in a rat model of focal cerebral ischemia. *Stroke* **31**:1686-1693.
Pugh, JC; Simmons, H (1994). Duck hepatitis B virus infection of Muscovy duck hepatocytes and nature of virus resistance in vivo. *J Virol.* **68**:2487-94.
Rehermann, B; Ferrari, C; Pasquinelli, C; Chisari, FV (1996). The hepatitis B virus persists for decades after patients' recovery from acute viral hepatitis despite active maintenance of a cytotoxic T-lymphocyte response. *Nat. Med.* **2**:1104-1108.
Rivett, AJ; Gardner, RC (2000). Proteasome inhibitors: from in vitro uses to clinical trials. *J. Pept. Sci.* **6**:478-488.
Rock, KL; Goldberg, AL (1999). Degradation of cell proteins and the generation of MHC class I-presented peptides. *Annu. Rev. Immunol.* **17:**739-779.
Schäfer, S; Tolle, T; Lottmann, S; Gerlich, WH (1998). Animal models and experimental systems in hepatitis B virus research. *In* R. C. Koshy, W. H. (ed.), Hepatitis B virus: Molecular mechanisms in disease and novel strategies for therapy. Imperial College Press, London p. 51-74.
Schmidtke, G; Holzhutter, HG; Bogyo, M; Kairies, N; Groll, M; de Giuli, R; Emch, S; Gröttrup, M (1999). How an inhibitor of the HIV-I protease modulates proteasome activity. *J. Biol. Chem.* **274**:35734-35740.
Schubert, U; Antön, LC; Bacik, I; Cox, JH; Bour, S; Bennink, JR; Orlowski, M; Strebel, K; Yewdell, JW (1998). CD4 glycoprotein degradation induced by Human Immunodeficiency Virus type 1 Vpu protein requires the function of proteasomes and the ubiquitin conjugating pathway. *J. Virol.* 72:2280-2288.
Schubert, U; Clouse, KA; Strebel, K (1995). Augmentation of virus secretion by the human immunodeficiency virus type 1 Vpu protein is cell type independent and occurs in cultured human primary macrophages and lymphocytes. *J. Virol.* **69**:7699-7711.
Schubert, U; Antön, LC; Gibbs, J; Norbury, CC; Yewdell, JW; Bennink, JR (2000) Rapid degradation of a large fraction of newly synthesized proteins by proteasomes. *Nature* **404**:770-774.
Schwartz, O; Marechal, V; Friguet, B; Arenzana-Seisdedos, F; Heard, J-M (1998). Antiviral activity of the proteasome on incoming human immunodeficiency virus type 1. *J. Virol.* **72**:3845-3850.
Schwartz, AL; Ciechanover, A (1999). The ubiquitin-proteasome pathway and pathogenesis of human diseases. *Annu. Rev Med.* **50**:57-74.
Sells, MA; Chen, ML; Acs, G (1987). Production of hepatitis B virus particles in Hep G2 cells transfected with cloned hepatitis B virus DNA. *Proc. Natl. Acad. Sci. USA* **84**:1005-1009.
Sirma, H; Weil, R; Rosmorduc, O; Urban, S; Israel, A; Kremsdorf, D; Brechot, C (1998). Cytosol is the prime compartment of hepatitis B virus X protein where it colocalizes with the proteasome. *Oncogene* **16**: 2051-2063.
Suzuki, R; Tamura, K; Li, J; Ishii, K; Matsuura, Y; Miyamura, T; Suzuki, T (2001). Ubiquitinmediated degradation of hepatitis C virus core protein is regulated by processing at its carboxyl terminus. *Virology* **280**:301-309.
Swanstrom, R; Wills, J (1997). Synthesis, assembly, and processing of viral proteins. In Retroviruses, J. Coffin, S. Hughes and H. Varmus eds. (Plainview, NY: Cold Spring Harbor Press), pp. 263-334.
Teicher, BA; Ara, G; Herbst, R; Palombella, VJ; Adams, J (1999). The proteasome inhibitor PS-341 in cancer therapy. *Clin. Cancer Res.* **5:**2638-2645.
Theodore, TS; Englund, G; Buckler-White, A; Buckler, CE; Martin, MA; Peden, KWC (1996). Construction and characterization of a stable full-length macrophage-tropic HIV type 1 molecular clone that directs the production of high titers of progeny virions. *AIDS Res. Hum. Retrovir.* **12**:191-194.
Trautwein, C; Manns, M (2001). Antivirale Therapie der chronischen Virushepatitis. *Internist* **42**:913-923.
Voges, D; Zwickl, P; Baumeister, W (1999). The 26S proteasome: a molecular machine designed for controlled proteolysis. *Annu. Rev. Biochem.* **68**:1015-1068.
Yagi, T; Hardin, JA; Valenzuela, YM; Miyoshi, H; Gores, GJ; Nyberg, SL (2001). Caspase inhibition reduces apoptotic death of cryopreserved porcine hepatocytes. *Hepatology* **33**:1432-40.

### Abkürzungsverzeichnis

- A3.01: humane CD4⁺ T-Zelllinie
- AIDS: Aquired Immunodeficiency Syndrome
- Annexin-V: Zellrezeptor Annexin-Fünf
- ATCC: American Type Culture Collection
- ATP: Adenosin-5'-triphosphat
- BIV: Bovines Immundefizienzvirus
- BLV: Bovines Leukämievirus
- CA: Capsid (HIV-1 p24^{CA} Antigen)
- cccDNA: komplett doppelsträngig supergecoiltes DNA-Genom
- CFTR: Cystis Fibrosis Transmembran Regulator
- d: Tag
- DHB(V): Enten-Hepatitis-B(-Virus)
- DHBV: Enten-Hepatitis-B-Virus
- DMEM: Dulbeccos' modifizierten Eagle's Medium
- DMSO: Dimethylsulfoxid
- DNA: desoxyribonucleic acid (Desoxyribonukleinsäure)
- ECL: enhanced chemiluminescence
- EDTA: Ethylendiamintetraessigsäure
- EIAV: Equine Infectious Anemia Virus
- ELISA: Enzyme Linked Immunosorbent Assay
- Env: Envelope
- ER: Endoplasmatisches Retikulum
- FDA: Fluoreszeindiazetat
- FIV: Feline Leukemia Virus
- Gag: Gruppenspezifisches Antigen, Core Portein von Retroviren
- h: Stunde(n)
- hr: Stunde(n)
- HAART: HAART-Therapie (highly active antiretroviral therapy)
- HAV: Hepatitis-A-Virus
- HBe: Hepatitis-B-Virus E-Antigen
- HBs: HBV-Oberflächenprotein
- HBV: Hepatitis-B-Virus
- HCC: Hepatozelluläres Karzinom
- HCV: Hepatitis-C-Virus
- HDV: Hepatitis-Delta-Virus
- HeLa-Zellen: ATCC: CCL2
- HEPES: (N-[2-hydroxyethyl]piperazine-N'-[2-ethanesulfonic acid])
- HEV: Hepatitis-E-Virus
- HFV: Hepatitis-F-Virus
- HGV: Hepatitis-G-Virus
- HIV: Humanes Immundefizienzvirus
- HIVAN: HIV associated nephropathy Syndrom
- HIV-1_{NL4-3}: Humanes Immundefizienzvirus, infektioeser Klon NL4-3
- HLS: HIV-associated lipodystrophy syndrome
- HPV: Humanes Papilloma-Virus
- HTLV: Humanes T-Zell Leukämie Virus
- i.d.R.: in der Regel
- IFN: Interferon
- IL: Interleukin
- kb: Kilobasen
- IκB: inhibitorischen Faktor IκB
- kDa: Kilodalton (Maß für Molekulargewicht)
- Ki: inhibitorische Konstante
- LC: Lactacystin
- L-Domäne: late assembly domain, späte Assemblierungsdomäne in retroviralen Gag-Proteinen
- LLnL: Leuzinyl- Leuzinly-nor-Leuzinal
- MA: Matrix, (p17^{MA} Protein von HIV-1)
- MDa: Megadalton
- MG 132: N-carbobenzoxyl-L-leucinyl-L-leucinyl-L-leucinal
- MG232: Borsäure-Derivat von MG132
- MHC: Major Histocompatibilitäts Komplex
- Mo-MuLV: Murines Leukämie-Virus
- MOI: multiplicity of infection
- MT-4-Zellen: humane CD4⁺ T-Zellinie, HTLVI transformiert
- nanoM: nano Molar (nM)
- NC: Nukleokapsid,
- NF-κB: Transkriptionsfaktor
- NLVS: 4-Hydroxy-5-iodo-3-nitrophenylactetyl-L-Leucinyl-L-Leucinyl-L-Leucin-vinylsulfon, auch bezeichnet als NLVS
- nM: nano Mol
- ocDNA: offen zirkuläre Form der DANN
- p27^{CA}: capsid (HIV-2 p27^{CA} Antigen)
- PBS: Phosphatpuffer, phosphate buffered saline
- PCR: polymerase chain reaction - Polymerase-Kettenreaktion
- PEH: Entenhepatozyten / Pekingentenhepatozyten
- PGPH: Postglutamyl-Peptid hydrolysierende
- PI: Proteasom-Inhibitor
- Pol: Polymerase (Retroviren)
- P-Protein: Hepatitis-B-Virus Polymerase Protein
- PR: Protease
- Pr55: Prekursor Gag Portein von HIV-1
- Pr58: Prekursor Gag Portein von HIV-2
- PreS: großes Hepatitis-B-Virus Hüllprotein, PräS
- PS: ProScript
- PS-273: Morpholin-CONH-(CH-Naphtyl)-CONH-(CH-isobutyl)-B(OH)₂
- PS-293: Enantiomer von PS-273
- PS-296: 8-Quinolyl-sulfonyl-CONH-(CH-Naphthyl)-CONH(-CH-isobutyl)-B(OH)₂
- PS-303: NH₂(CH-Naphtyl)-CONH-(CH-isobutyl)-B(OH)₂)
- PS-325: 2-Quinol-CONH-(CH-*homo*-Phenylalanin)-CONH-(CH-isobutyl)-B(OH)₂);
- PS-334: CH₃-NH-(CH-Naphtyl-CONH-(CH-Isobutyl)-B(OH)₂);
- PS-341: N-Pyrazinecarbonyl-L-Phenylalanin-L-leuzin-Borsäure, Molekülformel: C₁₉H₂₅BN₄O₄
- PS-383: Pyridyl-CONH-(CH*p*F-Phenylalanin)-CONH-(CH-isobutyl)-B(OH)₂
- PS-519: 1*R*-[1*S*, 4*R*, 5*S*]]-1-(1-Hydroxy-2-methylpropyl)-4-propyl-6-oxa-2-azabicyclo[3.2.0]heptane-3,7-dion, Molekülformel: C₁₂H₁₉NO₄
- PSI: N-Carbobenzoxy-Ile-Glu(OBut)-Ala-Leu-H
- RNA: Ribonucleinsäure (ribonucleic acid)
- RSV: Rouse Sarcoma Virus
- RT: Reverse Transkriptase
- SDS: Natriumdodecylsulfat (Sodium Dodecylsulfate)
- SDS-PAGE: SDS-Polyacrylamid Gelelektrophorese
- SIV: Simian immunodeficiency virus
- TBS: tris buffered saline
- TNF: Tumornekrosefaktor
- Thr: Threonin
- Tris: Tris-Puffer- Tris-(hydroxymethyl)-aminomethan
- Ub: Ubiquitin
- UPS: Ub-Proteasom-System
- Vpu: Virus Protein U
- WB: Western blot
- XXX: Kategory XXX = Literatur-Stellen nach dem Prioritätsdatum
- zLLL: N-carbobenzoxyl-L-leucinyl-L-leucinyl-L-leucinal

## Patentansprüche

1. Mittel zur Behandlung von Virus-Infektionen, **dadurch gekennzeichnet, dass** sie zur Hemmung der Freisetzung, Reifung und Replikation von Retroviren als wirksame Komponente mindestens einen Proteasom-Inhibitor enthalten.

2. Mittel nach Anspruch 1, **dadurch gekennzeichnet, dass** sie gegen Viren eingesetzt werden, die im Rahmen des Replikationszyklus von der Zelloberfläche freigesetzt werden und dass die wirksamen Komponenten als Proteasom-Inhibitoren in einer pharmazeutischen Zubereitung Substanzen enthalten,
2.1. welche die Aktivitäten des Ubiquitin/Proteasom-Pathway hemmen, regulieren oder anderweitig beeinflussen oder
2.2. die speziell die enzymatischen Aktivitäten des kompletten 26S Proteasom-Komplexes und der freien, nicht mit regulatorischen Untereinheiten assemblierten 20S katalytisch aktiven Proteasom-Struktur beeinflussen oder
2.3. die von Zellen höherer Eykaryoten aufgenommen werden und nach der Zellaufnahme mit den katalytischen Untereinheiten des Proteasoms in Wechselwirkung treten und dabei alle oder einzelne der proteolytischen Aktivitäten des Proteasoms - die Trypsin-, die Chymotrypsin- und die Postglutamyl-Peptid hydrolysierenden Aktivitäten - innerhalb des 26S oder auch des 20S Proteasom-Komplexes irreversibel oder reversibel blockieren oder
2.4. die in verschiedenen Formen in vivo oral, intravenös, intramuskulär, subkutan oder in verkapselter Form mit oder ohne Zellspezifität-tragende Veränderungen verabreicht werden, aufgrund der Anwendung eines bestimmtes Applikations- und/oder Dosis-Regimes eine geringe Zytotoxizität aufweisen, keine oder unbedeutende Nebenwirkungen auslösen, eine relative hohe metabolische Halbwertszeit und eine relative geringe Clearence-Rate im Organismus aufweisen oder
2.5. die
a) in natürlicher Form aus Mikroorganismen oder anderen natürlichen Quellen isoliert werden
b) durch chemische Modifikationen aus natürlichen Substanzen hervorgehen
c) total-synthetisch hergestellt werden
d) durch gentherapeutische Verfahren *in vivo* synthetisiert werden
e) durch gentechnische Verfahren in vitro oder
f) in Mikroorganismen hergestellt werden.

3. Mittel nach Anspruch 2.5, **dadurch gekennzeichnet, dass** als Proteasom-Inhibitoren Substanzen eingesetzt werden, die folgenden Substanzklassen angehören:
a) natürlich vorkommende Proteasom-Inhibitoren:
- Peptid-Derivate, welche C-terminal Epoxyketon-Strukturen enthalten
- β-Lacton-Derivate
- Aclacinomycin A (auch bezeichnet als Aclarubicin),
- Lactacystin und dessen chemische modifizierte Varianten, wie der Zellmembranpenetrierenden Variante "Clastolactacystein β-Lacton"
b) synthetisch hergestellte Proteasom-Inhibitoren:
- modifizierte Peptidaldehyde wie N-carbobenzoxy-L-leucinyl-L-leucinyl-L-leucinal (auch bezeichnet als MG132 oder zLLL), dessen Borsäure-Derivat MG232; N-Carbobenzoxy-Leu-Leu-Nva-H (bezeichnet als MG115; N-Acetyl-L-Leuzinyl-L-Leuzinyl-L-Norleuzinal (bezeichnet als LLnL), N-Carbobenzoxy-Ile-Glu(OBut)-Ala-Leu-H (auch bezeichnet als PSI);
c) Peptide, welche C-terminal eine α,β-Epoxyketon-Struktur tragen, ferner Vinyl-sulfone wie
3.c)1. Carbobenzoxy-L-Leucinyl-L-Leucinyl-L-Leucin-vinyl-sulfon oder
3.c)2. 4-Hydroxy-5-iodo-3-nitrophenylactetyl-L-Leucinyl-L-Leucinyl-L-Leucin-vinyl-sulfon (NLVS)
d) Glyoxal- oder Borsäure-Reste wie
3.d)1. Pyrazyl-CONH(CHPhe)CONH(CHisobutyl)B(OH)₂) sowie
3.d)2. Dipeptidyl-Borsäure-Derivate oder
e) Pinacol-Ester - wie Benzyloxycarbonyl(Cbz)-Leu-Leu-boroLeu-Pinacol-Ester
und dass als besonders geeignete Proteasom-Inhibitoren die Epoxyketone
3.1. Epoxomicin (Epoxomycin, Molekülformel: C₂₈H₈₆N₄O₇) und/oder
3.2. Eponemycin (Eponemicin, Molekülformel: C₂₀H₃₆N₂O₅)
eingesetzt werden.

4. Mittel nach Anspruch 3, **dadurch gekennzeichnet, dass** als besonders geeignete Proteasom-Inhibitoren aus der PS-Serie die Verbindungen
4.1. PS-519 als β-Lacton- sowie als Lactacystin-Derivat die Verbindung 1R-[1S, 4R, 5S]]-1-(1-Hydroxy-2-methylpropyl)-4-propyl-6-oxa-2-azabicyclo[3.2.0]heptane-3,7-dione-Molekülformel C₁₂H₁₉NO₄ - und/oder
4.2. PS-314 als Peptidyl-Borsäure-Derivat die Verbindung N-Pyrazinecarbonyl-L-Phenylalanin-L-Leuzin-Borsäure - Molekülformel C₁₉H₂₅BN₄O₄ - und/oder
4.3. PS-273 (Morpholin-CONH-(CH-Naphthyl)-CONH-(CH-isobutyl)-B(OH)₂) und dessen Enantiomer PS-293 und/oder
4.4. die Verbindung PS-296 (8-Quinolyl-sulfonyl-CONH-(CH-Napthyl)-CONH(-CH-isobutyl)-B(OH)₂) und/oder
4.5. PS-303 (NH₂(CH-Naphthyl)-CONH-(CH-isobutyl)-B(OH)₂) und/oder
4.6. PS-321 als (Morpholin-CONH-(CH-Napthyl)-CONH-(CH-Phenylalanin)-B(OH)₂); - und/oder
4.7. PS-334 (CH₃-NH-(CH-Naphthyl-CONH-(CH-Isobutyl)-B(OH)₂) und/oder
4.8 die Verbindung PS-325 (2-Quinol-CONH-(CH-*homo*-Phenylalanin)-CONH-(CH-isobutyl)-B(OH)₂) und/oder
4.9. PS-352 (Phenyalanin-CH₂-CH₂-CONH-(CH-Phenylalanin)-CONH-(CH-isobutyl)l-B(OH)₂) und/oder
4.10. PS-383 (Pyridyl-CONH-(CH*p*F-Phenylalanin)-CONH-(CH-isobutyl)-B(OH)₂)
eingesetzt werden.

5. Mittel nach Anspruch 1 bis 4, **dadurch gekennzeichnet, dass** die pharmazeutischen Zubereitungen neben Proteasom-Inhibitoren auch andere Mittel enthalten, welche das zelluläre Ubiquitin-System beeinflussen, regulieren oder hemmen, wie die Aktivitäten
5.1. der Ubiquitin-konjugierenden Enzyme und/oder
5.2. der Ubiquitin-hydrolysierenden Enzyme
5.3. von zellulären Faktoren, die mit Ubiquitin in Wechselwirkung treten
5.4. von zellulären Faktoren, die mit Ubiquitin als
5.4.1. Mono-Ubiquitin oder als
5.4.2. Poly-Ubiquitin
in Wechselwirkung treten.

6. Verwendung von Proteasom-Inhibitoren der Ansprüche 1 bis 5 zur Herstellung von Mitteln und/ oder pharmazeutischen Zubereitungen zur Behandlung von Virus-Infektionen durch Hemmung der Freisetzung, Reifung und Replikation von Retroviren.

7. Verwendung von Proteasom-Inhibitoren nach Anspruch 6 zur Herstellung von Arzneimitteln
7.1. zur Behandlung und Prophylaxe von durch Retroviren verursachten Erkrankungen
7.2. zur Behandlung von HIV-induzierten pathologischen Erscheinungen wie AIDS und AIDS-assoziierte Erkrankungen, zur Verhinderung des Krankheitsausbruches und zur Reduzierung der Infektionsausbreitung im Organismus (Reduzierung von "viral load") von symptomlosen HIV-1-/HIV-2-seropositiven und HIV-1-/HIV-2-infizierten Personen und zur Bekämpfung / Behandlung von AIDS in fortgeschrittener Krankheitsphase
7.3. zur Hemmung von späten Prozessen im Replikationszyklus von Retroviren
7.4. zur Hemmung der Assemblierung und Freisetzung von Virionen von der Zelloberfläche
7.5. zur Hemmung der proteolytischen Prozessierung der Gag-Strukturproteine durch virale Protease
7.6. zur Behandlung / Bekämpfung / Verhinderung von HIV-induzierter Demenz, insbesondere zur Verhinderung der HIV-Infektion von Neuronen, Glia, und Endothelzellen in Kapillaren des Gehirns
7.7. zur Verhinderung der Etablierung einer systemischen HIV-1-/HIV-2-Infektion unmittelbar nach Kontakt mit infektiösen Viren, etwa bei Nadel-Stich-Verletzungen mit HIVkontaminiertem Blut oder Blutprodukten
7.8. zur Behandlung und Prophylaxe von HIV-induzierten Störungen im Lipidstoffwechsel, speziell dem HLS-Syndrom (HIV-associated lipodystrophy syndrome) oder
7.9. zur Behandlung und Prophylaxe von HIV-induzierten Störungen in der Nierenfunktion, speziell dem HIVAN-Syndrom (HIV associated nephropathy).

8. Verwendung nach Anspruch 6 und 7, **dadurch gekennzeichnet, dass** die Freisetzung, Reifung und Replikation von
a) Spumaviren oder
b) Mammalian C-Typ Oncoviren oder
c) BLV (Bovine Leukemia Virus) oder
d) HTLV (Human T-Cell Leukemia Virus) - HTLV-I / HTLV-II - oder
e) Leukämieviren oder
f) RSV (Rous Sarcoma Virus) Viren oder
g) Lentiviren
g1) Humanes Immundefizienzvirus Type 1 (HIV-1) oder
g2) Humanes Immundefizienzvirus Type 2 (HIV-2) oder
g3) Affenimmundefizienzvirus (SIV) oder
g4) Katzen-Immundefizienzvirus (FIV) oder
g5) Rinder-Immundefizienzvirus (BIV)
gehemmt werden.

9. Verwendung nach Anspruch 6 und 7 in Kombination mit
9.1. anderen anti-retroviralen Medikamenten
9.2. Blockern der Reversen Transkriptase und/oder der viralen Protease
9.3. anti-retroviralen Therapien basierend auf gentherapeutischen Interventionen
9.4. intrazellulärer Immunisierung
9.5. dem Einbringen von anti-HIV-1-/HIV-2-wirksamen Genen in Stammzellen und/oder peripheren CD4⁺ Lymphozyten.

10. Verwendung nach Anspruch 6 und 7 zur Behandlung von Koinfektionen mit HBV und Immundefizienzviren HIV-1 und HIV-2 und/ oder zur Behandlung von HBV/HIV-Koinfektionen in Kombination mit der HAART-Therapie.
